(19) Europäisches Patentamt
European Patent Office
Office européen des brevets

(11) Publication number: **0 279 149 B1**

(12) # EUROPEAN PATENT SPECIFICATION

(45) Date of publication of patent specification: **30.12.92**

(51) Int. Cl.5: **C07D 401/12**, A61K 31/415, A61K 31/44

(21) Application number: **87850362.2**

(22) Date of filing: **20.11.87**

Consolidated with 87908006.7/0332647 (European application No./publication No.) by decision dated 23.05.90.

(54) **Benzimidazole derivatives, process for their production and a pharmaceutical composition containing the same.**

(30) Priority: **21.11.86 SE 8604998**
**23.12.86 SE 8605551**
**16.10.87 SE 8704049**

(43) Date of publication of application:
**24.08.88 Bulletin 88/34**

(45) Publication of the grant of the patent:
**30.12.92 Bulletin 92/53**

(84) Designated Contracting States:
**AT BE CH DE ES FR GB GR IT LI LU NL SE**

(56) References cited:
**EP-A- 0 045 200**
**EP-A- 0 176 308**
**EP-A- 0 221 041**
**GB-A- 2 134 523**

**"Design of prodrugs" by H. Bundgaard, 1985, pp. 7-9 & 21-24, Elsevier, Amsterdam**

**"Drug delivery systems" by R.L. Juliano, 1980, pp. 124-128, Oxford University Press, New York**

(73) Proprietor: **Aktiebolaget Hässle**
**Kärragatan 5**
**S-431 83 Mölndal(SE)**

(72) Inventor: **Alminger, Tomas Börje**
**Hassungared PL 4204**
**S-437 00 Lindome(SE)**
Inventor: **Bergman, Rolf Axel**
**Alegardsgatan 384**
**S-431 45 Mölndal(SE)**
Inventor: **Bundgaard, Hans**
**Tjornevei 36**
**DK-2970 Horsholm(DK)**
Inventor: **Lindberg, Per Lennart**
**Knapehall 64**
**S-436 00 Askim(SE)**
Inventor: **Sunden, Gunnel Elisabeth**
**Eketrägatan 24A**
**S-417 12 Göteborg(SE)**

(74) Representative: **Hjertman, Ivan T. et al**
**AB ASTRA Patent and Trade Mark Department**
**S-151 85 Södertälje(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### Field of the invention

The object of the present invention is to provide novel compounds, and therapeutically acceptable salts thereof, which inhibit exogenously or endogenously stimulated gastric acid secretion and thus can be used in the prevention and treatment of peptic ulcer.

The present invention relates to the use of the compounds of the invention, especially therapeutically acceptable salts thereof, for inhibiting gastric acid secretion in mammals and man. In a more general sense, the compounds of the invention may be used for prevention and treatment of gastrointestinal inflammatory diseases, and gastric acid-related diseases in mammals and man, such as gastritis, gastric ulcer, duodenal ulcer, and reflux esophagitis. Furthermore, the compounds may be used for treatment of other gastrointestinal disorders where gastric antisecretory effect is desirable e.g. in patients with gastrinomas, and in patients with acute upper gastrointestinal bleeding. They may also be used in patients in intensive care situations, and pre-and postoperatively to prevent acid aspiration and stress ulceration. The invention also relates to pharmaceutical compositions containing at least one compound of the invention, or a therapeutically acceptable salt thereof, as active ingredient. In a further aspect, the invention relates to processes for preparation of such new compounds, to novel intermediates in the preparation of the compounds of the invention, and to the use of the active compounds for the preparation of pharmaceutical compositions for the medical use indicated above.

### Prior art and background of the invention

Benzimidazole derivatives intended for inhibiting gastric acid secretion are disclosed in numerous patent documents. Among these can be mentioned GB 1 500 043, GB 1 525 958, US 4 182 766, EP 0 005 129, and BE 890 024. Benzimidazole derivatives proposed for use in the treatment or prevention of special gastrointestinal inflammatory diseases are disclosed in EP-A-0 045 200. N-substituted 2-(pyridylalkylenesulfinyl)-benzimidazoles are disclosed in EP-A-0 176 308, and in EP-A-221 041. The N1 substituents in the said last documents differ from the N1 substituents in the compounds of the present invention.

For certain pharmaceutical usage forms there is a great need for high water solubility of the compound to be used. For instance, for intravenous and intramuscular injection formulations, the dose is to be dissolved in a small volume of an aqueous medium. This, of course, requires a high aqueous solubility. However, a high aqueous solubility is often a great advantage also in other cases e.g. for oral formulations.

The compounds in the prior art generally have a low water solubility, which does not admit manufacture of such highly concentrated water solution which are needed for intravenous and intramuscular injections. For example the compounds presented in the European patent application, publication no. 0176308, disclosing N-substituted benzimidazole derivatives, have low water solubility and are thus not suitable for the above-mentioned parenteral use.

### The invention

It has been found that the compounds of the following formula I are effective as inhibitors of gastric acid secretion in mammals and man, and that the said compounds I exhibit an unexpectedly high solubility in water compared with compounds in the prior art.

The compounds of the invention wherein X is SO generally show higher chemical stability in water solutions at the pH where they exhibit optimal stability compared to the corresponding compounds without the N-1 substitution, at the same pH. Some of the compounds of the invention show exceedingly high chemical stability in solution. The compounds of the formula I are therefore particularly suitable for parenteral, especially intravenous and intramuscular administration. The high solubility and chemical stability also render the compounds of the invention suitable for other administration routes, such as for instance oral and rectal administration.

The compounds of the invention are of the following formula I:

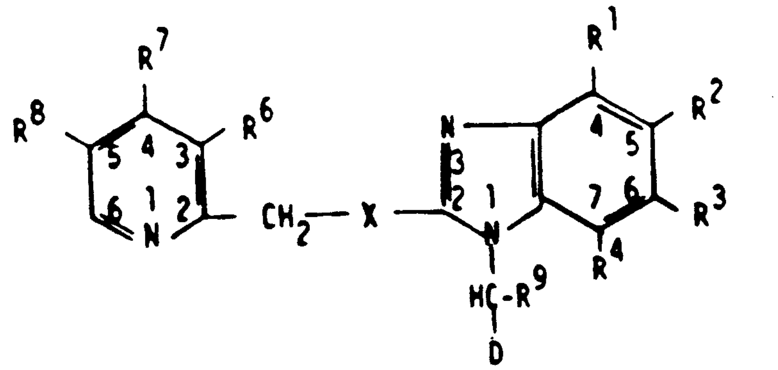

I

and physiologically acceptable salts thereof,
wherein

| | |
|---|---|
| X is | -S- or -SO-; |
| $R^1$, $R^2$, $R^3$ and $R^4$, | which are the same or different, are<br>(a) H<br>(b) alkyl containing 1-8, especially 1-6 carbon atoms<br>(c) alkoxy containing 1-8, especially 1-6 carbon atoms<br>(d) alkoxyalkyl containing 1-3 carbon atoms in each alkyl part<br>(e) alkoxyalkoxy containing 1-3 carbon atoms in each alkyl part<br>(f) halogen<br>(g) $-COR^{10}$<br>(h) alkylthio containing 1-6 carbon atoms in the alkyl part<br>(i) alkylsulfinyl containing 1-7 carbon atoms in the alkyl part<br>(j) phenylalkyl or phenylalkoxy, containing 1-6 carbon atoms in the alkyl and alkoxy parts, respectively,<br>(k) phenyl, or phenoxy, whereby each phenyl group optionally is substituted by 1-3 substituents, the same or different and selected from halogen, $CF_3$, (1-5C) alkyl and (1-5C) alkoxy<br>(l) haloalkoxy containing 1-6 carbon atoms and 1-11, especially 1-6 halogen atoms; |
| D is | 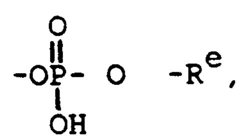 |
| $R^6$ is | (a) H<br>(b) alkyl containing 1-8, especially 1-6 carbon atoms<br>(c) alkoxy containing 1-8, especially 1-6 carbon atoms<br>(d) halogen |
| $R^8$ is | (a) H<br>(b) alkyl containing 1-8, especially 1-6 carbon atoms<br>(c) alkoxy containing 1-8, especially 1-6 carbon atoms<br>(d) halogen<br>(e) phenylalkyl containing 1-4 carbon atoms in the alkyl part |
| $R^7$ is | (a) H<br>(b) alkyl containing 1-7 carbon atoms<br>(c) alkoxy containing 1-7 carbon atoms<br>(d) alkoxyalkyl containing 1-3 carbon atoms in each alkyl part<br>(e) alkoxyalkoxy containing 1-3 carbon atoms in each alkyl part<br>(f) phenoxy, whereby the phenyl group optionally is substituted by 1 or 2 substituents, the same or different and selected from halogen, $CF_3$, (1-3C) alkyl or (1-3C)alkoxy |

(g) phenylalkyl or phenylalkoxy containing 1-7 carbon atoms in the alkyl and alkoxy part, respectively, whereby the phenyl part optionally is substituted by 1 or 2 substituents, the same or different and selected from halogen, $CF_3$, (1-3C)alkyl and (1-3C) alkoxy
(h) alkenyloxy containing up to 7 carbon atoms in the alkenyl part
(i) alkynyloxy containing up to 7 carbon atoms in the alkynyl part
(j) alkylthio containing 1-7, preferably 1-3 carbon atoms in the alkyl part
(k) phenylthio or phenylalkylthio containing 1-3, preferably 1 carbon atom in the alkyl part
(l) dialkylamino containing 1-7, preferably 1-3 carbon atoms in each of the alky parts
(m) morpholino
(n) piperidino
(o) N-methylpiperazino
(p) pyrrolidino
(q) fluoroalkoxy containing 2-5 carbon atoms and 1-9 fluorine atoms

or $R^6$ and $R^7$, or $R^7$ and $R^8$ together with the adjacent carbon atoms in the pyridine ring form a 5- or 6-membered, saturated or unsaturated ring, which may optionally contain an oxygen, sulphur or an optionally alkylated nitrogen atom;

$R^9$ is (a) H
(b) alkyl containing 1-4 carbon atoms;
$R^{10}$ is (a) alkyl containing 1-6 carbon atoms
(b) alkoxy containing 1-6 carbon atoms
(c) phenyl
$R^e$ is (a) H
(b) (1-6C)alkyl
(c) phenyl(0-3C)alkyl;

and whereby the group D preferably is in the form of a mono- or di-ionic salt containing a physiologically acceptable counter cation; with the proviso that either or both of $R^6$ and $R^8$ is halogen when $R^7$ is dialkylamino, morpholino, pieperidino, N-methyl-piperazino or pyrrolidino.

It should be understood that the expressions "alkyl" and "alkoxy" include straight, branched, and cyclic structures including cycloalkylalkyl and cycloalkylalkoxy, respectively.

The number of carbon atoms in a given radical is in this specification either expressly stated or given in abbreviated form within parentheses such as (1-3C)alkyl.

The compounds of the invention that are sulfoxides have an asymmetric centre in the sulfur atom, i.e. these compounds exist as two optical isomers (enantiomers), or if they also contain one or more asymmetric carbon atoms the compounds have two or more diastereomeric forms, each existing in two enantiomeric forms.

Both the pure enantiomers, racemic mixtures (50% of each enantiomer) and unequal mixtures of the two are within the scope of the present invention. It should be understood that all the diasteromeric forms possible (pure enantiomers or racemic mixtures) are within the scope of the invention.

The compounds of the invention that are sulfides ($X=S$) may be asymmetric due to one or more asymmetric carbon atoms, as described above. The different diastereomeric forms possible as well as the pure enantiomers and racemic mixtures are within the scope of the invention.

Preferred groups of compounds of the formula I are:

1. Compounds wherein X is -SO-.
2. Compounds wherein X is -S-.
3. Compounds wherein $R^1$, $R^2$, $R^3$ and $R^4$ are the same or different and selected from hydrogen, (1-4C)-alkyl, (5-6C)cycloalkyl, (1-4C)alkoxy, (1-2C)alkoxy(1-2C)alkyl, (1-2C)alkoxy(1-2C)alkoxy, halogen, (2-4C)-alkanoyl, phenylcarbonyl, (1-2C)alkoxycarbonyl, phenyl (1-3C)alkoxy, phenyl or halo(1-4C)alkoxy.
4. Compounds wherein $R^1$, $R^2$, $R^3$ and $R^4$ are the same or different and selected from hydrogen, (1-4C)-alkyl, (1-4C)alkoxy, (1-2C)alkoxy(1-2C)alkyl, fluorine, (2-4C)alkanoyl, (1-2C)alkoxycarbonyl, fluoro(1-4C)-alkoxy.
5. Compounds wherein $R^1$, $R^2$, $R^3$ and $R^4$ are the same or different and selected from hydrogen, methyl, ethyl, isopropyl, t-butyl, methoxy, ethoxy, methoxymethyl, ethoxymethyl, fluorine, trifluoromethoxy, tetrafluoroethoxy.
6. Compounds wherein $R^1$, $R^2$, $R^3$ and $R^4$ are the same or different and selected from hydrogen, t-butyl, methoxy, ethoxy, methoxymethyl, ethoxymethyl, fluorine, trifluoromethoxy, tetrafluoroethoxy,

7. Compounds wherein $R^1$ and $R^4$ are H and $R^2$ and $R^3$ are selected from t-butyl, methoxy, methoxymethyl, fluorine, trifluoromethoxy,
8. Compounds wherein $R^1$, $R^2$, $R^3$ and $R^4$ are H, alkyl containing 1-6 carbon atoms, or alkoxy containing 1-6 carbon atoms.
9. Compounds wherein $R^1$ and $R^4$ are H and $R^2$ and $R^3$ both are alkoxy containing 1-3 carbon atoms.
10. Compounds wherein $R^1$, $R^2$, $R^3$ and $R^4$ all are H.
11. Compounds wherein $R^1$ and $R^4$ are alkoxy containing 1-3 carbon atoms and $R^2$ and $R^3$ are H.
12. Compounds wherein $R^1$, $R^3$ and $R^4$ are H and $R^2$ is $OCH_3$.
13. Compounds wherein $R^1$, $R^2$ and $R^4$ are H and $R^3$ is $OCH_3$.
14. Compounds wherein $R^7$ is alkyl containing 1-6 carbon atoms, alkoxy containing 1-6 carbon atoms.
15. Compounds wherein $R^7$ is phenoxy or phenylalkoxy, optionally substituted.
16. Compounds wherein $R^7$ is alkoxy containing 1-6 carbon atoms.
17. Compounds wherein $R^9$ is H or $CH_3$, especially H.
18. Preferred substituents in position 1 of the benzimidazole nucleus are those where $R^9$ is H and D is as exemplified in Table 1 below.
19. Compounds wherein D is

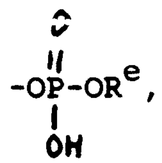

particularly alkali salts thereof.
20. Compounds wherein D is

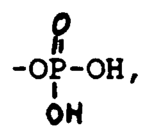

particularly alkali salts thereof.
21. Compounds wherein D is

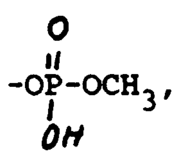

particularly alkali salts thereof.
22. Compounds wherein D is

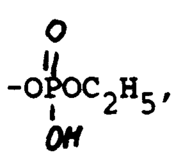

particularly alkali salts thereof.
23. Compounds wherein

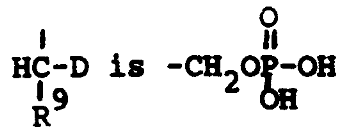

and alkali metal salts thereof.

24. Compounds wherein

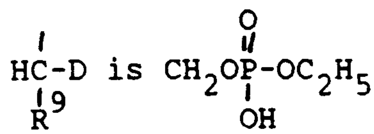

and alkali metal salts thereof.

25. Compounds wherein $R^9$ is H and D is

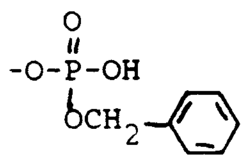

26. Preferred benzimidazole structures are: unsubstituted, 5-methoxy and 6-methoxy-substituted.

27. Compounds wherein $R^1$, $R^2$, $R^3$, and $R^4$ are the same or different and selected from hydrogen, (1-4C)alkyl, (5-6C) cycloalkyl, (1-4C)alkoxy, (1-2C)alkoxy(1-2C)alkyl, (1-2C) alkoxy(1-2C)alkoxy, halogen, (2-4C)alkanoyl, phenylcarbonyl, (1-2C)alkoxycarbonyl, phenyl(1-3C)alkoxy, phenyl or halo (1-4C)alkoxy;

D is

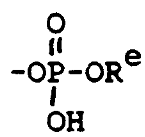

and alkali metal salts thereof;

X is SO;

$R^6$ and $R^8$ are $CH_3$; and $R^7$ is $OCH_3$.

28. Preferred of the pyridine fragments are: 3,5-dimethyl-4-methoxy-, 3-methyl-4-methoxy-, 5-ethyl-4-methoxy-, 4-methoxy-, 4-ethoxy-, 4-isopropoxy-, 3,5-dimethyl-, 3,4-dimethyl-, 4,5-dimethyl-, 3-methyl-4-(2,2,2-trifluoro)ethoxy-, 3,4-dimethoxy-, 4,5-dimethoxy-, 3-methyl-4-ethylthio-, 3-methyl-4,5-dimethoxy-, 3,4,5-trimethyl, 3-ethyl-4-methoxy-, 3-n-propyl-4-methoxy-, 3-isopropyl-4-methoxy-, 3-t-butyl-4-methoxy-substituted.

29. Particularly preferred of the pyridine fragments are: 3,5-dimethyl-4-methoxy-, 3-methyl-4-methoxy, 3-ethyl-4-methoxy, 3-isopropyl-4-methoxy, 4-methoxy, 4-ethoxy- and 4-isopropoxy-substituted, especially 3,5-dimethyl-4-methoxy-substituted.

30. Additional preferred compounds are obtained by combining the indicated preferred meanings for some or all of the radicals X and $R^1$-$R^{10}$ as indicated in the groups 1-29 above.

31. Preferred of the pyridinylmethylsulfinyl benzimidazole moieties are

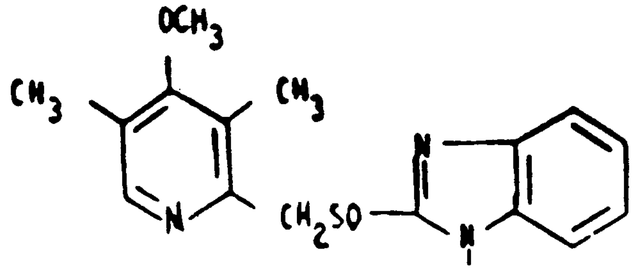

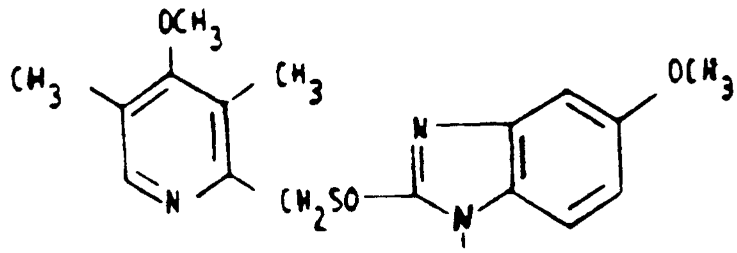

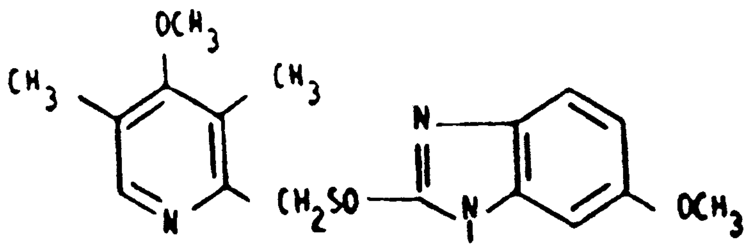

32. Preferred groups of the radicals $R^6$ and $R^8$ are H, $CH_3$, $C_2H_5$, n-$C_3H_7$, i-$C_3H_7$, and t-$C_4H_9$.
33. Compounds of the formula

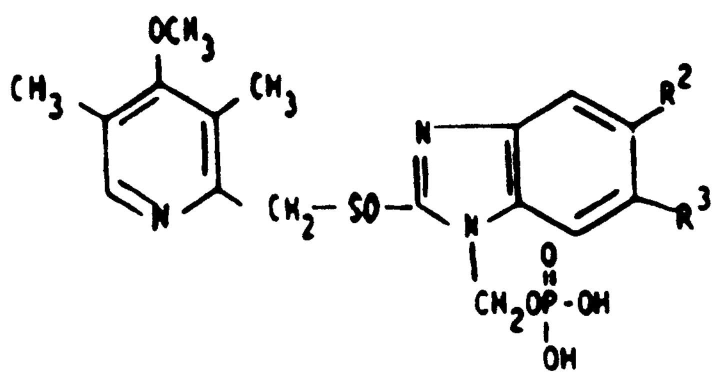

or a mono- or di-ionic salt thereof containing a physiologically acceptable counter cation, wherein $R^2$ and $R^3$ are combined as follows:

| $R^2$ | $R^3$ |
|---|---|
| H | $OCH_3$ |
| $OCH_3$ | H |

34. The most preferred compounds of the invention are

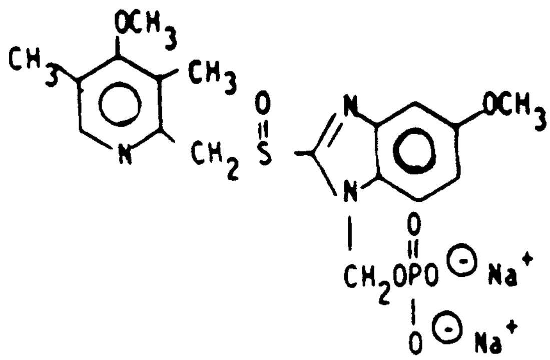
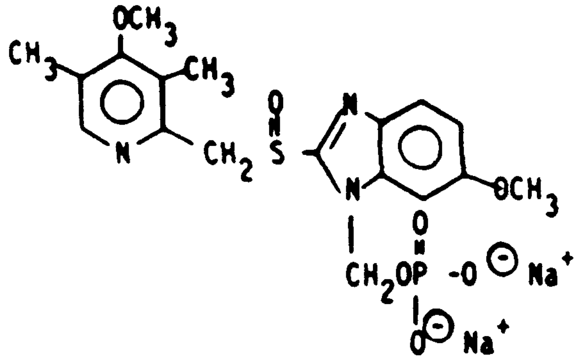

35. Other preferred compounds of the invention are

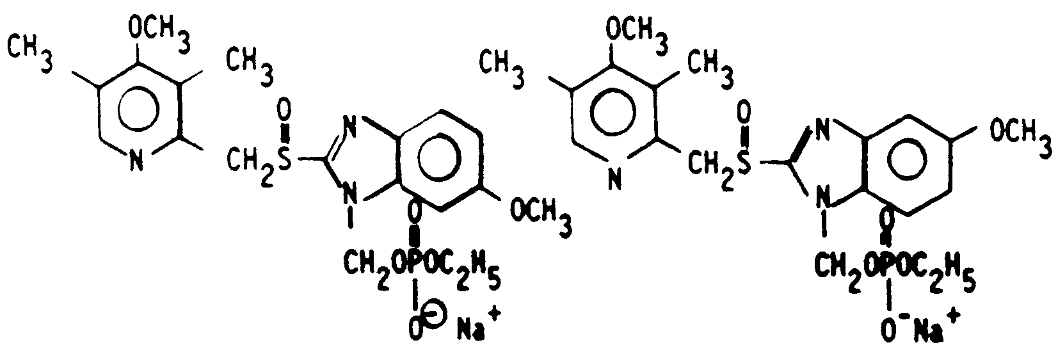

Illustrative examples of the various radicals in the formula I are as follows. These illustrative examples will be applicable to the different radicals depending on the number of carbon atoms prescribed for each radical.

The group alkyl in the definitions of $R^1$, $R^2$, $R^3$, $R^4$, $R^6$, $R^7$, $R^8$ $R^9$, $R^{10}$ and $R^e$ are exemplified by methyl, ethyl, n-propyl, isopropyl, n-butyl, isobutyl, t-butyl, n-pentyl, n-hexyl, cyclopropyl, cyclopentyl, cyclohexyl, cyclopropylmethyl, cyclopentylmethyl, cyclopentylethyl, and cyclohexylmethyl. Lower alkyl groups containing 1-4 carbon atoms are especially preferred.

The group alkoxy in the defintions of $R^1$, $R^2$, $R^3$, $R^4$, $R^6$, $R^7$, $R^8$ and $R^{10}$ are exemplified by methoxy, ethoxy, n-propoxy, iso-propoxy, n-butoxy, i-butoxy, s-butoxy, t-butoxy, n-pentoxy, i-pentoxy, n-hexoxy, cyclopropoxy, cyclopentoxy, cyclohexoxy, cyclopropylmethoxy, cyclopentylmethoxy, cyclopentylethoxy, and cyclohexylmethoxy. Lower alkoxy groups are preferred, especially those containing 1-4 carbon atoms, preferably a lower alkoxy group having especially preferred 1-3 carbon atoms, e.g. methoxy, ethoxy, n-propoxy or isopropoxy.

Halogen in the definitions of $R^1$, $R^2$, $R^3$, $R^4$, $R^6$ and $R^8$ is chloro, bromo, fluoro and iodo, preferably chloro, bromo, and fluoro.

In $R^1$, $R^2$, $R^3$, $R^4$ and $R^7$ when representing alkylthio or alkylsulfinyl is the alkyl preferably a lower alkyl having especially preferred 1-4 carbon atoms, e.g. methylthio, methylsulfinyl, ethylthio, ethylsulfinyl, isopropylthio, n-butylsulfinyl or isobutylthio.

The groups phenylalkyl, phenylalkoxy, and phenylalkylthio, when present in $R^1$, $R^2$, $R^3$, $R^4$, $R^7$, $R^8$, and $R^e$ have preferably up to 10 carbon atoms in the phenyl group. Especially preferred are 1-3 carbon atoms in the alkyl group or alkoxy group, respectively, e.g. phenylmethyl, phenylethyl, phenylmethoxy, phenylethoxy, phenylpropyl, phenylisopropoxy, phenylmethylthio, and phenylethylthio.

$R^1$, $R^2$, $R^3$, $R^4$ and $R^7$ representing an alkoxy alkyl or alkoxyalkoxy group are exemplified by methoxymethyl, methoxyethyl, methoxypropyl, ethoxymethyl, ethoxyethyl, propoxyethyl, methoxymethoxy, methoxyethoxy, methoxypropoxy, ethoxyethoxy and propoxyethoxy.

$R^7$ representing an alkenyloxy or alkynyloxy group has preferably 2-7 carbon atoms, especially preferred 3-4 carbon atoms, e.g. allyloxy, propargyloxy, 2-butenyloxy and 2-butynyloxy.

$R^6$ and $R^7$, or $R^7$ and $R^8$ representing a 5- or 6-membered saturated or unsaturated ring is preferably a saturated carbocyclic ring or a saturated ring containing an oxygen or a sulphur atom in the 4-position in the

pyridine ring, e.g. $-CH_2CH_2CH_2-$, $-CH_2CH_2CH_2CH_2-$, $-O-CH_2CH_2-$, $-O-CH_2CH_2CH_2-$, $-SCH_2CH_2-$, or $SCH_2CH_2CH_2-$.

$R^1$, $R^2$, $R^3$ and $R^4$ when representing haloalkoxy is preferably a lower haloalkoxy. Especially preferred are lower fluoroalkoxy, or fluorochloroalkoxy groups, e.g. $OCF_3$, $OCHF_2$, $OCF_2CHF_2$, $OCF_2CF_3$, $OCF_2Cl$, $OCH_2CF_3$.

$R^7$ when representing fluoroalkoxy is exemplified by $OCH_2CF_3$, $OCH_2CF_2CF_3$ and $OCH_2CF_2CHF_2$.

$R^7$ when representing a dialkylamino group is preferably $-N(CH_3)_2$, or $-N(C_2H_5)_2$.

For the compounds with the general formula I containing an asymmetric centre, both the pure enantiomers and the racemic mixtures are within the scope of the present invention.

Further illustrative examples of the radicals in the formula I are given in the examples and lists of specific compounds given elsewhere in this specification.

It is believed that compounds of formula I are metabolized before exerting their effect. Such metabolism may occur in the N-substituent in position 1 in the benzimidazole nucleus. Moreover, compounds of the invention wherein X is S are believed to exert their antisecretory activity after metabolism to compounds wherein X is SO.

Preparation

B. Compounds of the formula I wherein $R^9$ is H, are prepared by reacting a compound of the formula IV

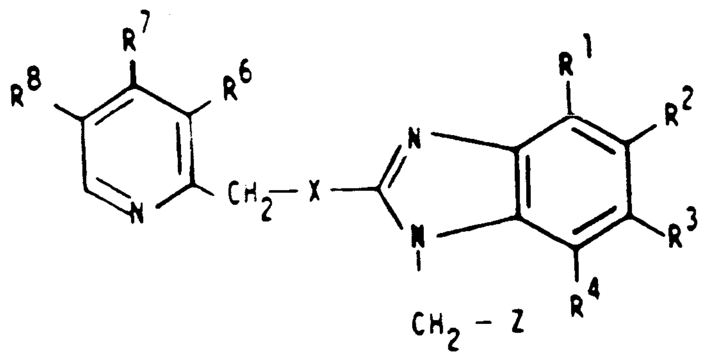

IV

wherein $R^1$, $R^2$, $R^3$, $R^4$, $R^6$, $R^7$, $R^8$ and X are as defined under formula I, and Z is halogen such as Cl, Br or I or a functionally equivalent group, with a compound of the formula VI, VII, VIII, or IX,

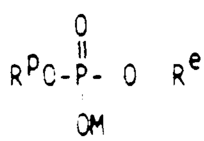

;

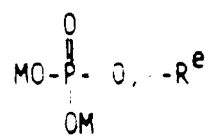

VI

VII

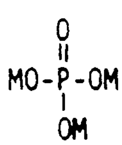

;

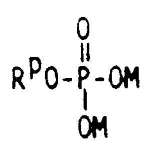

VIII

IX

wherein $R^e$ is as defined under formula I above, $R^p$ is a suitable protecting group such as cyanoethyl, benzyl or p-nitrophenyl, and M is a counter ion such as $Na^+$, $K^+$, $Ag^+$ or trialkylammonium. In the case

where a protecting group is used, such protecting group is removed after the coupling reaction (see under E below).

C. Compounds of the formula I wherein X is SO are prepared by oxidizing a compound of the same formula I

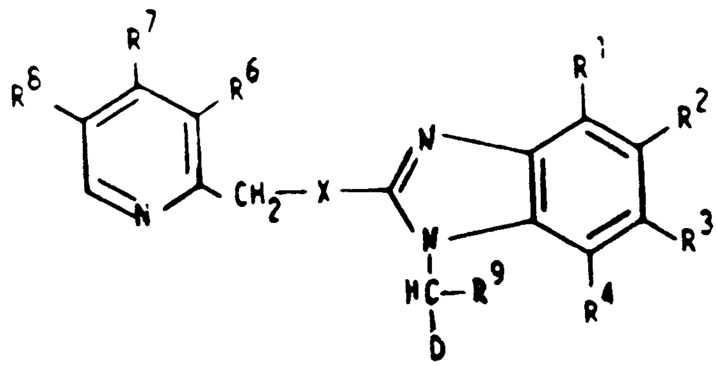

I

wherein X is S, $R^1$, $R^2$, $R^3$, $R^4$, $R^6$, $R^7$, $R^8$, $R^9$ and D have the meanings given above. This oxidation may be carried out by using an oxidizing agent selected from the group consisting of nitric acid, hydrogen peroxide, peracids, peresters, ozone, dinitrogentetraoxide, iodosobenzene, N-halosuccinimide, 1-chlorobenzotriazole, t-butylhypochlorite, diazabicyclo-|2,2,2|-octane bromine complex, sodium metaperiodate, selenium dioxide, manganese dioxide, chromic acid, cericammonium nitrate, bromine, chlorine, and sulfuryl chloride. The oxidation usually takes place in a solvent wherein the oxidizing agent is present in some excess in relation to the product to be oxidized.

The oxidation may also be carried out enzymatically by using an oxidating enzyme or microbiotically by using a suitable microorganism.

D. Compounds of the formula I are prepared by reacting a compound of the formula IIA

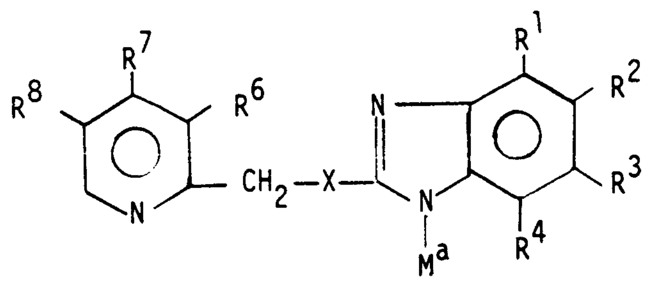

IIA

wherein $R^1$, $R^2$, $R^3$, $R^4$, $R^6$, $R^7$, $R^8$ and X are as defined under formula I and $M^a$ is either a metal cation such as $Na^+$, $K^+$, $Li^+$ or $Ag^+$ or a quaternary ammonium ion, such as tetrabutylammonium with a compound of the formula

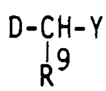

XII

wherein D and $R^9$ are as defined under formula I and Y is halogen such as Cl, Br or I, or a functionally equivalent group.

The reaction of a compound of formula IIA with a compound of formula XII is suitably carried out under protective gas in absence of water. Suitable solvents are hydrocarbons such as toluene and benzene and halogenated hydrocarbons such as methylene chloride and chloroform.

The reaction of the compounds of formula IIA and XII may be carried out at a temperature between the ambient temperature and the boiling temperature of the reaction mixture.

E. Removal of a protective group in the D substituent is done by methods well known in the art. Thus, for instance, the phosphates may be protected as dibenzyl- or diphenyl esters, which can be cleaved by

basic hydrolysis. The dibenzyl esters may also be cleaved by sodium iodide in acetone. The cyanoethyl protecting group may be removed by treatment with a base such as NaOH.

Depending on the process conditions and the starting materials, the end products of the formula I are obtained either in neutral or salt form. Both the neutral compounds and the salts of these end products are included within the scope of the invention. Thus, salts may be obtained as well as hemi, mono, sesqui or polyhydrates.

Acid addition salts of the amino-containing compounds may in a manner known per se be transformed into free base using basic agents such as alkali or by ion exchange. The free bases obtained may then be converted into salts with organic or inorganic acids. In the preparation of acid addition salts preferably such acids are used which form suitable therapeutically acceptable salts. Examples of such acids are hydrohalogen acids, sulfonic acid, phosphoric acid, nitric acid, and perchloric acid; aliphatic, alicyclic, aromatic or heterocyclic carboxyl or sulfonic acids, such as formic acid, acetic acid, propionic acid, succinic acid, glycolic acid, lactic acid, malic acid, tartaric acid, citric acid, ascorbic acid, maleic acid, hydroxymaleic acid, pyruvic acid, phenylacetic acid, benzoic acid, p-aminobenzoic acid, p-hydroxybenzoic acid, salicylic acid or p-aminosalicylic acid, embonic acid, methanesulfonic acid, ethanesulfonic acid, hydroxyethanesulfonic acid, ethylenesulfonic acid, halogenbenzenesulfonic acid, toluenesulfonic acid, napthylsulfonic acid or sulfanilic acids, methionine, tryptophane, lysine or arginine.

Base addition salts of the carboxylic acid - or phosphorous - containing compounds may in a corresponding way be transformed into the acid form, and then reconverted to a therapeutically suitable salt such as sodium and potassium salts.

Racemates obtained can be separated according to known methods, e.g. recrystallization from an optically active solvent.

In the case of diastereomeric mixtures (racemate mixtures) these may be separated into stereoisomeric (diastereomeric) pure racemates by means of chromatography or fractional crystallization.

The starting materials utilized in the methods B-E are in some cases novel. These novel starting materials may, however, be obtained according to processes known per se.

Starting materials of the formula IV are novel and constitute as such part of the invention. Compounds of the formula IV wherein Z is Cl are prepared by reaction of a compound of the formula IV wherein Z is OH with a chlorinating agent such as $SOCl_2$ in the presence of a suitable base such as triethylamine in a suitable solvent such as $CH_2Cl_2$, toluene, acetonitrile, tetrahydrofuran or mixtures thereof. For the preparation of compounds of the formula IV wherein Z is Br or I, analogous methods are used utilizing suitable reagents containing bromine, such as $BBr_3$, or iodine. Examples of these intermediates are given in Example I1 to I39 in Table 3 below.

The starting materials having X = S, utilized in Method C may be obtained according to Method B.

For clinical use the compounds of the invention are formulated into pharmaceutical formulations for oral, rectal, parenteral or other mode of administration. It is especially preferred to formulate the compounds of the invention into pharmaceutical formulations for parenteral administration. The pharmaceutical formulation contains a compound of the invention in combination with a pharmaceutically acceptable carrier. The carrier may be in the form of a solid, semi-solid or liquid diluent, or a capsule. These pharmaceutical preparations are a further object of the invention. Usually the amount of active compounds is between 0.l-95% by weight of the preparation, between 0.2-20% by weight in preparations for parenteral use and between l and 50% by weight in preparations for oral administration.

In the preparation of pharmaceutical formulations containing a compound of the present invention in the form of dosage units for oral administration the compound selected may be mixed with a solid, powdered carrier, such as lactose, saccharose, sorbitol, mannitol, starch, amylopectin, cellulose derivatives, gelatin, or another suitable carrier, as well as with lubricating agents such as magnesium stearate, calcium stearate, sodium stearyl fumarate and polyethylenglycol waxes. The mixture is then processed into granules or pressed into tablets. Granules and tablets containing sulfoxides may be coated with an enteric coating which protects the active compound from acid catalyzed degradation as long as the dosage form remains in the stomach. The enteric coating is chosen among pharmaceutically acceptable enteric-coating materials e.g. beeswax, shellac or anionic film-forming polymers such as cellulose acetate phthalate, hydroxypropylmethylcellulose phthalate, partly methyl esterified methacrylic acid polymers and the like, if preferred in combination with a suitable plasticizer. To this coating various dyes may be added in order to distinguish among tablets or granules with different active compounds or with different amounts of the active compound present.

Soft gelatine capsules may be prepared with capsules containing a mixture of the active compound or compounds of the invention, vegetable oil, fat, or other suitable vehicle for soft gelatine capsules. Soft gelatine capsules may also be enteric-coated as described above. Hard gelatine capsules may contain

granules or enteric-coated granules of the active compound. Hard gelatine capsules may also contain the active compound in combination with a solid powdered carrier such as lactose, saccharose, sorbitol, mannitol, potato starch, amylopectin, cellulose derivatives or gelatine. The hard gelatine capsules may be enteric-coated as described above.

Dosage units for rectal administration may be prepared in the form of suppositories which contain the active substance mixed with a neutral fat base, or they may be prepared in the form of a gelatine rectal capsule which contains the active substance in a mixture with a vegetable oil, paraffin oil or other suitable vehicle for gelatine rectal capsules, or they may be prepared in the form of a ready-made micro enema, or they may be prepared in the form of a dry micro enema formulation to be reconstituted in a suitable solvent just prior to administration.

Liquid preparation for oral administration may be prepared in the form of syrups or suspensions, e.g solutions or suspensions containing from 0.2% to 20% by weight of the active ingredient and the remainder consisting of sugar or sugar alcohols and a mixture of ethanol, water, glycerol, propylene glycol and polyethylene glycol. If desired, such liquid preparations may contain colouring agents, flavouring agents, saccharine and carboxymethyl cellulose or other thickening agents. Liquid preparations for oral administration may also be prepared in the form of a dry powder to be reconstituted with a suitable solvent prior to use.

Solutions for parenteral administration may be prepared as a solution of a compound of the invention in a pharmaceutically acceptable solvent, preferably in a concentration from 0.1% to 10% by weight. These solutions may also contain stabilizing agents and/or buffering agents and may be manufactured in different unit dose ampoules or vials. Solutions for parenteral administration may also be prepared as a dry preparation to be reconstituted with a suitable solvent extemporaneously before use.

The typical daily dose of the active substance varies within a wide range and will depend on various factors such as for example the individual requirement of each patient, the route of administration and the disease. In general, oral and parenteral dosages will be in the range of 5 to 500 mg per day of active substance.

The invention is illustrated by the following examples.

Example 10

**Preparation of Phosphoric acid, benzyl[2-[(4-methoxy-3,5-dimethyl-2-pyridinyl)methyl sulfinyl]-1H-benzimidazole-1-yl]methyl diester, sodium salt. (Method E)**

Phosphoric acid dibenzyl[2-[(4-methoxy-3,5-dimethyl-2-pyridinyl) methyl sulfinyl]-1H-benzimidazole-1-yl]methyl triester (90 mg, 0.15 mmoles) was dissolved in methanol/water (2 ml, 1:1). Sodium hydrogen carbonate (58 mg, 0,7 mmoles) was added and the mixture was refluxed for 2 hours on a waterbath. Concentration of the mixture at reduced pressure and chromatography of the residue on silica gel (ethyl acetate-methanol-water; 20:4:3) gave the essentially pure title compound.
Yield: 30 mg (37 %).

Examples 29 and 32

**a) Preparation of mixture of phosphoric acid, [6-methoxy-2-[(4-methoxy-3,5-dimethyl-2-pyridinyl)-methyl sulfinyl]-1H-benzimidazole-1-yl]methyl ester, di-sodium salt and phosphoric acid, [5-methoxy-2-[(4-methoxy-3,5-dimethyl-2-pyridinyl)methyl sulfinyl]-1H-benzimidazole-1-yl]methyl ester, disodium salt, 2:1 (Method B)**

Tributylamine (14 ml, 0.059 mol) was added with stirring to a solution of phosphoric acid, 85 per cent (2 ml, 0.030 mol) in ethanol (10 ml). The solvent was evaporated and the residue taken up in methylene chloride (25 ml). The organic phase was dried over sodium sulphate, filterered and evaporated. A mixture of 1-chloromethyl-6-methoxy-2-[(4-methoxy-3,5-dimethyl-2-pyridinyl)methyl sulfinyl]-1H-benzimidazole and 1-chloromethyl-5-methoxy-2-[(4-methoxy-3,5-dimethyl-2-pyridinyl)methyl sulfinyl]-1H-benzimidazole, 2:1 (1.0 g, 0.0025 mol) and the tributylammonium salt of phosphoric acid, prepared above, were dissolved in methylene chloride (50 ml). The methylene chloride was distilled off and the residue was heated on a waterbath for 5 minutes at 60 °C. The residue was dissolved in methylene chloride (50 ml) and again the methylene chloride was distilled off and the oily product mixture was heated on a waterbath for 5 minutes at 60 °C. This procedure was repeated four times until the reaction was completed. The residue was dissolved in methylene chloride and washed with three portions of water. The organic layer was dried, filtered and

evaporated. The crude mixture of tributylammonium salts was dissolved in methylene chloride (25 ml) and water (10 ml) added. A solution of sodium hydroxide was added with stirring while pH of the aqueous layer was carefully controlled. When pH reached 9.0-9.5 in the aqueous phase (0.005 mol NaOH added) the mixture was centrifuged. The aqueous layer was washed with three portions (25 ml) of methylene chloride and then freeze dried to give 760 mg, 61 % of the title compounds. NMR spectrum of the mixture was in accordance with the spectra of the pure isomers given in Table 2.

**b) Preparation of Phosphoric acid, [5-methoxy-2-[(4-methoxy-3,5-dimethyl-2-pyridinyl) methyl sulfinyl]-1-H-benzimidazole-1-yl] methyl ester, disodium salt**

From the mixture of tributyl, ammonium. salts prepared in step a) above phosphoric acid, [5-methoxy-2-[(4-methoxy-3,5-dimethyl-2-pyridinyl) methyl sulfinyl]-1H-benzimidazole-1-yl]methyl ester, tributylammonium salt was obtained through chromatography on a reversed phase column, eluted with 15 per cent acetonitrile in water. After freeze drying the compound was dissolved in methylene chloride acid treated with sodiumhydroxide as described above. Pure phosphoric acid, [5-methoxy-2-[(4-methoxy-3,5-dimethyl-2-pyridinyl)methyl sulfinyl]-1-H-benzimidazole-1-yl]methyl ester, disodium salt was obtained in about 1 % yield. NMR spectrum in Table 2.

**c) Preparation of Phosphoric acid, [6-methoxy-2-[(4-methoxy-3,5-dimethyl-2-pyridinyl)methyl sulfinyl]-1H-benzimidazole-1-yl]methyl ester, di-sodium salt**

Method B

The title compound was prepared as described in step a) above for the isomeric mixture, starting from pure 1-chloromethyl-6-methoxy-2-[(4-methoxy 3,5-dimethyl-2-pyridinyl)methyl sulfinyl]-1H-benzimidazole (1.9 g, 0.0048 mol), phosphoric acid, 85 per cent (4 ml, 0.060 mol), tributyl amine (28 ml, 0.118 mol) and 100 ml methylene chloride. The product was crystallized from an ethanol-water mixture giving 0.85 g, 35 h. NMR spectrum of the compound was identical with the spectrum given in Table 2.

Method E

To the residue from the preparation of Phosphoric acid, cyanoethyl[6-methoxy-2-[(4-methoxy-3,5-dimethyl-2-pyridinyl)methyl sulfinyl]-1H-benzimidazole-1-yl]methyl diester, triethylammonium salt was added a solution of NaOH (0.30 g, 0.0075 mol) dissolved in water (25 ml). After heating the mixture on a waterbath (60 °C) for 15 minutes it was washed twice with methylene chloride (25 ml) and evaporated to dryness, giving the desired compund (0.25 g). The identity of the product was confirmed with NMR. Yield 22 %.

Example 33

**Preparation of Phosphoric acid, ethyl[6-methoxy-2-[(4-methoxy-3,5-dimethyl-2-pyridinyl)methyl sulfinyl] 1H-benzimidazole-1-yl] methyl diester, sodium salt**

Pure 1-chloromethyl-6-methoxy-2[(4-methoxy-3,5-dimethyl-2-pyridinyl)-methyl sulfinyl]1H-benzimidazole (0.3 g, 0.00076 mol) and the mono-triethylammonium salt of phosphoric acid mono ethyl ester (0.5 g, 0.0022 mol) were mixed together with triethyl amine (0.3 ml) and methylene chloride (10 ml). The solvent was evaporated and the residue was heated on a water bath at 60 °C for 5 minutes. Methylene chloride was added, distilled off and the product was again heated for 5 minutes at 60 °C. This procedure was repeated four times until the reaction was completed. The crude material was purified by chromatography on a reversed phase column with water-acetonitrile 90:10 as eluent. The pure fractions were combined, evaporated and one equivalent of sodium hydroxide was added. The solution was evaporated and dried in vacuum, yielding 0.37 g, 26 % of pure product. The title compound wad identified with NMR.

Example 45

**Preparation of Phosphoric acid, p-nitrophenyl[2-[(4-methoxy-3,5-dimethyl-2-pyridinyl)methyl sulfinyl]-1H-benzimidazole-1-yl]methyl diester, sodium salt**

1-Chloromethyl-2-[(4-methoxy-3,5-dimethyl-2-pyridinyl)methyl sulfinyl]-1H-benzimidazole (0.22 g, 0.60

mmol), disodium-p-nitrophenylphosphate x $6H_2O$ (2.4 g, 6.4 mmol) were dissolved in 15 ml of acetonitrile containing 10 ml of water. The solution was refluxed for 1 hour. The solvents were then removed in vacuum and the residue dissolved in water. The aqueous layer was washed with methylene chloride and then evaporated. The crude material was purified by flash chromatography on silica with ethylacetate-methanol-water (20:4:3) as eluent, giving 32 mg (10 %) of pure title compound.

Example 54

**Preparation of Phosphoric acid, ethyl[2-[(4-(2,2,2 trifluoro ethoxy)-3-methyl-2-pyridinyl)methyl sulfinyl]-1H-benzimidazole-1-yl]methyl diester, sodium salt**

1-chloromethyl-2-[(4-(2,2,2-trifluoroethoxy)-3-methyl-2-pyridinyl)methyl sulfinyl]1-H-benzimidazole (40.5 mg, 0.1 mmol) and the di-tributylammonium salt of phosphoric acid mono ethyl ester (198 mg, 0.4 mmol) were mixed together with tributyl amine (19 mg, 0.1 mmol) and methylene chloride (10 ml). The solvent was evaporated and the residue heated on a water bath at 60 ° C for 5 min. Methylene chloride was added, distilled off and the product was again heated for 5 minutes at 60 ° C. This procedure was repeated four times until the reaction was completed. The residue was dissolved in methylene chloride and washed with three portions of water. The organic layer was dried, filtered and evaporated. The crude mixture of tributylammonium salts was dissolved in methylene chloride and water was added. A solution of sodiumhydroxide was added until pH = 10. The mixture was centrifuged and the aqueous layer washed with three portions of methylene chloride. Freeze drying gave the pure title compound as identified by NMR (Table 2).

Example 57 and 58

**Preparation of a mixture of Phosphoric acid, [6-methoxy-[2-[(4-isopropyloxy-2-pyridinyl) methyl sulfinyl]-1H-benzimidazole-1-yl]methyl ester, dipotassium salt and Phosphoric acid, [5-methoxy-2-[4-isopropyloxy-2-pyridinyl)methyl sulfinyl]-1H-benzimidazole-1-yl]methyl ester, dipotassium salt.**

The title compounds were prepared as described in examples 29 and 32 but with potassium hydroxide instead of sodium hydroxide.

Example 63 and 64

**Preparation of a mixture of Phosphoric acid, methyl[6-methoxy-2-[(4-isopentyloxy-3,5-dimethyl-2-pyridinyl)methyl sulfinyl]-1H-benzimidazole-1-yl]methyl diester, sodium salt and Phosphoric acid, methyl[5-methoxy-2-[(4-isopentyloxy-3,5-dimethyl-2-pyridinyl)methyl sulfinyl]-1H-benzimidazole-1-yl]methyl diester, sodium salt.**

The title compounds were prepared as described in example 54, but with the di-tributylammonium salt of phosphoric acid monomethyl ester.

Examples 75 and 76

These compounds are identified in Table 1 and were prepared as exemplified in Example 54, with the exception that potassium hydroxide was used instead of sodium hydroxide.

Preparation of intermediates

**[2-[(4-Methoxy-3,5-dimethyl-2-pyridinyl)methyl sulfinyl]-1H-benzimidazole-1-yl] methanol.**

2-[(4-Methoxy-3,5-dimethyl-2-pyridinyl)methyl sulfinyl]-1H-benzimidazole (3.15 g, 10 mmoles) and N,N-dimethylaminopyridine (120 mg, 1 mmol) was dissolved in methylene chloride (50 ml). A solution of formaldehyde (5 M, 10 ml, 50 mmol ) was added and the mixture was stirred violently for 2 minutes. The phases were separated and the methylene chloride solution was dried (sodium sulphate), filtered and evaporated to dryness. The slightly red residue was the title compound as an essentially pure oil.
NMR (500 MHz, $CDCl_3$, ):2.15, 2.27, 3.70, 4,89, 5.89, 7.33, 7.63, 7.96

**1-Hydroxymethyl-(5-methoxy) and 1-hydroxymethyl-(6-methoxy)-2-[(4-methoxy-3,5-dimethyl-2-**

**pyridinyl)methyl sulfinyl]-1H-benzimidazole.**

5-methoxy-2-[(4-methoxy-3,5-dimethyl-2-pyridinyl)methyl sulfinyl]-1H-benzimidazole (34.5 g 0.1 mol) was dissolved in methylenechloride (500 ml). A solution of formaldehyde (5 M, 100 ml, 0.5 mol) was added and the mixture was stirred violently for 2 minutes. The phases were separated and the methylene chloride solution was dried (sodium sulfate) filtered and evaporated to dryness at low temperature (<30 °C). The slightly red residue was an isomeric mixture (ratio 1:2) of the title compounds and an essentially pure oil. This oil was used without purification in the subsequent reaction.

Example I 12

**1-Chloromethyl-2-[(4-methoxy-3,5-dimethyl-2-pyridinyl)methyl sulfinyl]-1H-benzimidazole**

[2-[(4-Methoxy-3,5-dimethyl-2-pyridinyl)methyl sulfinyl]-1H-benzimidazol-1-yl]methanol (4.8 g, 14 mmoles) and triethyl amine (1.6 g, 15 mmoles) was dissolved in methylene chloride (100 ml). A solution of thionyl chloride (1.8 g, 15 mmoles) in methylene chloride (10 ml) was added with such a velocity that the reaction mixture refluxed gently. After 10 minutes at room temperature the methylene chloride was distilled off at reduced pressure and the residue was taken up in ethyl acetate (100 ml) and water (50 ml). The phases were separated and the ethyl acetate phase was dried over sodium sulphate and evaporated. Chromatography on silicia gel (ethyl acetate) gave the crystalline title compound which was recrystallized in a ethyl acetate/diethyl ether mixture. Mp 140-141 °C. Yield 1.5 g (27 %).

The compounds according to examples I 1 - I 11, I 13 - I 20, I 23- I 28 and I 30 - I 39 have been prepared by the method illustrated in example I 12.

Examples I 21 and I 22

**1-chloromethyl-(5-methoxy) and (6-methoxy)-2-[(4-methoxy-3,5-dimethyl-2-pyridinyl) methyl sulfinyl]-1H-benzimidazole.**

1-hydroxy methyl-(5-methoxy)and-(6-methoxy-2-[(4-methoxy-3,5-dimethyl-2-pyridinyl) methyl sulfinyl]-1H-benzimidazole (30 g 0.08 mol ratio 1:2) was dissolved in toluene (500 ml) and the solution was cooled to -30 °C. A solution of thionyl chloride (19 g 0.16 mol) in toluene (100 ml) was added dropwise at -30 °C and the mixture was stirred for 10 minutes at -30 °C. Then a solution of triethyl amine (45 g 0.45 mol) in toluene (200 ml) was added dropwise. After the addition the temperature was raised and the mixture stirred at room temperature for 30 minutes. The mixture was evaporated and the residue (120 g) was chromatographed on silica gel (ethylacetate-methylenechloride 50-50)giving the title compound as an isomeric mixture (ratio 1:3) Yield 11.9 g.

NMR (500 MHz, $CDCl_3$) 2.23, 2.25, 2.26, 3.72, 3.87, 3.92, 4.88, 4.95, 4.96, 6.17, 6.18, 6.54, 6.57, 6.95, 7.01, 7.19, 7.26, 7.43, 7.67, 8.17. If desired the pure 1-chloromethyl 6-methoxy-2-[(4-methoxy-3,5-dimethyl-2-pyridinyl)methyl sulfinyl]-1H-benzimidazole was obtained when the isomeric mixture was crystallized in acetonitrile.

NMR (500 MHz, $CDCl_3$) 2.23, 2.25, 3.72, 3.92, 4.88, 4.96, 6.17, 6.57, 6.95, 7.01, 7.67, 8.17.

**Preparation of Phosphoric acid, cyanoethyl[6-methoxy-2-[(4-methoxy-3,5-dimethyl-2-pyridinyl)-methyl sulfinyl]-1H-benzimidazole-1-yl]methyl diester, triethylammonium salt (Method B)**

1-Chloromethyl-6-methoxy-2[(4-methoxy-3,5-dimethyl-2-pyridinyl)methyl sulfinyl]-1H-benzimidazole (0.90 g, 0.0023 mol) was added under stirring to a solution of mono-triethylammonium salt of phosphoric acid cyanoethyl ester (0.70 g, 0.0028 mol) and triethyl amine (0.65 g, 0.0064 mol) in methylene chloride (20 ml). The mixture was refluxed overnight. The methylene chloride was distilled off and the residue was heated on a waterbath for 10 minutes at 60 °C and then used without further purification in the subsequent reaction.

**Preparation of Phosphoric acid, dibenzyl[2-[(4-methoxy-3,5-dimethyl-2-pyridinyl)methyl sulfinyl]-1H-benzimidazole-1-yl]methyl triester**

1-Chloromethyl-2-[4-methoxy-3,5-dimethyl-2-pyridinyl)methyl sulfinyl]-1H-benzimidazole (1.29 g, 3,5 mmoles) and the silversalt of phosphoric acid dibenzylester (1.79 g, 4,6 mmoles) was suspended in dry

toluene. The mixture was heated on the water-bath at 90 °C for 3 hours, whereafter the toluene was distilled off at reduced pressure. The residue was taken up in methylene chloride (50 ml). Filtration (Hyflo) and evaporation of methylene chloride from the filtrate gave an oily product mixture which was chromatographed on silica gel (ethylacetate).
Yield: 0,9 (42 %) of the essentially pure title compound as an oil.
NMR (500 MHz, ($CDCl_3$ $\delta$): 2.20 (ds, 6H), 3.65 (s,3H), 4.8-5.0 (m,6H), 6.25-6.3 (m,1H), 6.45-6.5 (m,1H), 7.15-7.40 (m,12H), 7.65 (d,1H), 7.80 (d,1H), 8.1 (s,1H).

Isomeric forms.

The isomeric identity of the pure example no. 29 was assigned by using NOE NMR techniques on its synthetic precursor no. I 22. All the other benzimidazole isomers have been structurally assigned by relating their NMR-spectra to that of example no. 29.

The preparation of the precursors no. I 21 and I 22 results in the formation of an isomeric mixture in the ratio 1:2. As a consequence, the target compounds no. 32 and 29 are formed as isomers in the same ratio 1:2. In the other examples, the isomeric ratios are sometimes different from 1:2 and are dependent on the substituents, especially the substituents on the benzimidazole nucleus.

A summary of the examples follows in Table 1 below.

Table 1. Summary of examples

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 9 | SO | H | H | $OCH_3$ | H | 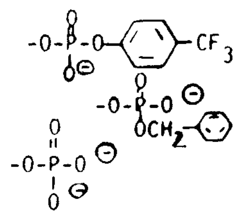 | $CH_3$ | $OCH_3$ | $CH_3$ | H | | | | |
| 10 | SO | H | H | H | H | | $CH_3$ | $OCH_3$ | $CH_3$ | H | (10) | 37 | NMR | Sodium salt |
| 11 | SO | H | H | H | H | | $CH_3$ | $OCH_3$ | $CH_3$ | H | B (29) | 20 | NMR | Sodium salt |
| 28 | SO | H | H | $OCH_3$ | H | 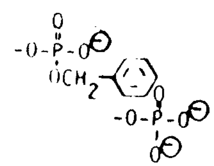 | $CH_3$ | $OCH_3$ | $CH_3$ | H | | | | |
| 29 | SO | H | H | $OCH_3$ | H | | $CH_3$ | $OCH_3$ | $CH_3$ | H | E (29)<br>B (29) | 22<br>35 | NMR | Sodium salt<br>Sodium salt |
| 32 | SO | H | $OCH_3$ | H | H | 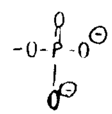 | $CH_3$ | $OCH_3$ | $CH_3$ | H | B 32 | 1 | NMR | Sodium salt |

Table 1. cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 33 | SO | H | H | $OCH_3$ | H | 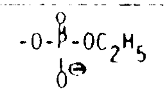 | $CH_3$ | $OCH_3$ | $CH_3$ | H | B (33) | 26 | NMR | Sodium salt |
| 36 | SO | H | H | H | H | 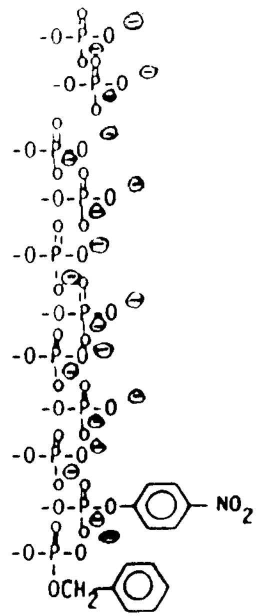 | $CH_3$ | $OCH_2CF_3$ | H | H | B (29) | | NMR | Sodium salt |
| 37 | SO | $CH_3$ | $OCH_2CH_2OCH_3$ | $CH_3$ | H | | H | $CH_3$ | $CH_3$ | H | B (29) | | NMR | Sodium salt |
| 38 | SO | H | $CH_3$ | $OCH_2CH_2OCH_3$ | $CH_3$ | | H | $CH_3$ | $CH_3$ | H | | | | |
| 39 | SO | H | 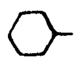 | H | H | | H | $CH_3$ | $CH_2CH_3$ | H | B (29) | | NMR | Sodium salt |
| 40 | SO | H | H | 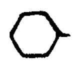 | H | | H | $CH_3$ | $CH_2CH_3$ | H | B (29) | | NMR | Sodium salt |
| 41 | SO | H | $CO_2CH_3$ | $CH_3$ | H | | $CH_3$ | $CH_3$ | H | H | B (29) | | NMR | Sodium salt |
| 42 | SO | H | $CH_3$ | $CO_2CH_3$ | H | | $CH_3$ | $CH_3$ | H | H | B (29) | | NMR | Sodium salt |
| 43 | SO | H | $CH(CH_3)_2$ | H | H | | $CH_3$ | H | $CH_3$ | H | B (29) | | NMR | Sodium salt |
| 44 | SO | H | H | $CH(CH_3)_2$ | H | | $CH_3$ | H | $CH_3$ | H | B (29) | | NMR | Sodium salt |
| 45 | SO | H | H | H | H | | $CH_3$ | $OCH_3$ | $CH_3$ | H | B (45) | 10 | NMR | Sodium salt |
| 46 | SO | H | H | $OCH_3$ | H | | $CH_3$ | $OCH_3$ | $CH_3$ | H | B (10) | | NMR | Sodium salt |

cont.

Table 1. cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 47 | SO | H | $OCH_3$ | H | H | $-O-P(O)(O^{\ominus})O^{\ominus}$ | $CH_3$ | $OCH_2CH_2CH(CH_3)_2$ | $CH_3$ | H | B (29) | | NMR | Sodium salt |
| 48 | SO | H | H | $OCH_3$ | H | $-O-P(O)(O^{\ominus})O^{\ominus}$ | $CH_3$ | $OCH_2CH_2CH(CH_3)_2$ | $CH_3$ | H | B (29) | | NMR | Sodium salt |
| 49 | SO | H | 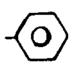 | H | H | $-O-P(O)(O^{\ominus})O^{\ominus}$ | $CH_3$ | $OCH_3$ | $CH_3$ | H | B (29) | | NMR | Sodium salt |
| 50 | SO | H | H | 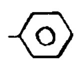 | H | $-O-P(O)(O^{\ominus})O^{\ominus}$ | $CH_3$ | $OCH_3$ | $CH_3$ | H | B (29) | | NMR | Sodium salt |
| 51 | SO | $OCH_3$ | H | H | $OCH_3$ | $-O-P(O)(O^{\ominus})O^{\ominus}$ | $CH_3$ | $OCH_3$ | $CH_3$ | H | B (29) | | NMR | Sodium salt |
| 52 | SO | H | Br | H | H | $-O-P(O)(O^{\ominus})O^{\ominus}$ | $CH_3$ | $OCH_2CH=CH_2$ | $CH_3$ | H | B (29) | | NMR | Sodium salt |
| 53 | SO | H | H | Br | H | $-O-P(O)(O^{\ominus})O^{\ominus}$ | $CH_3$ | $OCH_2CH=CH_2$ | $CH_3$ | H | B (29) | | NMR | Sodium salt |
| 54 | SO | H | H | H | H | $-O-P(O)(O^{\ominus})OCH_2CH_3$ | $CH_3$ | $OCH_2CF_3$ | H | H | B (54) | | NMR | Sodium salt |
| 55 | SO | H | Br | H | H | $-O-P(O)(O^{\ominus})OCH_2CH_3$ | $CH_3$ | $OCH_2CH=CH_2$ | $CH_3$ | H | B (54) | | NMR | Sodium salt |
| 56 | SO | H | H | Br | H | $-O-P(O)(O^{\ominus})OCH_2CH_3$ | $CH_3$ | $OCH_2CH=CH_2$ | $CH_3$ | H | B (54) | | NMR | Sodium salt |
| 57 | SO | H | $OCH_3$ | H | H | $-O-P(O)(O^{\ominus})-O^{\ominus}$ | H | $O-CH(CH_3)_2$ | H | H | B (57) | | NMR | Potassium salt |
| 58 | SO | H | H | $OCH_3$ | H | $-O-P(O)(O^{\ominus})O^{\ominus}$ | H | $O-CH(CH_3)_2$ | H | H | B (57) | | NMR | Potassium salt |
| 59 | SO | $CH_3$ | $OCH_2CH_2OCH_3$ | $CH_3$ | H | $-O-P(O)(O^{\ominus})OCH_2CH_3$ | H | $CH_3$ | $CH_3$ | H | B (54) | | NMR | Sodium salt |
| 60 | SO | H | $CH_3$ | $OCH_2CH_2OCH_3$ | $CH_3$ | $-O-P(O)(O^{\ominus})OCH_2CH_3$ . | H | $CH_3$ | $CH_3$ | H | | | | |

cont.

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 61 | SO | H | 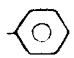 | H | H | $-O-P(O)(O^{\ominus})OCH_2CH_3$ | $CH_3$ | $OCH_3$ | $CH_3$ | H | B (54) | | NMR | Sodium salt |
| 62 | SO | H | H | 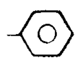 | H | $-O-P(O)(O^{\ominus})OCH_2CH_3$ | $CH_3$ | $OCH_3$ | $CH_3$ | H | B (54) | | NMR | Sodium salt |
| 63 | SO | H | $OCH_3$ | H | H | $-O-P(O)(O^{\ominus})OCH_3$ | $CH_3$ | $OCH_2CH_2CH(CH_3)_2$ | $CH_3$ | H | B (63) | | NMR | Sodium salt |
| 64 | SO | H | H | $OCH_3$ | H | $-O-P(O)(O^{\ominus})OCH_3$ | $CH_3$ | $OCH_2CH_2CH(CH_3)_2$ | $CH_3$ | H | B (63) | | NMR | Sodium salt |
| 65 | SO | H | $OCH_3$ | H | H | $-O-P(O)(O^{\ominus})O^{\ominus}$ | $CH_3$ | $OCH_2CH_2OCH_3$ | $CH_3$ | H | B (57) | | NMR | Potassium salt |
| 66 | SO | H | H | $OCH_3$ | H | $-O-P(O)(O^{\ominus})O^{\ominus}$ | $CH_3$ | $OCH_2CH_2OCH_3$ | $CH_3$ | H | B (57) | | NMR | Potassium salt |
| 69 | SO | H | $CH_2CH_2OCH_3$ | H | H | $-O-P(O)(O^{\ominus})O^{\ominus}$ | $CH_3$ | $OCH_3$ | $CH_3$ | H | B (57) | | NMR | Potassium salt |
| 70 | SO | H | H | $CH_2CH_2OCH_3$ | H | $-O-P(O)(O^{\ominus})^{\ominus}$ | $CH_3$ | $OCH_3$ | $CH_3$ | H | B (57) | | NMR | Potassium salt |
| 71 | SO | H | $CH(CH_3)_2$ | H | H | $-O-P(O)(OCH_3)O^{\ominus}$ | $CH_3$ | H | $CH_3$ | H | B (67) | | NMR | Potassium salt |

cont.

Table 1. cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 72 | SO | H | H | $CH(CH_3)_2$ | H | $O-P(O)O^{\ominus}$ $OCH_3$ | $CH_3$ | H | $CH_3$ | H | B (67) | | NMR | Potassium salt |
| 73 | SO | H | $OCH_3$ | H | H | $O-P(O)O^{\ominus}$ $OCH_2CH_3$ | $CH_3$ | $OCH_2CH_2OCH_3$ | $CH_3$ | H | B (54) | | NMR | Sodium salt |
| 74 | SO | H | H | $OCH_3$ | H | $O-P(O)O^{\ominus}$ $OCH_2CH_3$ | $CH_3$ | $OCH_2CH_2OCH_3$ | $CH_3$ | H | B (54) | | NMR | Sodium salt |
| 75 | SO | H | $CH_2CH_2OCH_3$ | H | H | $-OP(O)CH_2CH_3$ $O^{\ominus}$ | $CH_3$ | $OCH_3$ | $CH_3$ | H | B (75) | | NMR | Potassium salt |
| 76 | SO | H | H | $CH_2CH_2OCH_3$ | H | $-OP(O)OCH_2CH_3$ $O^{\ominus}$ | $CH_3$ | $OCH_3$ | $CH_3$ | H | B (76) | | NMR | Potassium salt |
| 77 | SO | H | $OCH_3$ | H | H | $-OP(O)O^{\ominus}$ $OCH_2-$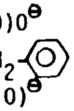 | $CH_3$ | $OCH_3$ | $CH_3$ | H | | | | Sodium salt |
| 78 | SO | H | $OCF_2CHF_2$ | H | H | $O-P(O)^{\ominus}$ $OCH_2CH_3$ | H | $OCH_3$ | H | H | | | | Sodium salt |
| 79 | SO | H | H | $OCF_2CHF_2$ | H | $O-P(O)O^{\ominus}$ $OCH_2CH_3$ | H | $OCH_3$ | H | H | | | | Sodium salt |
| 80 | SO | H | $OCF_2CHF_2$ | H | H | $O-P(O)O^{\ominus}$ $O^{\ominus}$ | H | $OCH_3$ | H | H | | | | Sodium salt |
| 81 | SO | H | H | $OCF_2CHF_2$ | H | $O-P(O)O^{\ominus}$ $O^{\ominus}$ | H | $OCH_3$ | H | H | | | | Sodium salt |

cont.

Table 1. cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 82 | S | H | H | H | H | $-OP(O)O^{\ominus}$<br>$O^{\ominus}$ | $CH_3$ | $OCH_3$ | $CH_3$ | H | B (57) | | NMR | Potassium salt |
| 83 | S | H | H | H | H | $-OP(O)OCH_2CH_3$<br>$O^{\ominus}$ | $CH_3$ | $OCH_3$ | $CH_3$ | H | B (54) | | NMR | Sodium salt |
| 84 | SO | H | $OCH_2CH_2$- 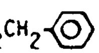 | H | H | $-OP(O)OCH_3$<br>$O^{\ominus}$ | $CH_3$ | $OCH_3$ | $CH_3$ | H | B (67) | | NMR | Potassium salt |
| 85 | SO | H | H | $OCH_2CH_2$- 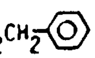 | H | $-OP(O)OCH_3$<br>$O^{\ominus}$ | $CH_3$ | $OCH_3$ | $CH_3$ | H | B (67) | | NMR | Potassium salt |
| 86 | SO | H | -CO- 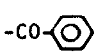 | H | H | $-OP(O)O^{\ominus}$<br>$O^{\ominus}$ | $CH_3$ | $OCH_3$ | $CH_3$ | H | B (57) | | NMR | Potassium salt |
| 87 | SO | H | H | -CO- 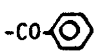 | H | $-OP(O)O^{\ominus}$<br>$O^{\ominus}$ | $CH_3$ | $OCH_3$ | $CH_3$ | H | B (57) | | NMR | Potassium salt |
| 88 | SO | H | $OCH_3$ | H | H | $-OP(O)O^{\ominus}$<br>$O^{\ominus}$ | H | $OCH_2$- 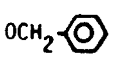 | $CH_2CH_3$ | H | B (57) | | NMR | Potassium salt |
| 89 | SO | H | H | $OCH_3$ | H | $-OP(O)O^{\ominus}$<br>$O^{\ominus}$ | H | $OCH_2$- 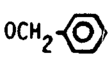 | $CH_2CH_3$ | H | B (57) | | NMR | Potassium salt |

Table 1 cont. Further illustrative examples of compounds of the invention.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 91 | SO | H | H | H | H | $O-P(O)O^{\ominus}$<br>$O^{\ominus}$ | $CH_3$ | 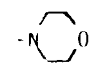 | $CH_3$ | H | | | | Potassium salt |
| 92 | SO | H | H | H | H | $O-P(O)O^{\ominus}$<br>$O^{\ominus}$ | $CH_3$ | $SC_2H_5$ | H | H | | | | Potassium salt |
| 93 | SO | H | H | H | H | $O-P(O)OC_2H_5$<br>$O^{\ominus}$ | $CH_3$ | $SC_2H_5$ | H | H | | | | Potassium salt |
| 94 | SO | H | H | H | H | $O-P(O)O^{\ominus}$<br>$O^{\ominus}$ | $-CH_2CH_2S-$ | | H | H | | | | Sodium salt |
| 95 | SO | H | $OCH_3$ | F | H | $O-P(O)OC_2H_5$<br>$O^{\ominus}$ | H | $OCH_3$ | $C_2H_5$ | H | | | | Sodium salt |
| 96 | SO | H | F | $OCH_3$ | H | $O-P(O)OC_2H_5$<br>$O^{\ominus}$ | H | $OCH_3$ | $C_2H_5$ | H | | | | Sodium salt |
| 97 | SO | H | F | H | H | $O-P(O)O^{\ominus}$<br>$O^{\ominus}$ | H | $OCH_3$ | $n-C_4H_9$ | H | | | | Potassium salt |
| 98 | SO | H | H | $OCH_3$ | H | $O-P(O)OCH_3$<br>$O^{\ominus}$ | H | $OCH_3$ | $n-C_5H_{11}$ | H | | | | Sodium salt |

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 104 | SO | H | H | $OCF_3$ | H | $-O-P(O)-O^{\ominus}$<br>$O^{\ominus}$ | H | $OCH_3$ | H | H | | | | Sodium salt |
| 105 | SO | H | H | $OCF_3$ | H | $-O-P(O)O^{\ominus}$<br>$O^{\ominus}$ | $CH_3$ | $OCH_3$ | H | H | | | | Sodium salt |
| 106 | SO | H | H | $OCF_3$ | H | $-O-P(O)OCH_3$<br>$O^{\ominus}$ | H | $OCH_3$ | $CH_3$ | H | | | | Sodium salt |
| 110 | SO | H | H | H | H | $-O-P(O)-O^{\ominus}$<br>$O^{\ominus}$ | $CH_3$ | 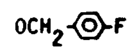 | H | H | | | | Potassium salt |
| 111 | SO | H | H | $OCH_3$ | H | $-O-P(O)-O^{\ominus}$<br>$O^{\ominus}$ | $CH_3$ | 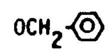 | H | H | | | | Sodium salt |
| 112 | SO | H | H | H | H | $-O-P(O)-O^{\ominus}$<br>$O^{\ominus}$ | $CH_3$ | 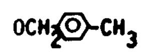 | H | H | | | | Sodium salt |
| 113 | SO | H | H | H | H | $-O-P(O)-OCH_3$<br>$O^{\ominus}$ | $CH_3$ | 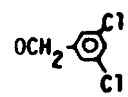 | H | H | | | | Sodium salt |
| 114 | SO | H | H | $CH_3$ | H | $-O-P(O)OC_2H_5$<br>$O^{\ominus}$ | $CH_3$ | 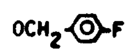 | H | H | | | | Sodium salt |

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 115 | SO | H | H | H | H | $-O-P(O)O^{\ominus}$<br>$O^{\ominus}$ | $CH_3$ | O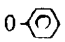 | H | H | | | | Potassium salt |
| 116 | SO | H | H | $CH_3$ | H | $-O-P(O)O^{\ominus}$<br>$O^{\ominus}$ | H | $OCH_2$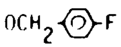 | $CH_3$ | H | | | | Potassium salt |
| 117 | SO | H | H | H | H | $-O-P(O)OC_2H_5$<br>$O^{\ominus}$ | $CH_3$ | $OCH_2$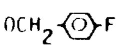 | H | H | | | | Sodium salt |
| 118 | SO | H | H | H | H | $-O-P(O)-O^{\ominus}$<br>$O^{\ominus}$ | H | $OCH_2CF_3$ | H | H | | | | Sodium salt |
| 119 | SO | H | H | H | H | $-O-P(O)-O^{\ominus}$<br>$O^{\ominus}$ | $CH_3$ | $OCH_2CF_2CF_3$ | H | H | | | | Potassium salt |
| 120 | SO | H | H | H | H | $-O-P(O)-OC_2H_5$<br>$O^{\ominus}$ | $CH_3$ | $OCH_2CF_2CHF_2$ | H | H | | | | Potassium salt |
| 121 | SO | H | H | $OCH_3$ | H | $-O-P(O)-O^{\ominus}$<br>$O^{\ominus}$ | $CH_3$ | $OCH_2CF_3$ | H | H | | | | Sodium salt |
| 122 | SO | H | H | H | H | $-O-P(O)-O^{\ominus}$<br>$O^{\ominus}$ | H | $O(CH_2)_2CH_3$ | H | H | | | | Potassium salt |
| 125 | SO | H | H | H | H | $-O-P(O)-O^{\ominus}$<br>$O^{\ominus}$ | $OCH_3$ | $OCH_3$ | H | H | | | | Sodium salt |

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 126 | SO | H | H | H | H | $-O-P(O)-OCH_3$<br>$O^{\ominus}$ | $OCH_3$ | $O(CH_2)_2CH_3$ | H | H | | | | Potassium salt |
| 127 | SO | H | H | $OCH_3$ | H | $-O-P(O)-OC_2H_5$<br>$O^{\ominus}$ | $OCH_3$ | $OCH_3$ | H | H | | | | Potassium salt |
| 128 | SO | H | $OCH_3$ | $OCH_3$ | H | $-O-P(O)-OC_2H_5$<br>$O^{\ominus}$ | $OCH_3$ | $OCH_3$ | H | H | | | | Sodium salt |
| 129 | SO | H | H | $OCH_3$ | H | $-O-P(O)-OCH_3$<br>$O^{\ominus}$ | $OCH_3$ | $OCH_2CF_3$ | H | H | | | | Sodium salt |
| 130 | SO | H | H | $CF_3$ | H | $-O-P(O)-O^{\ominus}$<br>$O^{\ominus}$ | $OCH_3$ | $OCH_3$ | H | H | | | | Sodium salt |
| 136 | SO | H | H | H | H | $-O-P(O)O^{\ominus}$<br>$O^{\ominus}$ | H | $SCH_3$ | $C_2H_5$ | H | | | | Sodium salt |

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 137 | SO | H | H | $OCH_3$ | H | $-O-\underset{\mid}{P}(O)OC_2H_5$ <br> $O^{\ominus}$ | H | $OCH_2-$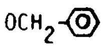 | $C_2H_5$ | H | | | | Sodium salt |
| 138 | SO | H | H | $OCH_3$ | H | $-O-\underset{\mid}{P}(O)O^{\ominus}$ <br> $O^{\ominus}$ | H | $S-$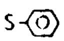 | $C_2H_5$ | H | | | | Potassium salt |
| 139 | SO | H | H | $OCH_3$ | H | $-O-\underset{\mid}{P}(O)O^{\ominus}$ <br> $O^{\ominus}$ | H | $O-$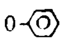 | $C_2H_5$ | H | | | | Sodium salt |
| 140 | SO | H | H | $OCH_3$ | H | $-O-\underset{\mid}{P}(O)OC_3H_7$ <br> $O^{\ominus}$ | $C_2H_5$ | $SC_4H_9$ | H | H | | | | Sodium salt |
| 141 | SO | H | H | H | H | $-O-\underset{\mid}{P}(O)OCH_3$ <br> $O^{\ominus}$ | $CH_3$ | $SCH_3$ | H | H | | | | Potassium salt |
| 144 | SO | H | H | $OCF_3$ | H | $-O-\underset{\mid}{P}(O)O^{\ominus}$ <br> $O^{\ominus}$ | $OCH_3$ | $OCH_3$ | H | H | | | | Potassium salt |
| 145 | SO | H | H | $OCH_3$ | H | $-O-\underset{\mid}{P}(O)OC_2H_5$ <br> $O^{\ominus}$ | $C_2H_5$ | $OCH_3$ | H | H | | | | Potassium salt |

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 146 | SO | H | H | $OCF_3$ | H | $-O-P(O)O^{\ominus}$<br>$O^{\ominus}$ | $OCH_3$ | $OCH_3$ | H | H | | | | Sodium salt |
| 147 | SO | H | $OCF_3$ | H | H | $-O-P(O)O^{\ominus}$<br>$O^{\ominus}$ | $OCH_3$ | $OCH_3$ | H | H | | | | Sodium salt |
| 148 | SO | H | H | $OCF_2CHF_2$ | H | $-O-P(O)O^{\ominus}$<br>$O^{\ominus}$ | $OCH_3$ | $OCH_3$ | H | H | | | | Sodium salt |
| 149 | SO | H | H | $OCF_3$ | H | $-O-P(O)OCH_2$- 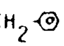<br>$O^{\ominus}$ | $CH_3$ | $OCH_3$ | $OCH_3$ | H | | | | Sodium salt |
| 152 | SO | H | $OCF_2H$ | $OCF_2H$ | H | $-O-P(O)OC_2H_5$<br>$O^{\ominus}$ | $CH_3$ | $OCH_3$ | $OCH_3$ | H | | | | Sodium salt |
| 153 | SO | H | $OCH_3$ | $OCF_2H$ | H | $-O-P(O)OCH_3$<br>$O^{\ominus}$ | $CH_3$ | $OCH_3$ | $CH_3$ | H | | | | Sodium salt |
| 154 | SO | H | H | $OCF_2CF_2H$ | H | $-O-P(O)O^{\ominus}$<br>$O^{\ominus}$ | H | $OCH_3$ | $OCH_3$ | H | | | | Sodium salt |
| 156 | SO | H | H | $OCH_3$ | H | $-O-P(O)O^{\ominus}$<br>$O^{\ominus}$ | Cl | 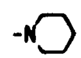 | $CH_3$ | H | | | | Sodium salt |

Table 1 cont.

| Ex. | X | $R^1$ | $R^2$ | $R^3$ | $R^4$ | D | $R^6$ | $R^7$ | $R^8$ | $R^9$ | Method (Ex. No.) | Yield % | Identifying data | Physical form |
|---|---|---|---|---|---|---|---|---|---|---|---|---|---|---|
| 157 | SO | H | H | $OCF_2CHF_2$ | H | $-O-P(O)OCH_3$<br>$O^{\ominus}$ | Cl | 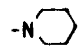 | $CH_3$ | H | | | | Sodium salt |
| 158 | SO | H | H | $OCH_3$ | H | $-O-P(O)OCH_3$<br>$O^{\ominus}$ | Cl | 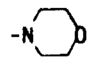 | H | H | | | | Sodium salt |
| 159 | SO | H | H | $OCH_3$ | H | $-O-P(O)O^{\ominus}$<br>$O^{\ominus}$ | H | 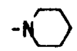 | Br | H | | | | Sodium salt |
| 160 | SO | H | $OCH_3$ | H | H | $-O-P(O)O^{\ominus}$<br>$O^{\ominus}$ | Br | 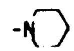 | H | H | | | | Sodium salt |
| 161 | SO | H | H | $OCH_3$ | H | $-O-P(O)O^{\ominus}$<br>$O^{\ominus}$ | $CH_3$ | 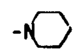 | Br | H | | | | Sodium salt |
| 162 | SO | H | H | $OCH_3$ | H | $-O-P(O)OCH_3$<br>$O^{\ominus}$ | H | 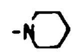 | Br | H | | | | Sodium salt |
| 163 | SO | H | H | $OCH_3$ | H | $-O-P(O)OC_2H_5$<br>$O^{\ominus}$ | Cl | 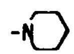 | $CH_3$ | H | | | | Sodium salt |
| 164 | SO | H | H | $OCH_3$ | H | $-O-P(O)-O^{\ominus}$<br>$O^{\ominus}$ | $CH_3$ | $OCH_3$ | $CH_3$ | $CH_3$ | | | | Sodium salt |

Identifying data for the compounds according to the examples in Table 1 are given in Table 2 below.

Table 2

| Ex. | Solvent | NMR data δ ppm (500 MHz) |
|---|---|---|
| 10 | DMSO-$d_6$ | 2.15 (s,3H), 2.20 (s,3H), 3.65 (s,3H), 4.50-4.55 (m,2H), 4.75 (d,1H), 5.05 (d,1H), 5.95-6.0 (m,1H), 6.05-6.10 (m,1H), 7.10-7.40 (m,7H), 7.75 (d,1H), 7.85 (d,1H), 8.15 (s,1H) |
| 11 | $D_2O$ | 2.00 (s,3H), 2.20 (s,3H), 3.50 (s,3H), 4.85-4.90 (m,1H), 5.00-5.05 (m,1H), 5.70-5.75 (m,1H), 5.80-5.85 (m,1H), 7.40-7.45 (m,1H), 7.50-7.55 (m,1H), 7.75 (d,1H), 7.80 (d,1H), 8.10 (s,1H). |
| 29 | $D_2O$ | 2.0 (s,3H), 2.2 (s,3H), 3.5 (s,3H), 3.95 (s,3H), 4.85-4.9 (m,1H), 5.0-5.05(m,1H), 5.65-5.7 (m,1H), 5.75-5.8 (m,1H), 7.05 (dd,1H), 7.35 (d,1H), 7.65 (d,1H), 8.1 (s,1H) |

Table 2 cont.

| Ex. | Solvent | NMR data δppm (500 MHz) |
|---|---|---|
| 32 | $D_2O$ | 1.97 (s,3H) 2.16 (s,3H), 3,47 (s, 3H), 3.87 (s,3H) 4.81-4.88 (m,1H) 4.97-5.02 (m,1H) 5.61-5.67 (m,1H), 5.70-5.75 (m,1H), 7.14 (dd,1H), 7.26 (d,1H) 7.78 (d,1H) 8.09 (s,1H) |
| 33 | $D_2O$ | 0.85 (t,3H), 1.96 (s,3H), 2.16 (s,3H) 3.43 (s,3H), 3.39-3.59 (m,2H), 3.87 (s,3H) 4.78-4.90 (m,1H), 4.97-5.05 (m,1H), 5.64-5.75 (m,2H), 7.06 (dd,1H), 7.22 (d,1H) 7.67 (dd,1H), 8.10 (s,1H) |

Table 2 cont.

| Ex. | Solvent | NMR data δ ppm (500 MHz) |
|---|---|---|
| 36 | $D_2O$ | 2.12 (s,3H), 4.63-4.73 (m,2H), 5.02(d,1H)* 5.13 (d,1H)* 5.80-5.86 (m,1H), 5.87-5.97 (m,1H), 7.00 (d,1H) 7.52 (t,1H), 7.60(t,1H) 7.83 (d,1H) 7.99 (d,1H), 8.23 (d,1H) |
| 37 | $D_2O$ | 1.85 (s,3H), 2.16 (s,3H), 2.39 (s,3H), 2.47 (s,3H) 3.52 (s,3H), 3.86-3.91 (m,2H), 4.05-4.10 (m,2H) 4.72 (d,1H)*, 4.80 (d,1H)*, 5.75 (dd1H), 5.90 (dd,1H), 6.73 (s,1H), 7.56 (s,IH), 8.09 (s,1H) |
| 39} 40} | $D_2O$ | 1.12 (s,6H), 1.24-2.00 (m,20H), 1.99 (s,6H), 2.56 (q,4H), 2.66-2.80 (m,2H), 4.81-5.99 (m,4H)* 5.68 (dd1H), 5.76 (dd1H), 5.82 (dd1H), 5.91 (dd1H) 6.76 (s,1H), 6.77 (s,1H), 7.38 (d1H), 7.49 (d,1H) 7.55 (s,1H), 7.65 (d,1H), 7.69 (s,1H), 7.76 (d1H) 8.12 (s,1H), 8.14 (s,1H) |
| 41 | $D_2O$ | 2.15 (s,3H), 2.25 (s,3H), 2.73 (s,3H), 4.01 (s,3H), 5.03 (d,1H)*, 5.10 (d,1H)*, 5.79 (dd,1H), 5.87 (dd,1H), 7.21 (d,1H), 7.80 (s,1H), 8.09 (s,1H), 8.29 (s,1H) |
| 42 | $D_2O$ | 2.15 (s,3H), 2.25 (s,3H), 2.67 (s,3H), 4.03 (s,3H), 5.02 (d,1H)*, 5.09 (d,1H)*, 5.79 (dd,1H), 5.88 (dd,1H), 7.69 (s,1H), 7.72 (d,1H), 8.13 (d,1H), 8.38 (s,1H) |

Table 2 cont.

| Ex. | Solvent | NMR data δ ppm (500 MHz) |
|---|---|---|
| 43, 44 | $D_2O$ | 1.31 (d,6H), 1.33 (d,6H), 2.19 (s,6H), 2.25 (s,6H), 3.11 (m,1H), 3.13 (m,1H), 4.91 (d2H)*, 5.03 (d2H)*, 5.76-5.82 (m,2H), 5.82-5.99 (m,2H), 7.36 (d,2H), 7.42 (dd,1H), 7.52 (dd,1H), 7.68 (d,1H), 7.72 (d,1H), 7.75 (d,1H), 7.79 (d,1H), 8.07 (d,1H), 8.08 (d,1H) |
| 45 | $D_2O$ | 2.15 (s,3H), 2.18 (s,3H), 3.67 (s,3H), 4.67 (d,1H), 5.01 (d,1H), 6.05 (t,1H), 6.16 (t,1H), 7.10 (d,2H), 7.28 (t,1H), 7.33 (t,1H), 7.69 (d,1H), 7.73 (d,1H), 7.95 (d,2H), 8.16 (s,1H) |
| 46 | $D_2O$ | 1.95 (s,3H), 2.11 (s,3H), 3.45 (s,3H), 3.79 (s,3H), 4.48-4.49 (m,2H), 4.65 (d,1H)*, 4.88 (d,1H)*, 5.72 (t,1H), 5.80 (t,1H), 6.81 (d,2H), 6.97 (d,1H), 7.09-7.21 (m,4H), 7.57 (d,1H), 8.06 (s,1H) |
| 47 | $D_2O$ | 0.87 (d,3H), 0.88 (d,3H), 1.47-1.57 (m,2H), 1.59-1.70 (m,1H), 1.91 (s,3H), 2.21 (s,3H), 3.20-3.27 (m,1H), 3.40-3.47 (m,1H), 3.95 (s,3H), 4.90 (d,1H)*, 5.10 (d,1H)*, 5.55-5.62 (m,1H), 5.65-5.72 (m,1H), 7.20 (dd,1H), 7.33 (d,1H), 7.73 (d,1H), 8.25 (s,1H) |
| 48 | $D_2O$ | 0.87 (d,3H), 0.88 (d,3H), 1.47-1.57 (m,2H), 1.59-1.70 (m,1H), 1.90 (s,3H), 2.22 (s,3H), 3.20-3.27 (m,1H), 3.40-3.47 (m,1H), 3.97 (s,3H), 4.90 (d,1H)*, 5.09 (d.1H)*, 5.55-5.62 (m,1H), 5.65-5.72 (m,1H), 7.13 (dd,1H), 7.39 (d,1H), 7.73 (d,1H), 8.25 (s,1H) |

Table 2 cont.

| Ex. | Solvent | NMR data δ ppm (500 MHz) |
|---|---|---|
| 49) 50) | $D_2O$ | 2.00 (s,3H), 2.02 (s,3H), 2.18 (s,6H), 3.46 (s,6H), 5.0 (s,)*, 5.75-5.82 (m,2H), 5.88 (dd,2H), 7.39-7.61 (m,7H), 7.65-7.92 (m,7H), 7.98 (s,1H), 8.07 (s,1H), 8.14 (s,1H), 8.15 (s,1H) |
| 51 | $D_2O$ | 1.98 (s,3H), 2.24 (s,3H), 3.51 (s,3H), 3.98 (s,3H), 4.00 (s,3H), 5.1 (s)*, 5.89-5.93 (m,2H), 6.88 (d,1H), 6.96 (d,1H), 8.18 (s,1H) |
| 52) 53) | $D_2O$ | 2.02 (s,6H), 2.23 (s,6H), 4.00-4.07 (m,2H), 4.11-4.17 (m,2H), 4.93 (d,2H)*, 5.08 (d,2H)*, 5.33 (dd,4H), 5.66-5.75 (m,2H), 5.76-5.87 (m,2H), 5.95-6.05 (m,2H), 7.61 (dd 1H), 7.67 (dd,1H), 7.70 (d,1H), 7.78 (d,1H), 8.00 (d,1H), 8.10 (d,1H), 8.18 (s,2H) |
| 54 | $D_2O$ | 0.85 (t,3H), 1.99 (s,3H), 3.42-3.58 (m,2H), 5.0 (s)*, 5.78 (dd,1H), 5.82 (dd,1H), 6.92 (d,1H), 7.45 (t,1H), 7.53 (t,1H), 7.72 (d,1H), 7.80 (d,1H), 8.16 (d,1H) |
| 55) 56) | $D_2O$ | 0.95 (t,6H), 2.10 (s,6H), 2.20 (s,6H), 3.50-3.72 (m,4H), 4.00-4.10 (m,2H), 4.10-4.20 (m,2H), 5.10 (s)*, 5.37 (d,d,4H), 5.66-5.75 (m,2H), 5.76-5.87 (m,2H), 5.95-6.05 (m,2H), 7.60-7.90 (m,4H), 8.07 (d,1H), 8.13 (d,1H), 8.18 (s,2H) |
| 57) 58) | $D_2O$ | 1.01 (d,6H), 1.02 (d,6H), 3.88 (s,3H), 3.93 (s,3H), 4.20 (m,2H), 5.0 (s)*, 5.65-5.73 (m,2H), 5.80-5.88 (m,2H), 6.67-7.13 (m,7H), 7.23-7.56 (m,3H), 8.23 (d,2H) |

Table 2 cont.

| Ex. | Solvent | NMR data δ ppm (500 MHz) |
|---|---|---|
| 59 | $D_2O$ | 0.88 (t,3H), 1.76 (s,3H), 2.06 (s,3H), 2.35 (s,3H), 2.37 (s,3H), 3.46 (s,3H), 3.49-3.60 (m,2H), 3.79-3.80 (m,2H), 3.94-3.96 (m,2H), 4.62 (d,1H), 4.82 (d,1H), 5.65 (dd 1H), 5.78 (dd 1H), 6.60 (s,1H), 7.32 (s,1H), 8.03 (s,1H) |
| 61 } 62 } | $D_2O$ | 0.95 (t,6H), 2.07 (s,6H), 2.25 (s,6H), 3.48 (s,3H), 3.51 (s,3H), 3.45-3.58 (m,2H), 3.58-3.71 (m,2H), 5.2 (s)*, 5.76-5.93 (m,4H), 7.50-7.70 (m,6H), 7.79-7.93 (m,7H), 7.97 (d,1H), 8.04 (s,1H), 8.13 (s,1H), 8.22 (s,2H) |
| 63 | $D_2O$ | 0.78 (d,3H), 0.80 (d,3H), 1.40 (t,2H), 1.55 (m,1H), 1.79 (s,3H), 2.10 (s,3H), 3.15 (d,3H), 3.85 (s,1H), 5.0 (s)*, 5.55 (dd,1H), 5.62 (dd,1H), 7.10 (dd,1H) 7.28 (d,1H), 7.54 (d,1H), 8.15 (s,1H) |
| 64 | $D_2O$ | 0.78 (d,3H), 0.80 (d,3H), 1.40 (t,2H), 1.55 (m,1H), 1.79 (s,3H), 2.10 (s,3H), 3.15 (d,3H), 3.85 (s,1H), 5.0 (s)*, 5.55 (dd,1H), 5.62 (dd,1H), 7.02 (dd,1H), 7.17 (d,1H), 7.64 (d,1H), 8.15 (s,1H) |
| 65 | $D_2O$ | 1.91 (s,3H), 2.14 (s,3H), 3.30 (s,3H), 3.44-3.62 (m,4H), 3.88 (s,3H), 4.82 (d,1H)*, 5.01 (d,1H)*, 5.56-5.66 (m,1H), 5.66-5.75 (m,1H), 7.13 (dd,1H), 7.24 (d,1H), 7.66 (d,1H), 8.12 (s,1H) |

Table 2 cont.

| Ex. | Solvent | NMR data δ ppm (500 MHz) |
|---|---|---|
| 66 | $D_2O$ | 1.91 (s,3H), 2.14 (s,3H), 3.30 (s,3H), 3.44-3.62 (m,4H), 3.90 (s,3H), 4.82 (d,1H)*, 5.01 (d,1H)*, 5.56-5.66 (m,1H), 5.66-5.75 (m,1H), 7.03 (dd,1H), 7.31 (d,1H), 7.62 (d,1H), 8.12 (s,1H) |
| 69 | $D_2O$ | 1.98 (s,3H), 2.19 (s,3H), 2.97 (m,2H), 3.38 (s,3H), 3.47 (s,3H), 3.76-3.83 (m,2H), 5.04 (s)*, 5.69-5.80 (m,2H), 7.44 (d,1H), 7.65 (s,1H), 7.76 (d,1H), 8.14 (s,1H) |
| 70 | $D_2O$ | 1.98 (s,3H), 2.19 (s,3H), 3.07 (m,2H), 3.38 (s,3H), 3.45 (s,3H), 3.76-3.87 (m,2H), 5.04 (s)*, 5.69-5.80 (m,2H), 7.36 (d,1H), 7.70 (s,1H), 7.72 (d,1H), 8.14 (s,1H) |
| 71 | $D_2O$ | 1.26 (d,6H), 2.10 (s,3H), 2.18 (s,3H), 3.03 (m,1H), 3.20 (d,3H), 4.85 (d,1H)*, 5.01 (d,1H)*, 5.74-5.84 (m,2H), 7.26 (s,1H), 7.45 (d,1H), 7.65 (d,1H), 7.68 (s,1H), 8.05 (s,1H) |

Table 2 cont.

| Ex. | Solvent | NMR data δ ppm (500 MHz) |
|---|---|---|
| 72 | $D_2O$ | 1.28 (d,6H), 2.10 (s,3H), 2.18 (s,3H), 3.07 (m,1H), 3.20 (d,3H), 4.85 (d,1H)*, 5.01 (d,1H)*, 5.74-5.84 (m,2H), 7.26 (s,1H), 7.36 (d,1H), 7.62 (s,1H), 7.72 (d,1H), 8.05 (s,1H) |
| 73 | $D_2O$ | 0.88 (t,3H), 1.91 (s,3H), 2.13 (s,3H), 3.30 (s,3H), 3.46-3.57 (m,2H), 3.86 (s,3H), 5.00 (d)*, 5.64-5.75 (m,2H), 7.10 (d,1H), 7.27 (s,1H), 7.56 (d,1H), 8.12 (s,1H) |
| 74 | $D_2O$ | 0.88 (t,3H), 1.90 (s,3H), 2.13 (s,3H), 3.30 (s,3H), 3.46-3.57 (m,2H), 3.87 (s,3H), 5.00 (d)*, 5.64-5.75 (m,2H), 7.03 (d,1H), 7.18 (s,1H), 7.63 (d,1H), 8.12 (s,1H) |
| 75 } 76 } | $D_2O$ | 0.93 (t,6H), 2.05 (s,6H), 2.26 (s,6H) 3.13 (t,2H), 3.14 (t,2H), 3.44 (s,6H), 3.47 (s,3H), 3.49 (s,3H), 3.47-3.69 (m,4H), 3.86 (t,4H), 5.13 (d)*, 5.74-5.85 (m,4H), 7.46 (d,1H), 7.51 (d,1H), 7.66 (s,1H), 7.73 (d,1H), 7.77 (s,1H), 7.84 (d,1H), 8.22 (s,2H) |
| 82 | $D_2O$ | 2.11 (s,3H), 2.19 (s,3H), 3.65 (s,3H), 4.51 (s,2H), 5.80 (d,2H), 7.37 (t,1H) 7.42 (t,1H), 7.67 (d,1H), 7.73 (d,1H), 8.07 (s,1H) |
| 83 | $D_2O$ | 0.90 (t,3H), 2.03 (s,3H), 2.14 (s,3H), 3.56 (q,2H), 3.59 (s,3H), 4.43 (s,2H), 5.71 (d,1H), 7.27-7.38 (m,2H), 7.53 (d,1H), 7.63 (d,1H), 8.02 (s,1H) |

**Table 2 cont.**

| Ex. | Solvent | NMR data ppm (500 MHz) |
|---|---|---|
| 84 | $D_2O$ | 1.93 (s,3H), 2.11 (s,3H), 3.03 (t,2H), 3.23 (d,3H), 3.36 (s,3H), 4.24 (t,2H), 4.86 (d,1H)*, 5.06 (d,1H)*, 5.65-5.75 (m,1H), 5.75-5.87 (m,1H), 7.04 (d,1H), 7.24 (s,1H), 7.27-7.48 (m,5H), 7.57 (d,1H), 8.12 (s,1H) |
| 85 | $D_2O$ | 1.95 (s,3H), 2.13 (s,3H), 3.09 (t,2H), 3.23 (d,3H), 3.28 (s,3H), 4.34 (m,2H), 4.86 (d,1H)*, 5.06 (d,1H)*, 5.65-5.75 (m,1H), 5.75-5.87 (m,1H), 6.97 (d,1H), 7.20 (s,1H), 7.27-7.48 (m,5H), 7.65 (d,1H), 8.14 (s,1H) |
| 86) 87) | $D_2O$ | 2.09 (s,3H), 2.23 (s,3H), 3.60 (s,3H), 4.97 (d,1H)*, 5.09 (d,1H)*, 5.78-5.90 (m,1H), 5.90-6.03 (m,1H), 7.57-7.94 (m,5H), 7.99 (s,2H), 8.13 (s,1H), 8.17 (s,1H) |
| 88) 89) | $D_2O$ | 0.93 (t,6H), 2.34 (q,4H), 3.68 (s,3H), 3.72 (s,3H) 4.65 (s,4H), 5.1 (s)*, 5.50-5.84 (m,4H), 6.35 (s,2H), 6.58-7.52 (m,16H), 8.02 (s,2H) |

* Protons exchange upon staying in $D_2O$

A summary of examples of intermediate compounds I1 - I39 is given in Table 3 below.

Table 3. Intermediates, summary of working examples I1 - I39.

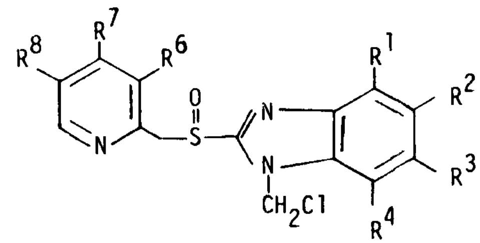

| Example | X | $R^1$ | $R^2$ | $R^3$ | $R^4$ | $R^6$ | $R^7$ | $R^8$ | Identifying data |
|---|---|---|---|---|---|---|---|---|---|
| I1 | SO | H | H | H | H | $CH_3$ | $OCH_2CF_3$ | H | NMR |
| I2 | SO | $CH_3$ | $OCH_2CH_2OCH_3$ | $CH_3$ | H | H | $CH_3$ | $CH_3$ | NMR |
| I3 | SO | H | $CH_3$ | $OCH_2CH_2OCH_3$ | $CH_3$ | H | $CH_3$ | $CH_3$ | |
| I4 | SO | H | 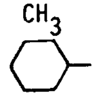 | H | H | H | $CH_3$ | $CH_2CH_3$ | NMR |
| I5 | SO | H | H | 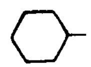 | H | H | $CH_3$ | $CH_2CH_3$ | NMR |
| I6 | SO | H | $OCH_3$ | H | H | H | $OCH_2$- 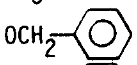 | $CH_2CH_3$ | NMR |
| I7 | SO | H | H | $OCH_3$ | H | H | $OCH_2$- 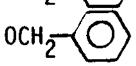 | $CH_2CH_3$ | NMR |
| I8 | SO | H | $CO_2CH_3$ | $CH_3$ | H | $CH_3$ | $CH_3$ | H | NMR |
| I9 | SO | H | $CH_3$ | $CO_2CH_3$ | H | $CH_3$ | $CH_3$ | H | NMR |
| I10 | SO | H | $CH(CH_3)_2$ | H | H | $CH_3$ | H | $CH_3$ | NMR |
| I11 | SO | H | H | $CH(CH_3)_2$ | H | $CH_3$ | H | $CH_3$ | NMR |
| I12 | SO | H | H | H | H | $CH_3$ | $OCH_3$ | $CH_3$ | NMR |

cont.

Table 3. cont.

| Example | X | $R^1$ | $R^2$ | $R^3$ | $R^4$ | $R^6$ | $R^7$ | $R^8$ | Identifying data |
|---|---|---|---|---|---|---|---|---|---|
| I13 | S | H | H | H | H | $CH_3$ | $OCH_3$ | $CH_3$ | NMR |
| I14 | SO | H | $OCH_3$ | H | H | $CH_3$ | $OCH_2CH_2CH(CH_3)_2$ | $CH_3$ | NMR |
| I15 | SO | H | H | $OCH_3$ | H | $CH_3$ | $OCH_2CH_2CH(CH_3)_2$ | $CH_3$ | NMR |
| I16 | SO | H | 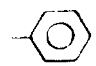 | H | H | $CH_3$ | $OCH_3$ | $CH_3$ | NMR |
| I17 | SO | H | H | 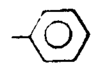 | H | $CH_3$ | $OCH_3$ | $CH_3$ | NMR |
| I18 | SO | $OCH_3$ | H | H | $OCH_3$ | $CH_3$ | $OCH_3$ | $CH_3$ | NMR |
| I19 | SO | H | Br | H | H | $CH_3$ | $OCH_2CH=CH_2$ | $CH_3$ | NMR |
| I20 | SO | H | H | Br | H | $CH_3$ | $OCH_2CH=CH_2$ | $CH_3$ | NMR |
| I21 | SO | H | $OCH_3$ | H | H | $CH_3$ | $OCH_3$ | $CH_3$ | NMR |
| I22 | SO | H | H | $OCH_3$ | H | $CH_3$ | $OCH_3$ | $CH_3$ | NMR |
| I23 | SO | H | $OCH_3$ | H | H | H | $OCH(CH_3)_2$ | H | NMR |
| I24 | SO | H | H | $OCH_3$ | H | H | $OCH(CH_3)_2$ | H | NMR |
| I25 | SO | H | $OCF_3CF_2H$ | H | H | H | $OCH_3$ | H | NMR |
| I26 | SO | H | H | $OCF_3CF_2H$ | H | H | $OCH_3$ | H | NMR |
| I27 | SO | H | $CH_2OCOCH_3$ | H | H | $CH_3$ | $OCH_3$ | H | NMR |
| I28 | SO | H | H | $CH_2OCOCH_3$ | H | $CH_3$ | $OCH_3$ | H | NMR |

cont.

Table 3. cont.

| Example | X | $R^1$ | $R^2$ | $R^3$ | $R^4$ | $R^6$ | $R^7$ | $R^8$ | Identifying data |
|---|---|---|---|---|---|---|---|---|---|
| I30 | SO | H | $CH_2CH_2OCH_3$ | H | H | $CH_3$ | $OCH_3$ | $CH_3$ | NMR |
| I31 | SO | H | H | $CH_2CH_2OCH_3$ | H | $CH_3$ | $OCH_3$ | $CH_3$ | NMR |
| I32 | SO | H | $OCH_3$ | H | H | $CH_3$ | $OCH_2CH_2OCH_3$ | $CH_3$ | NMR |
| I33 | SO | H | H | $OCH_3$ | H | $CH_3$ | $OCH_2CH_2OCH_3$ | $CH_3$ | NMR |
| I34 | SO | H | $OCH_2CH_2$- 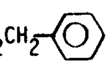 | H | H | $CH_3$ | $OCH_3$ | $CH_3$ | NMR |
| I35 | SO | H | H | $OCH_2CH_2$- 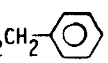 | H | $CH_3$ | $OCH_3$ | $CH_3$ | NMR |
| I36 | SO | H | -CO- 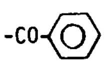 | H | H | $CH_3$ | $OCH_3$ | $CH_3$ | NMR |
| I37 | SO | H | H | -CO- 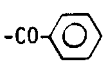 | H | $CH_3$ | $OCH_3$ | $CH_3$ | NMR |
| I38 | SO | H | $OCF_2CF_2H$ | H | H | H | $OCH_3$ | H | NMR |
| I39 | SO | H | H | $OCF_2CF_2H$ | H | H | $OCH_3$ | H | NMR |

Identifying data for compunds according to examples I1 - I39 are given in Table 4 below:

Table 4. NMR data

| Ex. Solvent | NMR data ppm (500 MHz) |
|---|---|
| I 1 $CDCl_3$ | 2.27 (s,3H), 4.38 (d,1H), 4.41 (d,1H), 4.94 (d,1H), 5.03 (d,1H), 6.24 (d,1H), 6,60 (d,1H), 6.66 (d,1H), 7.41 (t,1H), 7.47 (t,1H), 7.57 (d,1H), 7.82 (d,1H), 8.30 (d,1H) |
| I 2 $CDCl_3$ | 2.19 (s,3H), 2.23 (s,3H), 2.48 (s,3H), 2.57 (s,3H), 3.50 (s,3H), 3.76-3.78 (m,2H), 3.97-3.99 (m,2H), 4.75 (d,1H), 4.81 (d,1H), 6.10 (d,1H), 6.45 (d,1H), 7.05 (s,1H), 7.16 (s,1H), 8.29 (s,3H) |
| I 4 $CDCl_3$<br>I 5 | 1.20 (t,3H), 1.25-1.95 (m,11H), 2.21 (s,3H), 2.60-2.64 (m,2H), 4.75-4.83 (m,2H), 6.14-6.18 (m,1H), 6.44-6.49 (m,1H), 7.03 (d,1H), 7.18 (m,1H), 7.26-7.45 (m,1H), 7.65-7.73 (m,1H), 8.31 (s,1H) |
| I 6 $CDCl_3$ | 1.18 (t,3H), 2.61 (q,2H), 3.85 (s,3H), 4.78-4.82 (m,4H), 6.10 (d,1H), 6.39 (d,1H), 6.62 (s,1H), 6.94 (d,1H), 7.12 (dd 1H), 7.32-7.43 (m,5H), 7.43 (d,1H), 8.24 (s,1H) |
| I 7 $CDCl_3$ | 1.18 (t,3H), 2.61 (q,2H), 3.86 (s,3H), 4.78-4.82 (m,4H), 6.09 (d,1H), 6.41 (d,1H), 6.60 (s,1H), 6.94 (d,1H), 7.03 (dd,1H), 7.32-7.43 (m,5H), 7.71 (d,1H) 8.24 (s,1H) |
| I 8 $CDCl_3$ | 2.29 (s,6H), 2.78 (s,3H), 3.93 (s,3H), 4.97-5.05 (m,2H), 6.19 (d,1H), 6.50 (d,1H), 7.00 (d,1H), 7.39 (s,1H), 8.18 (d,1H), 8.41 (s,1H) |

Table 4 cont.

| Ex. | Solvent | NMR data ppm (500 MHz) |
|---|---|---|
| I 9 | $CDCl_3$ | 2.29 (s,6H), 2.72 (s,3H), 3.96 (s,3H), 4.97-5.05 (m,2H), 6.21 (d,1H), 6.51 (d,1H), 7.00 (d,1H), 7.65 (s,1H), 8.16 (s,1H), 8.18 (d,1H) |
| I 10} I 11} | $CDCl_3$ | 1.32 (d,6H), 1.34 (d,6H), 2.27 (s,6H), 2.34 (s,6H), 3.08 (m,1H), 3.09 (m,1H), 4.86 (d,1H), 4.87(d,1H), 4.96 (d,1H), 4.97 (d,1H), 6.19 (d,1H), 6.21 (d,1H), 6.54 (d,1H), 6.58 (d,1H), 7.28-7.74 (m,8H), 8.20 (s,2H) |
| I 12 | $CDCl_3$ | 2.22 (s,3H), 2.25 (s,3H), 3.71 (s,3H), 4.91 (d,1H), 4.97 (d,1H), 6.22 (d,1H), 6.59 (d,1H), 7.40 (t,1H), 7.46 (t,1H), 7.56 (d,1H), 7.82 (d,1H), 8.17 (s,1H) |
| I 13 | $CDCl_3$ | 2.26 (s,3H), 2.36 (s,3H), 3.79 (s,3H), 4.83 (s,2H), 5.94 (s,2H), 7.28-7.30 (m,2H), 7.39 (dd,1H), 7.70 (dd,1H), 8.23 (s,1H) |
| I 14 | $CDCl_3$ | 0.97 (d,6H), 1.65-1.69 (m,2H), 1.81-1.84 (m,1H), 2.23 (s,3H), 2.24 (s,3H), 3.75-3.79 (m,2H), 3.86 (s,3H), 4.90 (d,1H), 4.95 (d,1H), 6.17 (d,1H), 6.51 (d,1H), 6.95 (d,1H), 7.10 (dd,1H), 7.43 (d,1H), 8.16 (s,1H) |
| I 15 | $CDCl_3$ | 0.97 (d,6H), 1.65-1.69 (m,2H), 1.81-1.84 (m,1H), 2.23 (s,3H), 2.24 (s,3H), 3.75-3.79 (m, 2H), 3.91 (s,3H), 4.88 (d,1H), 4.96 (d,1H), 6.17 (d,1H), 6.54 (d,1H), 6.95 (d,1H), 7.01 (dd1H), 7.68 (d,1H), 8.16 (s,1H) |

Table 4 cont.

| EX. | Solvent | NMR data ppm (500 MHz) |
|---|---|---|
| I 16, I 17 | $CDCl_3$ | 2.23 (s,6H), 2.27 (s,3H), 2.28 (s,3H), 3.73 (s,6H) 4.95-4.97 (m,4H), 6.26 (d,1H), 6.27 (d,1H), 6.60 (d,1H), 6,63 (d,1H), 7.38-7.71 (m, 14 H), 7.87 (d,1H), 8.02 (s,1H), 8.18 (s,2H) |
| I 18 | $CDCl_3$ | 2.24 (s,3H), 2.27 (s,3H), 3.73 (s,3H), 3.97 (s,6H), 4.90 (d,1H), 5.05 (d,1H), 6.58 (d,1H), 6.65 (d,1H), 6.76 (d,1H), 6.83 (d,1H), 8.21 (s,1H) |
| I 19, I 20 | $CDCl_3$ | 2.22 (s,6H), 2.25 (s,6H), 4.31 (d,4H), 4.91 (d,2H), 4.95 (d,2H), 5.28 (d,2H), 5.39 (d 2H), 6.03 (m,2H), 6.15 (d 1H), 6.18 (d,1H), 6.48 (d,1H), 6.50 (d,1H), 7.50 (d,1H), 7.53 (d,1H), 7.56 (d,1H), 7.67 (d,1H), 7.72 (s,1H), 7.95 (s,1H), 8.14 (s,2H) |
| I 21 | $CDCl_3$ | 2.23 (s,3H), 2.26 (s,3H), 3.72 (s,3H), 3.87 (s,3H), 4.89 (d,1H), 4.95 (d,1H), 6.18 (d1H), 6.54 (d,1H), 7.19 (dd,1H), 7.26 (d,1H), 7.43 (d,1H), 8.17 (d,1H) |
| I 22 | $CDCl_3$ | 2.23 (s,3H), 2.25 (s,3H), 3.72 (s,3H), 3.92 (s,3H), 4.88 (d,1H), 4.96 (d,1H), 6.17 (d,1H), 6.57 (d,1H), 6.95 (d,1H), 7.01 (dd 1H), 7.67 (d,1H), 8.17 (s,1H) |
| I 23 | $CDCl_3$ | 1.25 (d,6H), 3.87 (s,3H), 4.44 (m,1H), 4.82 (dd,2H), 6.13 (d,1H), 6.41 (d,1H), 6.71 (d+s,2H), 7.12 (dd 1H), 7.26 (d,1H), 7.42 (d,1H), 8.35 (d,1H) |
| I 24 | $CDCl_3$ | 1.25 (d,6H), 3.92 (s,3H), 4.44 (m,1H), 4.82 (dd,2H), 6.13 (d,1H), 6.43 (d,1H), 6.71 (d+s,2H), 6.94 (d,1H), 7.02 (dd,1H), 7.69 (d,1H), 8.35 (d,1H) |

Table 4 cont.

| Ex. | Solvent | NMR data ppm (500 MHz) |
|---|---|---|
| I 25, I 26 | $CDCl_3$ | 3.81 (s,6H), 4.84 (dd,4H), 5.97 (m,2H), 6.16 (d,1H), 6.18 (d,1H), 6.39 (d,1H), 6.41 (d,1H), 6.77 (d+s,4H), 7.30 (dd,1H), 7.34 (dd 1H), 7.42 (d,1H), 7.54 (d,1H), 7.69 (d,1H), 7.81 (d,1H), 8.36 (d,2H) |
| I 27, I 28 | $CDCl_3$ | 2.11 (s,6H), 2.22 (s,6H), 3.85 (s,6H), 4.99 (m,4H), 5.24 (d,2H), 5.27 (d,2H), 6.44 (d,2H), 6.50 (d,1H), 6.51 (d,1H), 6.68 (d,2H), 7.36 (d,1H), 7.42 (d,1H), 7.62 (s,2H), 7.79 (d 2H), 8.20 (d,2H) |
| I 30, I 31 | $CDCl_3$ | 2.22 (s,6H), 2.25 (s,6H), 3.01-3.07 (m,4H), 3.36 3.37 (s,3H), 3.64-3.71 (m,4H), 3.71 (s,6H), 4.89 (d,2H), 4.95 (d,2H), 6.20 (d,2H), 6.56 (d,1H), 6.58 (d,1H), 7.28 (d,1H), 7.35 (d,1H), 7.41(s,1H), 7.47 (d,1H), 7.67 (s,1H), 7.72 (d,1H), 8.17 (s,2H) |
| I 32 | $CDCl_3$ | 2.26 (s,3H), 2.28 (s,3H), 3.42 (s,3H), 3.66-3.69 (m,2H), 3.82 (s,3H), 3.85-3.93 (m,2H), 4.87-4.96 (m,2H), 6.17 (d,1H), 6.49 (d,1H), 7.08 (dd,1H), 7.25 (d,1H), 7.42 (d,1H), 8.16 (s,1H) |
| I 33 | $CDCl_3$ | 2.26 (s,3H), 2.28 (s,3H), 3.42 (s,3H), 3.66-3.69 (m,2H), 3.89(s,3H), 3.85-3.93 (m,2H), 4.87-4.96 (m,2H), 6.17 (d,1H), 6.52 (d,1H), 6.98 (d,1H), 7.00 (dd,1H), 7.67 (d,1H), 8.16 (s,1H) |

Table 4 cont.

| Ex. | Solvent | NMR data ppm (500 MHz) |
|---|---|---|
| I 34 | $CDCl_3$ | 2.22 (s,3H), 2.24 (s,3H), 3.13 (t,2H), 3.71 (s,3H), 4.23 (t,2H), 4.88 (d,1H), 4.95 (d,1H), 6.16 (d,1H), 6.52 (d,1H), 7.09 (dd,1H), 7.25 (d,1H), 7.26-7.36 (m,5H), 7.41 (d,1H), 8.17 (s,1H) |
| I 35 | $CDCl_3$ | 2.22 (s,3H), 2.24 (s,3H), 3.16 (t,2H), 3.71 (s,3H), 4.26 (t,2H), 4.87 (d,1H), 4.96 (d,1H), 6.13 (d,1H), 6.52 (d,1H), 6.93 (d,1H), 7.01 (dd,1H), 7.26-7.36 (m,5H), 7.66 (d,1H), 8.17 (s,1H) |
| I 36 } I 37 } | $CDCl_3$ | 2.23 (s,6H), 2.27 (s,6H), 3.74 (s,6H), 4.96-4.98 (m,4H), 6.25 (d,1H), 6.28 (d,1H), 6.55 (d,1H), 6.57 (d,1H), 7.48-7.87 (m,15H), 8.03 (dd,1H), 8.14 (s,1H), 8.23 (s,1H) |
| I 38 } I 39 } | $CDCl_3$ | 3.74 (s,6H), 4.84 (m,4H), 5.86-6.08 (m,2H), 6.16 (d,1H), 6.18 (d,1H), 6.39 (d,1H), 6.41 (d,1H), 6.77 (d+s,4H), 7.29 (dd,1H), 7.35 (dd,1H), 7.42 (d,1H), 7.55 (d,1H), 7.69 (d,1H), 7.82 (d,1H), 8.36 (d,2H) |

Pharmaceutical preparations containing a compound of the invention as active ingredient are illustrated in the following formulations.

Syrup

A syrup containing 1 % (weight per volume) of active substance was prepared from the following ingredients:

| | |
|---|---|
| Compound according to Example 29 | 1.0 g |
| Sugar, powder | 30.0 g |
| Saccharine | 0.6 g |
| Glycerol | 5.0 g |
| Flavouring agent | 0.05 g |
| Ethanol 96 % | 5.0 g |
| Distilled water q.s. to a final volume of | 100 ml |

Sugar and saccharine were dissolved in 60 g of warm water. After cooling the active compound was added to the sugar solution and glycerol and a solution of flavouring agents dissolved in ethanol were added. The mixture was diluted with water to a final volume of 100 ml.

Enteric-coated tablets

An enteric coated tablet containing 20 mg of active compound was prepared from the following ingredients:

| | | |
|---|---|---|
| I | Active compound | 200 g |
| | Lactose | 700 g |
| | Methyl cellulose | 6 g |
| | Polyvinylpyrrolidone cross-linked | 50 g |
| | Magnesium stearate | 15 g |
| | Sodium carbonate | 6 g |
| | Distilled water | q.s. |
| II | Cellulose acetate phthalate | 200 g |
| | Cetyl alcohol | 15 g |
| | Isopropanol | 2000 g |
| | Methylene chloride | 2000 g |

I Compound according to example 5, powder, was mixed with lactose and granulated with a water solution of methyl cellulose and sodium carbonate. The wet mass was forced through a sieve and the granulate dried in an oven. After drying the granulate was mixed with polyvinylpyrrolidone and magnesium stearate. The dry mixture was pressed into tablet cores (10 000 tablets), each tablet containing 20 mg of active substance, in a tabletting machine using 6 mm diameter punches.

II A solution of cellulose acetate phthalate and cetyl alcohol in isopropanol/methylene chloride was sprayed onto the tablets I in an Accela Cota$^{R}$, Manesty coating equipment. A final tablet weight of 110 mg was obtained.

Solution for intravenous administration

A parenteral formulation for intravenous use, containing 4 mg of active compound per ml, was prepared from the following ingredients:

| | |
|---|---|
| Active compound | 4 g |
| Sterile water to a final volume of | 1000 ml |

The active compound was dissolved in water to a final volume of 1000 ml. The solution was filtered through a 0.22 µm filter and immediately dispensed into 10 ml sterile ampoules. The ampoules were sealed.

Tablets

Tablets containing 30 mg of active compound were prepared from the following ingredients:

| | |
|---|---|
| Compound according to Example 33 in Table I | 300 g |
| Lactose | 700 g |
| Methyl cellulose | 6 g |
| Polyvinyl pyrrolidone, cross-linked (PVP-XL) | 62 g |
| Disodium hydrogen phosphate | 2 g |
| Magnesium stearate | 30 g |
| Purified water | q.s. |

The active compound was mixed with lactose and part of the PVP-XL and granulated with a solution of methyl cellulose and disodium hydrogen phosphate. The wet mass was forced through a screen and dried in a fluidized bed dryer. After adding magnesium stearate and the remainder in PVP-XL and mixing, the drug mixture was compressed into tablets with a mean weight of 110 mg, each tablet containing 30 mg of the active compound.

Enteric coated tablets

500 g of the tablets above were enteric-coated. A solution of the composition below was sprayed onto the tablets in a fluidized bed apparatus using Wurster coating technique.

| Coating solution: | |
|---|---|
| Cellulose acetate phthalate | 40 g |
| Cetyl alcohol | 2 g |
| Isopropanol | 400 g |
| Dichloromethane | 400 g |

The final coated tablet weighed 117 mg.

Suppositories

Suppositories were prepared from the following ingredients using a welding procedure. Each suppository contained 40 mg of active compound.

| | |
|---|---|
| Active compound | 4 g |
| Witepsol H-15 | 180 g |

The active compound was homogenously mixed with Witepsol H-15 at a temperature of 41 °C. The

molten mass was volume filled into pre-fabricated suppository packages to a net weight of 1.84 g. After cooling the packages were heat sealed. Each suppository contained 40 mg of active compound.

Syrup

A syrup containing 1% of active substance was prepared from the following ingredients:

| | |
|---|---|
| Compound according to Example 33 in Table 1 | 1.0 g |
| Sugar, powder | 30.0 g |
| Saccharine | 0.6 g |
| Flavouring agent | 0.05 g |
| Ethanol 96% | 5.0 g |
| Purified water q.s. | to 100 ml |

Sugar and saccharine were dissolved in 60 g of warm water. After cooling the active compound was added to the sugar solution and a solution of flavouring agents dissolved in ethanol was added. The mixture was diluted with water to a final volume of 100 ml.

Solution for intravenous or intramuscular injection

| | |
|---|---|
| Compound according to Example 29 in Table 1 | 60 g |
| Water for injection to make | 1000 ml |

The active compound was dissolved in water to a final volume of 1000 ml. The solution was filtered through a sterile 0.22 pm filter and aseptically dispensed into 1 ml sterile ampoules. The ampoules were sealed.

Formulation for intravenous infusion

| | |
|---|---|
| Sterile compound according to Example 29 in Table 1 | 60 mg |
| Sterile injection vials and stoppers | |

Sterile active compound, 60 mg, was dispensed into 10 ml sterile injection vials. The vials were stoppered with sterile rubber stoppers. The whole filling operation was performed under aseptic conditions in a sterile production area under vertical laminar flow.

Just before use, the active compound dissolved in 10 ml of sterile water is transferred into 100 ml of normal saline solution for infusion to give a total volume of about 110 ml. The solution is administered as an intravenous infusion during a time period of about 30 minutes.

Biological Tests

Inhibiting effect in vivo on gastric acid secretion in conscious dog

Test method

Chronic gastric fistula dog were used. These dogs have been surgically provided with a gastric cannula in the stomach and a duodenal fistula, used for direct intraduodenal administration of test compounds. Following a 4 weeks' recovery period after surgery, tests were performed once a week on each dog. Food and water were withdrawn 18 hours before each test.

Gastric acid secretion was induced by a continuous 4 h infusion of histamine at individual doses (400-600 nmol/kg•h,iv) resulting in approximately 90% of maximal secretion of gastric acid. After 1 h of histamine stimulation, test compounds or vehicle were administered, either iv or im (vehicle = 0.5% saline) or id (vehicle = 0.5% Methocel®, 90 HG 15000, Dow Chem Corp.) via a catheter through the duodenal

fistula. The gastric juice was collected by free flow from the gastric cannula in consecutive 30 min. samples during the duration of the histamine infusion. The samples were titrated to pH 7.0 with 0.1 M or 1.0 M NaOH using an automatic titrator and the acid output was calculated. The acid output during the periods after administration of test compound or vehicle was expressed as the fraction of the output during the period preceding these administrations, and the per cent inhibition of acid secretion was calculated by comparing in each dog the fractional responses induced by the test compound to the corresponding vehicle-induced fractional responses.

The test results given in Table 5 below represent inhibition during the 2nd hour after dose.

Table 5. Gastric acid inhibition in the dog

| Test compound Example no. | Administered dose compound µmol/kg | Route of administration | Inhibition of acid secretion (%) Number of dogs or experiments given within parentheses. |
|---|---|---|---|
| 29 | 0,3 | iv | 45 (1) |
| | 0,5 | iv | 60 (1) |
| | 1 | iv | 80-85 (2) |
| | 1 | im | 90 (1) |
| Mixture of 29 and 32 (2:1) | 0,3 | iv | 55-64 (2) |
| | 1 | iv | 98-98 (2) |
| 32 | 0,3 | iv | 55 (1) |
| | 0,6 | iv | 53 (1) |
| 33 | 1 | id | 97 (1) |
| | 4 | id | 98 (1) |

Solubility

The solubility in water at room temperature for the compounds according to example 29 (disodium salt) was measured and was shown to be higher than 60 mg per ml. By comparison, the sodium salt of

omeprazole, 5-methoxy-2-[(4-methoxy 3-5-dimethyl-2-pyridinyl)methyl sulfinyl]-1-H-benzimidazole, has a solubility of 0.4 mg per ml at pH 9, a pH considered suitable for i.v. administration.

Chemical stability

The chemical stability of various compounds of the invention have been followed kinetically at low concentration at 37°C in aqueous buffer solution at different pH:es. The results shown in Table 6 below illustrate that the compounds of the invention have a high chemical stability.

Table 6

| Compound Example no. | t 1/2 (hours) | | | | |
|---|---|---|---|---|---|
| | pH 2 | pH 3 | pH 5 | pH 7.3 | pH 9.5 |
| Omeprazole-Na$^{\oplus}$ 1) | 0.07 | | 0.04 | 20 | |
| 29 | | | | 74 | ≫600 2) |
| 32 | | | | 51 | |
| 33 | | | No decomp. after 30 h | | |

1) Omeprazole-Na$^{\oplus}$ = 5-methoxy-2-[(4-methoxy-3,5-dimethyl-2-pyridinyl)methyl sulfinyl]-1H-benzimidazole sodium salt
2) 12% decomposition after 25 days at 22° at the concentration 50 mg/ml

**Claims**

**Claims for the following Contracting States : AT, BE, CH, DE, FR, GB, IT, LI, LU, NL, SE**

**1.** A compound of the formula

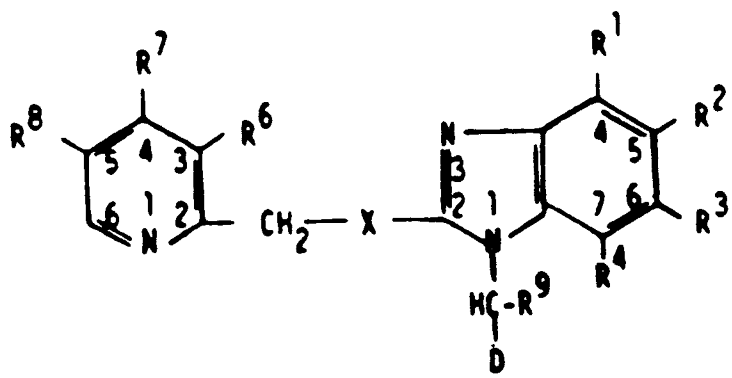

I

and physiologically acceptable salts thereof,
wherein

| | |
|---|---|
| X is | -S- or -SO-; |
| $R^1$, $R^2$, $R^3$ and $R^4$, | which are the same or different, are |
| | (a) H |
| | (b) alkyl containing 1-8 carbon atoms |
| | (c) alkoxy containing 1-8 carbon atoms |
| | (d) alkoxyalkyl containing 1-3 carbon atoms in each alkyl part |
| | (e) alkoxyalkoxy containing 1-3 carbon atoms in each alkyl part |
| | (f) halogen |
| | (g) -$COR^{10}$ |
| | (h) alkylthio containing 1-6 carbon atoms in the alkyl part |
| | (i) alkylsulfinyl containing 1-7 carbon atoms in the alkyl part |
| | (j) phenylalkyl or phenylalkoxy, containing 1-6 carbon atoms in the alkyl and alkoxy parts |

(k) phenyl, or phenoxy, whereby each phenyl group optionally is substituted by 1-3 substituents, the same or different and selected from halogen, $CF_3$, (1-5C) alkyl and (1-5C) alkoxy
(l) haloalkoxy containing 1-6 carbon atoms and 1-11 halogen atoms

D is

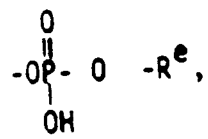

$R^6$ is (a) H
(b) alkyl containing 1-8 carbon atoms
(c) alkoxy containing 1-8 carbon atoms
(d) halogen

$R^8$ is (a) H
(b) alkyl containing 1-8 carbon atoms
(c) alkoxy containing 1-8 carbon atoms
(d) halogen
(e) phenylalkyl containing 1-4 carbon atoms in the alkyl part

$R^7$ is (a) H
(b) alkyl containing 1-7 carbon atoms
(c) alkoxy containing 1-7 carbon atoms
(d) alkoxyalkyl containing 1-3 carbon atoms in each alkyl part
(e) alkoxyalkoxy containing 1-3 carbon atoms in each alkyl part
(f) phenoxy, whereby the phenyl group optionally is substituted by 1 or 2 substituents, the same or different and selected from halogen, $CF_3$, (1-3C) alkyl or (1-3C)alkoxy
(g) phenylalkyl or phenylalkoxy containing 1-7 carbon atoms in the alkyl resp alkoxy part, whereby the the phenyl part optionally is substituted by 1 or 2 substituents, the same or different and selected from halogen, $CF_3$, (1-3C)-alkyl and (1-3C)alkoxy
(h) alkenyloxy containing up to 7 carbon atoms in the alkenyl part
(i) alkynyloxy containing up to 7 carbon atoms in the alkynyl part
(j) alkylthio containing 1-7 carbon atoms in the alkyl part
(k) phenylthio or phenylalkylthio containing 1-3, carbon atoms in the alkyl part
(l) dialkylamino containing 1-7 carbon atoms in each of the alkyl parts
(m) morpholino
(n) piperidino
(o) N-methylpiperazino
(p) pyrrolidino
(q) fluoroalkoxy containing 2-5 carbon atoms and 1-9 fluorine atoms

or $R^6$ and $R^7$, or $R^7$ and $R^8$ together with the adjacent carbon atoms in the pyridine ring form a 5- or 6-membered, saturated or unsaturated ring, which may optionally contain an oxygen, sulphur or an optionally alkylated nitrogen atom;

$R^9$ is (a) H
(b) alkyl containing 1-4 carbon atoms;

$R^{10}$ is (a) alkyl containing 1-6 carbon atoms
(b) alkoxy containing 1-6 carbon atoms
(c) phenyl

$R^e$ is (a) H
(b) (1-6C)alkyl
(c) phenyl(0-3C)alkyl;

and whereby the group D preferably is in the form of a mono or di-ionic salt containing a physiologically acceptable counter cation, with the proviso that either or both of $R^6$ and $R^8$ is halogen when $R^7$ is dialkylamino, morpholino, piperidino, N-methyl-piperazino or pyrrolidino.

2. A compound according to claim 1 wherein X is SO.

3. A compound according to claim 1 wherein X is S.

4. A compound according to claim 1 wherein $R^1$, $R^2$, $R^3$ and $R^4$ are the same or different and selected from hydrogen, (1-4C)alkyl, (5-6C)cycloalkyl, (1-4C)alkoxy, (1-2C)alkoxy(1-2C)alkyl, (1-2C)alkoxy(1-2C)-alkoxy, halogen, (2-4C)alkanoyl, phenylcarbonyl, (1-2C)alkoxycarbonyl, phenyl(1-3C)alkoxy, phenyl or halo(1-4C)alkoxy.

5. A compound according to claim 1 wherein:

$R^1$, $R^2$, $R^3$ and $R^4$ are
- (b) alkyl containing 1-6 carbon atoms
- (c) alkoxy containing 1-6 carbon atoms
- (l) haloalkoxy containing 1-6 carbon atoms and 1-6 halogen atoms; and/or

$R^6$ is
- (b) alkyl containing 1-6 carbon atoms
- (c) alkoxy containing 1-6 carbon atoms; and/or

$R^8$ is
- (b) alkyl containing 1-6 carbon atoms
- (c) alkoxy containing 1-6 carbon atoms; and/or

$R^7$ is
- (j) alkylthio containing 1-3 carbon atoms in the alkyl part
- (k) phenylalkylthio containing 1 carbon atom in the alkyl part
- (l) dialkylamino containing 1-3 carbon atoms in each of the alkyl parts

6. A compound according to claim 1 wherein $R^1$, $R^2$, $R^3$ and $R^4$ are H, alkyl containing 1-6 carbon atoms, or alkoxy containing 1-6 carbon atoms.

7. A compound according to claim 1 wherein $R^1$, $R^2$, $R^3$ and $R^4$ all are H.

8. A compound according to claim 1 wherein $R^1$, $R^3$ and $R^4$ are H and $R^2$ is $OCH_3$ or $R^1$, $R^2$ end $R^4$ are hydrogen and $R^3$ is $OCH_3$.

9. A compound according to claim 1 wherein $R^7$ is alkyl containing 1-6 carbon atoms or alkoxy containing 1-6 carbon atoms.

10. A compound according to claim 1 wherein $R^9$ is H.

11. A compound according to claim 1 wherein D is

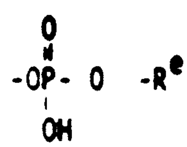

and alkali metal salts thereof, wherein $R^e$ is as defined in claim 1.

12. A compound according to claim 1 wherein the substituent in position 1 of the benzimidazole nucleus is

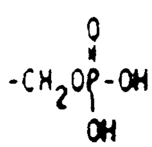

and alkali metal salts thereof.

**13.** A compound according to claim 1 wherein the substituent in position 1 of the benzimidazole nucleus is

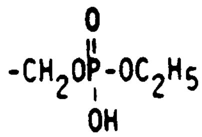

and alkali metal salts thereof.

**14.** A compound according to claim 1 wherein $R^9$ is H and D is selected from

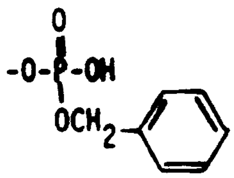

**15.** A compound according to claim 1 wherein the pyridine fragment is 3,5-dimethyl-4-methoxy-, 3-methyl-4-methoxy-, 5-ethyl-4-methoxy-, 4-methoxy-, 4-ethoxy, 4-isopropoxy-, 3,5-dimethyl-, 3,4-dimethyl-, 4,5-dimethyl-, 3-methyl-4(2,2,2-trifluoro)ethoxy-, 3,4-dimethoxy-, 4,5-dimethoxy-, 3-methyl-4-ethylthio-, 3-methyl-4,5-dimethoxy-, 3,4,5-trimethyl, 3-ethyl-4-methoxy-, 3-n-propyl-4-methoxy-, 3-isopropyl-4-methoxy-, or 3-t-butyl-4-methoxy - substituted.

**16.** A compound according to claim 1 wherein the pyridine fragment is 3,5-dimethyl-4-methoxy-, 3-methyl-4-methoxy-, 3-ethyl-4-methoxy, 3-isopropyl-4-methoxy, 4-methoxy-, 4-ethoxy- or 4-isopropoxy-substituted.

**17.** A compound according to claim 1 wherein
X is SO
$R^1$, $R^2$, $R^3$ and $R^4$ are as specified in claim 4,
D is as specified in claim 11, and $R^6$ and $R^8$ are $CH_3$, and $R^7$ is $OCH_3$.

**18.** A compound according to claim 1 wherein the pyridinylmethylsulfinyl benzimidazole moieties are

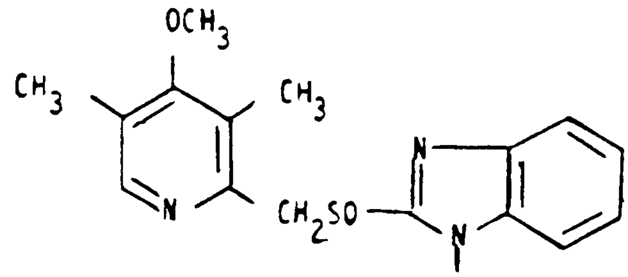

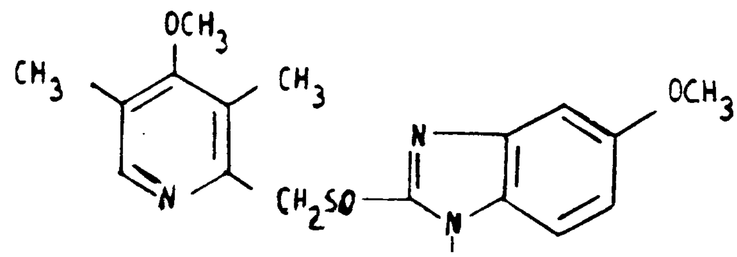

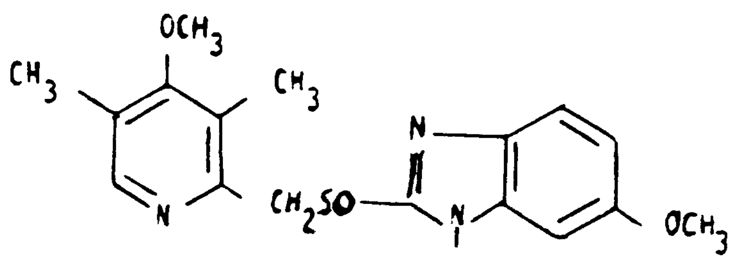

**19.** A compound of the formula

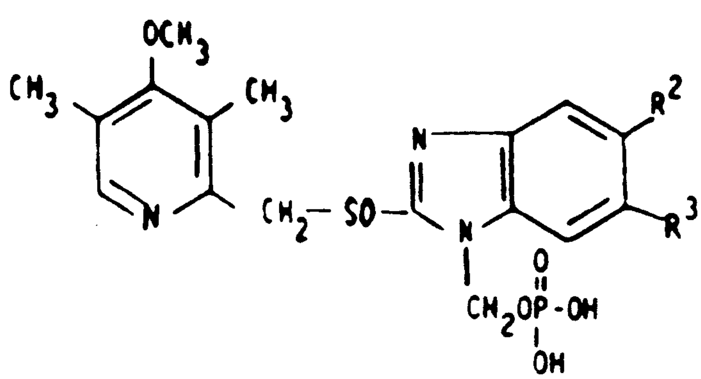

or a mono- or di-ionic salt thereof containing a physiologically acceptable counter cation, wherein $R^2$ and $R^3$ are combined as follows:

| $R^2$ | $R^3$ |
|---|---|
| H | $OCH_3$ |
| $OCH_3$ | H |

**20.** A pharmaceutical composition containing as active ingredient a compound according to any of claims 1-19.

**21.** A compound as defined in any of claims 1-19 for use in therapy.

**22.** A compound as defined in any of claims 1-19 for use in inhibiting gastric acid secretion in mammals and man.

**23.** A compound as defined in any of claims 1-19 for use in the treatment of gastrointestinal inflammatory diseases in mammals and man.

**24.** A compound according to any of claims 1-19 for use in the manufacture of a medicament for inhibiting gastric acid secretion in mammals and man.

**25.** A compound according to any of claims 1-19 for use in the manufacture of a medicament for the treatment of gastrointestinal inflammatory diseases in mammals and man.

**26.** A process for the preparation of a compound of the formula I according to claim 1, by:
C. Oxidizing a compound of the formula I.

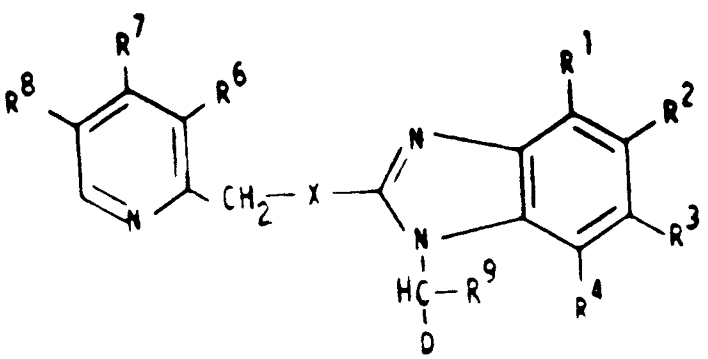

I

wherein X is S, and $R^1$, $R^2$, $R^3$, $R^4$, $R^6$, $R^7$, $R^8$, $R^9$ and D have the meanings given in claim 1, to give a compound of the same formula I wherein X is SO;
D. Reacting a compound of the formula IIA

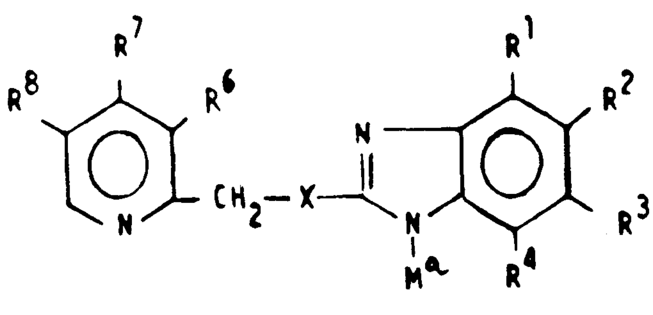

IIA

wherein $R^1$, $R^2$, $R^3$, $R^4$, $R^6$, $R^7$, $R^8$ and X are as defined under formula I and $M^a$ is either a metal

cation such as $Na^+$, $K^+$, $Li^+$ or $Ag^+$ or a quaternary ammonium ion, such as tetrabutylammonium with a compound of the formula

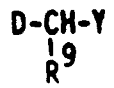

XII

wherein D and $R^9$ are as defined under formula I and Y is halogen such as Cl, Br or I, or a functionally equivalent group; or

E. Removal of a protective group in the D substituent;

whereafter, if necessary, protecting groups are removed and the compound of the formula I thus obtained if desired is converted to a salt or into an optical isomer.

**27.** A process for the preparation of a compound of the formula I according to claim 1, by Reacting a compound of the formula IV

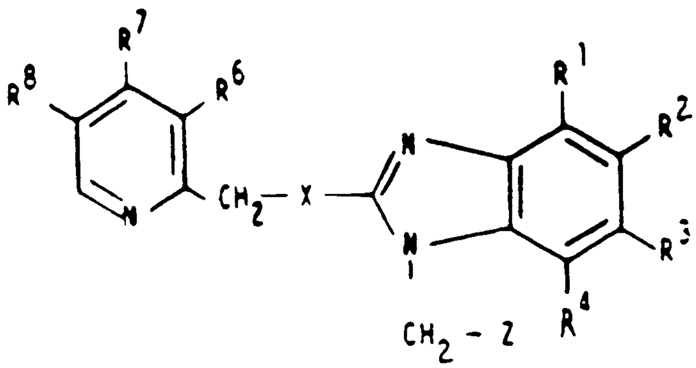

IV

wherein $R^1$, $R^2$, $R^3$, $R^4$, $R^6$, $R^7$, $R^8$ and X are as defined under formula I in claim 1 and Z is halogen such as Cl or a functionally equivalent group, with a compound of the formula VI, VII, VIII or IX

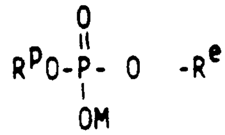

VI

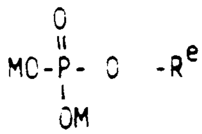

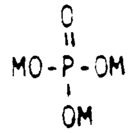

VII VIII

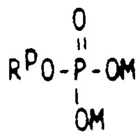

IX

wherein $R^e$ is as defined under formula I above, $R^P$ is a protecting group and M is a counter ion, whereafter, if necessary, protecting groups are removed and the compound of the formula I thus obtained if desired is converted to a salt or into an optical isomer.

**28.** A process according to any of claims 26 and 27 for the preparation of a compound according to claims 2-19.

**29.** A compound of the formula

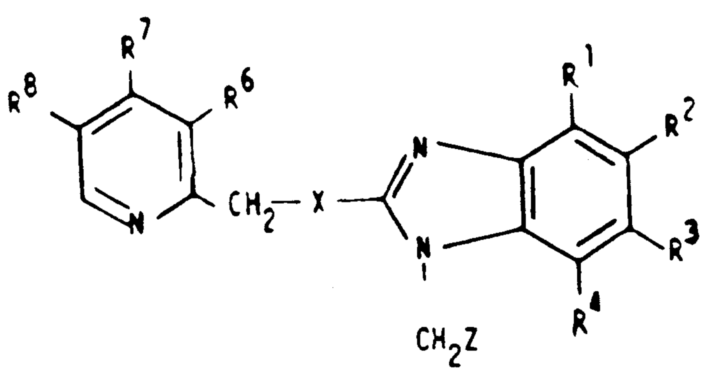

wherein $R^1$, $R^2$, $R^3$, $R^4$, $R^6$, $R^7$, $R^8$ and X are as defined in claim 1 and Z Is halogen such as Cl, Br, or I.

**Claims for the following Contracting State : ES**

**1.** A process for the preparation of a compound of the formula

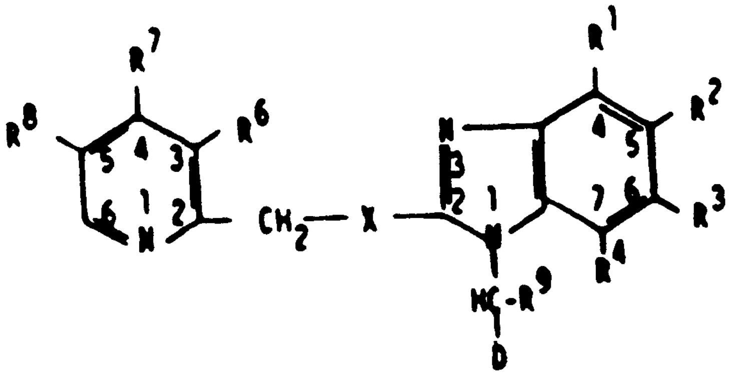

I

and physiologically acceptable salts thereof,
wherein

X is -S- or -SO-;

$R^1$, $R^2$, $R^3$ and $R^4$, which are the same or different, are
(a) H
(b) alkyl containing 1-8 carbon atoms
(c) alkoxy containing 1-8 carbon atoms
(d) alkoxyalkyl containing 1-3 carbon atoms in each alkyl part
(e) alkoxyalkoxy containing 1-3 carbon atoms in each alkyl part
(f) halogen
(g) $-COR^{10}$
(h) alkylthio containing 1-6 carbon atoms in the alkyl part
(i) alkylsulfinyl containing 1-7 carbon atoms in the alkyl part
(j) phenylalkyl or phenylalkoxy, containing 1-6 carbon atoms in the alkyl and alkoxy parts
(k) phenyl, or phenoxy, whereby each phenyl group optionally is substituted by 1-3 substituents, the same or different and selected from halogen, $CF_3$, (1-5C) alkyl and (1-5C) alkoxy
(l) haloalkoxy containing 1-6 carbon atoms and 1-11 halogen atoms

D is

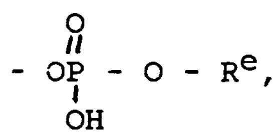

$R^6$ is
(a) H
(b) alkyl containing 1-8 carbon atoms
(c) alkoxy containing 1-8 carbon atoms
(d) halogen

$R^8$ is
(a) H
(b) alkyl containing 1-8 carbon atoms
(c) alkoxy containing 1-8 carbon atoms
(d) halogen
(e) phenylalkyl containing 1-4 carbon atoms in the alkyl part

$R^7$ is
(a) H
(b) alkyl containing 1-7 carbon atoms
(c) alkoxy containing 1-7 carbon atoms
(d) alkoxyalkyl containing 1-3 carbon atoms in each alkyl part
(e) alkoxyalkoxy containing 1-3 carbon atoms in each alkyl part
(f) phenoxy, whereby the phenyl group optionally is substituted by 1 or 2 substituents, the same or different and selected from halogen, $CF_3$, (1-3C)

alkyl or (1-3C)alkoxy
(g) phenylalkyl or phenylalkoxy containing 1-7 carbon atoms in the alkyl resp alkoxy part, whereby the the phenyl part optionally is substituted by 1 or 2 substituents, the same or different and selected from halogen, $CF_3$, (1-3C)-alkyl and (1-3C) alkoxy
(h) alkenyloxy containing up to 7 carbon atoms in the alkenyl part
(i) alkynyloxy containing up to 7 carbon atoms in the alkynyl part
(j) alkylthio containing 1-7 carbon atoms in the alkyl part
(k) phenylthio or phenylalkylthio containing 1-3, carbon atoms in the alkyl part
(i) dialkylamino containing 1-7 carbon atoms in each of the alkyl parts
(m) morpholino
(n) piperidino
(o) N-methylpiperazino
(p) pyrrolidino
(q) fluoroalkoxy containing 2-5 carbon atoms and 1-9 fluorine atoms

or $R^6$ and $R^7$, or $R^7$ and $R^8$ together with the adjacent carbon atoms in the pyridine ring form a 5- or 6-membered, saturated or unsaturated ring, which may optionally contain an oxygen, sulphur or an optionally alkylated nitrogen atom;

$R^9$ is (a) H
(b) alkyl containing 1-4 carbon atoms,

$R^{10}$ is (a) alkyl containing 1-6 carbon atoms
(b) alkoxy containing 1-6 carbon atoms
(c) phenyl

$R^e$ is (a) H
(b) (1-6C)alkyl
(c) phenyl(0-3C)alkyl;

and whereby the group D preferably is in the form of a mono- or di-ionic salt containing a physiologically acceptable counter cation, with the proviso that either or both of $R^6$ and $R^8$ is halogen when $R^7$ is dialkylamino, morpholino, piperidino, N-methyl-piperazino or pyrrolidino, by

C. Oxidizing a compound of the formula I.

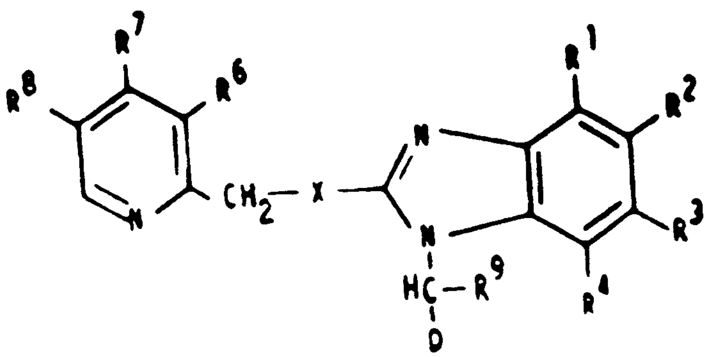

I

wherein X is S, and $R^1$, $R^2$, $R^3$, $R^4$, $R^6$, $R^7$, $R^8$, $R^9$ and D have the meanings given above, to give a compound of the same formula I wherein X is SO;

D. Reacting a compound of the formula IIA

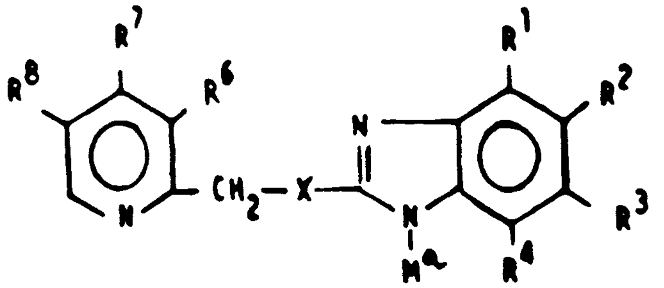

IIA

wherein $R^1$, $R^2$, $R^3$, $R^4$, $R^6$, $R^7$, $R^8$ and X are as defined above and $M^a$ is either a metal cation such as $Na^+$, $K^+$, $Li^+$ or $Ag^+$ or a quaternary ammonium ion, such as tetrabutylammonium with a compound of the formula

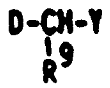

XII

wherein D and $R^9$ are as defined above and Y is halogen such as Cl, Br or I, or a functionally equivalent group; or

E. Removal of a protective group in the D substituent;

whereafter, if necessary, protecting groups are removed and the compound of the formula I thus obtained if desired is converted to a salt or into an optical isomer.

**2.** A process for the preparation of a compound of the formula I as defined in claim 1, by Reacting a compound of the formula IV

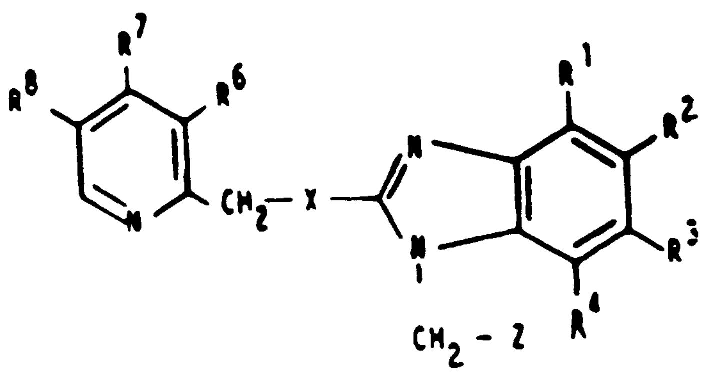

IV

wherein $R^1$, $R^2$, $R^3$, $R^4$, $R^6$, $R^7$, $R^8$ and X are as defined under formula I in claim 1 and Z is halogen such as Cl or a functionally equivalent group, with a compound of the formula VI, VII, VIII or IX

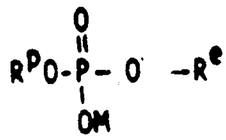

VI

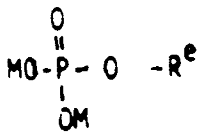

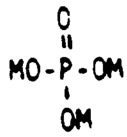

VII VIII

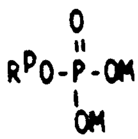

IX

wherein $R^e$ is as defined under formula I above, $R^P$ is a protecting group and M is a counter ion, whereafter, if necessary, protecting groups are removed and the compound of the formula I thus obtained if desired is converted to a salt or into an optical isomer.

**3.** A process according to claims 1 or 2 for the preparation of a compound as defined in claim 1 wherein X is SO.

**4.** A process according to claims 1 or 2 for the preparation of a compound as defined in claim 1 wherein X is S.

**5.** A process according to claims 1 or 2 for the preparation of a compound as defined in claim 1 wherein $R^1$, $R^2$, $R^3$ and $R^4$ are the same or different and selected from hydrogen, (1-4C)alkyl, (5-6C)cycloalkyl, (1-4C)alkoxy, (1-2C)alkoxy(1-2C)alkyl, (1-2C)alkoxy(1-2C)alkoxy, halogen, (2-4C)alkanoyl, phenylcarbonyl, (1-2C)alkoxycarbonyl, phenyl(1-3C)alkoxy, phenyl or halo(1-4C)alkoxy.

**6.** A process according to claims 1 or 2 for the preparation of a compound as defined in claim 1 wherein:

$R^1$, $R^2$, $R^3$ and $R^4$ are

(b) alkyl containing 1-6 carbon atoms
(c) alkoxy containing 1-6 carbon atoms
(l) haloalkoxy containing 1-6 carbon atoms and 1-6 halogen atoms; and/or

$R^6$ is

(b) alkyl containing 1-6 carbon atoms
(c) alkoxy containing 1-6 carbon atoms; and/or

$R^8$ is

(b) alkyl containing 1-6 carbon atoms
(c) alkoxy containing 1-6 carbon atoms; and/or

$R^7$ is

(j) alkylthio containing 1-3 carbon atoms in the alkyl part
(k) phenylalkylthio containing 1 carbon atom in the alkyl part
(l) dialkylamino containing 1-3 carbon atoms in each of the alkyl parts

7. A process according to claims 1 or 2 for the preparation of a compound as defined in claim 1 wherein $R^1$, $R^2$, $R^3$ and $R^4$ are H, alkyl containing 1-6 carbon atoms, or alkoxy containing 1-6 carbon atoms.

8. A process according to claims 1 or 2 for the preparation of a compound as defined in claim 1 wherein $R^1$, $R^2$, $R^3$ and $R^4$ all are H.

9. A process according to claims 1 or 2 for the preparation of a compound as defined in claim 1 wherein $R^1$, $R^3$ and $R^4$ are H and $R^2$ is $OCH_3$ or $R^1$, $R^2$ and $R^4$ are hydrogen and $R^3$ is $OCH_3$.

10. A process according to claims 1 or 2 for the preparation of a compound as defined in claim 1 wherein $R^7$ is alkyl containing 1-6 carbon atoms or alkoxy containing 1-6 carbon atoms.

11. A process according to claims 1 or 2 for the preparation of a compound as defined in claim 1 wherein $R^9$ is H.

12. A process according to claims 1 or 2 for the preparation of a compound as defined in claim 1 wherein D is

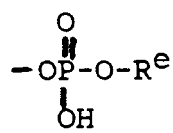

and alkali metal salts thereof, wherein $R^e$ is as defined in claim 1.

13. A process according to claims 1 or 2 for the preparation of a compound as defined in claim 1 wherein the substituent in position 1 of the benzimidazole nucleus is

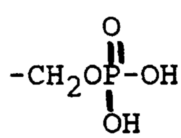

and alkali metal salts thereof.

14. A process according to claims 1 or 2 for the preparation of a compound as defined in claim 1 wherein the substituent in position 1 of the benzimidazole nucleus is

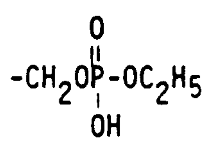

an alkali metal salts thereof.

15. A process according to claims 1 or 2 for the preparation of a compound as defined in claim 1 wherein $R^9$ is H and D is selected from

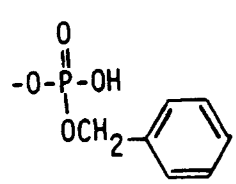

**16.** A process according to claims 1 or 2 for the preparation of a compound as defined in claim 1 where the pyridine fragment is 3,5-dimethyl-4-methoxy-, 3-methyl-4-methoxy-, 5-ethyl-4-methoxy-, 4-methoxy-, 4-ethoxy, 4-isopropoxy-, 3,5-dimethyl-, 3,4-dimethyl-, 4,5-dimethyl-, 3-methyl-4-(2,2,2-trifluoro)ethoxy-, 3,4-dimethoxy-, 4,5-dimethoxy-, 3-methyl-4-ethylthio-, 3-methyl-4,5-dimethoxy-, 3,4,5-trimethyl, 3-ethyl-4-methoxy-, 3-n-propyl-4-methoxy-, 3-isopropyl-4-methoxy-, or 3-t-butyl-4-methoxy - substituted.

**17.** A process according to claims 1 or 2 for the preparation of a compound as defined in claim 1 wherein the pyridine fragment is 3,5-dimethyl-4-methoxy-, 3-methyl-4-methoxy-, 3-ethyl-4-methoxy, 3-isopropyl-4-methoxy, 4-methoxy-, 4-ethoxy- or 4-isopropoxy-substituted.

**18.** A process according to claims 1 or 2 for the preparation of a compound as defined in claim 1 wherein
X is SO
$R^1$, $R^2$, $R^3$ and $R^4$ are as specified in claim 5
D is as specified in claim 12, and $R^6$ and $R^8$ are $CH_3$, and $R^7$ is $OCH_3$.

**19.** A process according to claims 1 or 2 for the preparation of a compound as defined in claim 1 wherein the pyridinylmethylsulfinyl benzimidazole moieties are

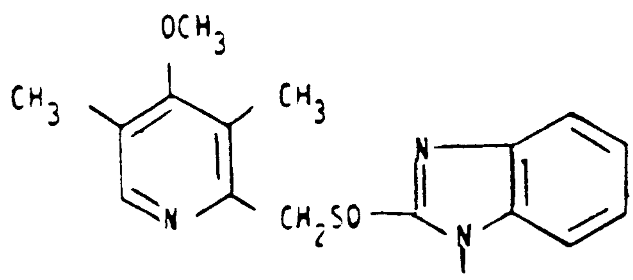

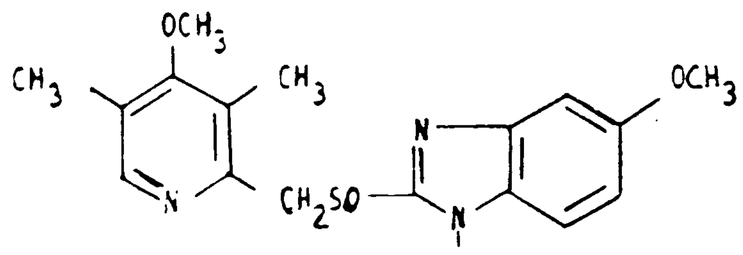

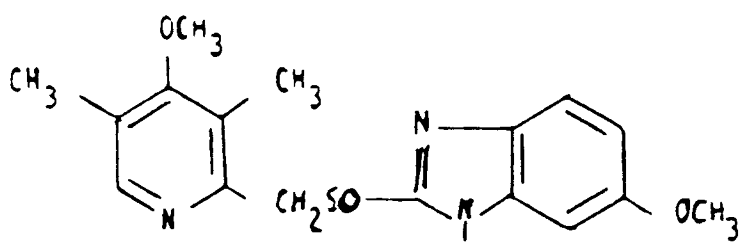

**20.** A process according to claims 1 or 2 for the preparation of a compound as defined in the formula

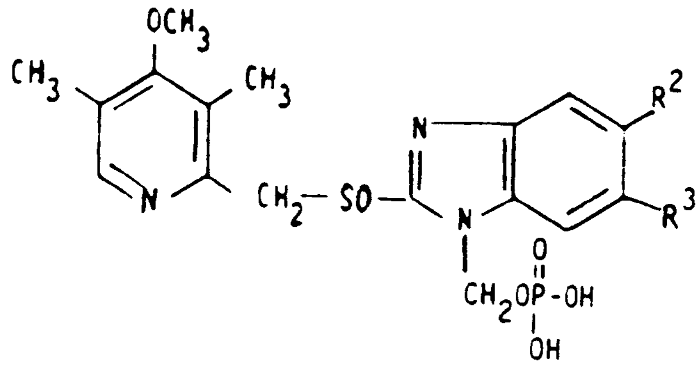

or a mono- or di-ionic salt thereof containing a physiologically acceptable counter cation, wherein $R^2$ and $R^3$ are combined as follows:

| $R^2$ | $R^3$ |
|---|---|
| H | $OCH_3$ |
| $OCH_3$ | H |

**Claims for the following Contracting State : GR**

**1.** A process for the preparation of a compound of the formula

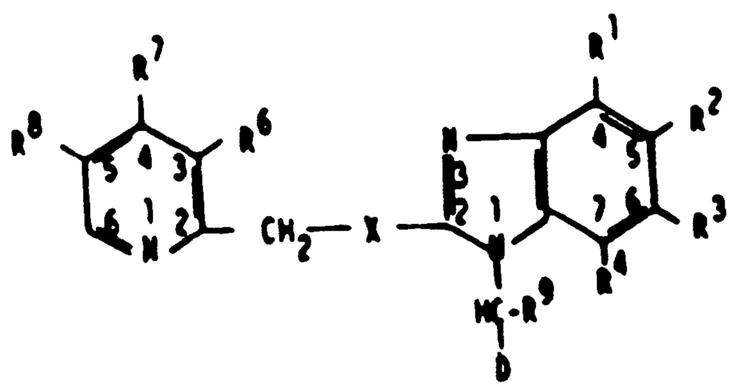

I

and physiologically acceptable salts thereof,
wherein

| | |
|---|---|
| X is | -S- or -SO-; |
| $R^1$, $R^2$, $R^3$ and $R^4$, | which are the same or different, are<br>(a) H<br>(b) alkyl containing 1-8 carbon atoms<br>(c) alkoxy containing 1-8 carbon atoms<br>(d) alkoxyalkyl containing 1-3 carbon atoms in each alkyl part<br>(e) alkoxyalkoxy containing 1-3 carbon atoms in each alkyl part<br>(f) halogen<br>(g) $-COR^{10}$<br>(h) alkylthio containing 1-6 carbon atoms in the alkyl part<br>(i) alkylsulfinyl containing 1-7 carbon atoms in the alkyl part<br>(j) phenylalkyl or phenylalkoxy, containing 1-6 carbon atoms in the alkyl and alkoxy parts |

(k) phenyl, or phenoxy, whereby each phenyl group optionally is substituted by 1-3 substituents, the same or different and selected from halogen, $CF_3$, (1-5C) alkyl and (1-5C) alkoxy
(l) haloalkoxy containing 1-6 carbon atoms and 1-11 halogen atoms

D is

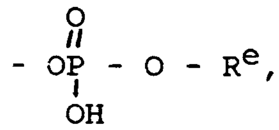

$R^6$ is (a) H
(b) alkyl containing 1-8 carbon atoms
(c) alkoxy containing 1-8 carbon atoms
(d) halogen

$R^8$ is (a) H
(b) alkyl containing 1-8 carbon atoms
(c) alkoxy containing 1-8 carbon atoms
(d) halogen
(e) phenylalkyl containing 1-4 carbon atoms in the alkyl part

$R^7$ is (a) H
(b) alkyl containing 1-7 carbon atoms
(c) alkoxy containing 1-7 carbon atoms
(d) alkoxyalkyl containing 1-3 carbon atoms in each alkyl part
(e) alkoxyalkoxy containing 1-3 carbon atoms in each alkyl part
(f) phenoxy, whereby the phenyl group optionally is substituted by 1 or 2 substituents, the same or different and selected from halogen, $CF_3$, (1-3C) alkyl or (1-3C)alkoxy
(g) phenylalkyl or phenylalkoxy containing 1-7 carbon atoms in the alkyl resp alkoxy part, whereby the the phenyl part optionally is substituted by 1 or 2 substituents, the same or different and selected from halogen, $CF_3$, (1-3C)-alkyl and (1-3C) alkoxy
(h) alkenyloxy containing up to 7 carbon atoms in the alkenyl part
(i) alkynyloxy containing up to 7 carbon atoms in the alkynyl part
(j) alkylthio containing 1-7 carbon atoms in the alkyl part
(k) phenylthio or phenylalkylthio containing 1-3, carbon atoms in the alkyl part
(l) dialkylamino containing 1-7 carbon atoms in each of the alkyl parts
(m) morpholino
(n) piperidino
(o) N-methylpiperazino
(p) pyrrolidino
(q) fluoroalkoxy containing 2-5 carbon atoms and 1-9 fluorine atoms

or $R^6$ and $R^7$, or $R^7$ and $R^8$ together with the adjacent carbon atoms in the pyridine ring form a 5- or 6-membered, saturated or unsaturated ring, which may optionally contain an oxygen, sulphur or an optionally alkylated nitrogen atom;

$R^9$ is (a) H
(b) alkyl containing 1-4 carbon atoms;

$R^{10}$ is (a) alkyl containing 1-6 carbon atoms
(b) alkoxy containing 1-6 carbon atoms
(c) phenyl

$R^e$ is (a) H
(b) (1-6C)alkyl
(c) phenyl(0-3C)alkyl;

and whereby the group D preferably is in the form of a mono- or di-ionic silt containing a physiologically acceptable counter cation, with the proviso that either or both of $R^6$ and $R^8$ is halogen when $R^7$ is dialkylamino, morpholino, piperidino, N-methyl-piperazino or pyrrolidino, by

C. Oxidizing a compound of the formula I.

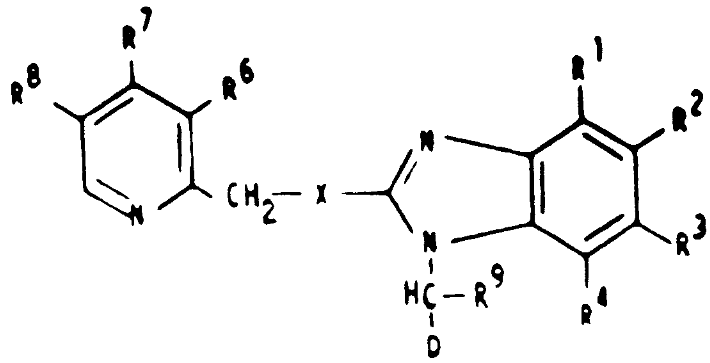

I

wherein X is S, and $R^1$, $R^2$, $R^3$, $R^4$, $R^6$, $R^7$, $R^8$, $R^9$ and D have the meanings given above, to give a compound of the same formula I wherein X is SO;

D. Reacting a compound of the formula IIA

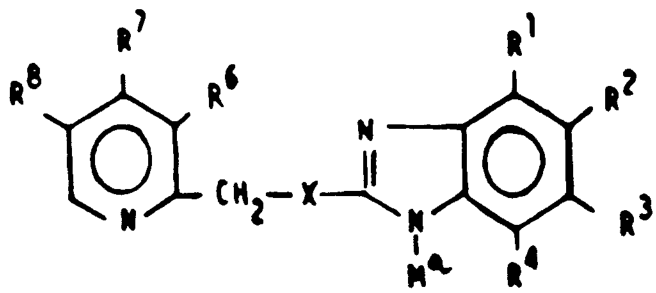

IIA

wherein $R^1$, $R^2$, $R^3$, $R^4$, $R^6$, $R^7$, $R^8$ and X are as defined above and $M^a$ is either a metal cation such as $Na^+$, $K^+$, $Li^+$ or $Ag^+$ or a quaternary ammonium ion, such as tetrabutylammonium with a compound of the formula

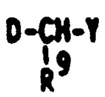

XII

wherein D and $R^9$ are as defined above and Y is halogen such as Cl, Br or I, or a functionally equivalent group; or

E. Removal of a protective group in the D substituent;

whereafter, if necessary, protecting groups are removed and the compound of the formula I thus obtained if desired is converted to a salt or into an optical isomer.

**2.** A process for the preparation of a compound of the formula I as defined in claim 1, by Reacting a compound of the formula IV

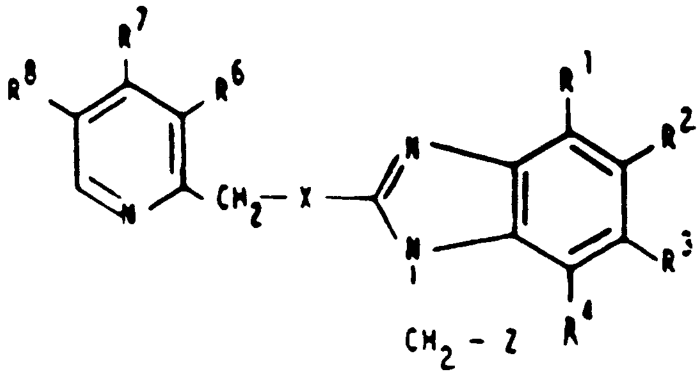

IV

wherein $R^1$, $R^2$, $R^3$, $R^4$, $R^6$, $R^7$, $R^8$ and X are as defined under formula I in claim 1 and Z is halogen such as Cl or a functionally equivalent group, with a compound of the formula VI, VII, VIII or IX

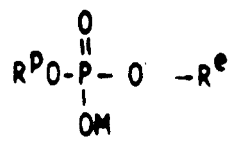

VI

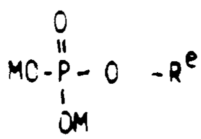

VII

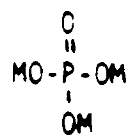

VIII

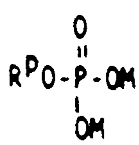

IX

wherein $R^e$ is as defined under formula I above, $R^P$ is a protecting group and M is a counter ion, whereafter, if necessary, protecting groups are removed and the compound of the formula I thus obtained if desired is converted to a salt or into an optical isomer.

**3.** A process according to claims 1 or 2 for the preparation of a compound as defined in claim 1 wherein X is SO.

**4.** A process according to claims 1 or 2 for the preparation of a compound as defined in claim 1 wherein X is S.

**5.** A process according to claims 1 or 2 for the preparation of a compound as defined in claim 1 wherein $R^1$, $R^2$, $R^3$ and $R^4$ are the same or different and selected from hydrogen, (1-4C)alkyl, (5-6C)cycloalkyl, (1-4C)alkoxy, (1-2C)alkoxy(1-2C)alkyl, (1-2C)alkoxy(1-2C)alkoxy, halogen, (2-4C)alkanoyl, phenylcarbonyl, (1-2C)alkoxycarbonyl, phenyl(1-3C)alkoxy, phenyl or halo(1-4C)alkoxy.

**6.** A process according to claims 1 or 2 for the preparation of a compound as defined in claim 1 wherein:

$R^1$, $R^2$, $R^3$ and $R^4$ are

(b) alkyl containing 1-6 carbon atoms
(c) alkoxy containing 1-6 carbon atoms
(l) haloalkoxy containing 1-6 carbon atoms and 1-6 halogen atoms; and/or

$R^6$ is

(b) alkyl containing 1-6 carbon atoms
(c) alkoxy containing 1-6 carbon atoms; and/or

$R^8$ is

(b) alkyl containing 1-6 carbon atoms
(c) alkoxy containing 1-6 carbon atoms; and/or

$R^7$ is

(j) alkylthio containing 1-3 carbon atoms in the alkyl part
(k) phenylalkylthio containing 1 carbon atom in the alkyl part
(l) dialkylamino containing 1-3 carbon atoms in each of the alkyl parts

**7.** A process according to claims 1 or 2 for the preparation of a compound as defined in claim 1 wherein $R^1$, $R^2$, $R^3$ and $R^4$ are H, alkyl containing 1-6 carbon atoms, or alkoxy containing 1-6 carbon atoms.

**8.** A process according to claims 1 or 2 for the preparation of a compound as defined in claim 1 wherein $R^1$, $R^2$, $R^3$ and $R^4$ all are H.

**9.** A process according to claims 1 or 2 for the preparation of a compound as defined in claim 1 wherein $R^1$, $R^3$ and $R^4$ are H and $R^2$ is $OCH_3$ or $R^1$, $R^2$ and $R^4$ are hydrogen and $R^3$ is $OCH_3$.

**10.** A process according to claims 1 or 2 for the preparation of a compound as defined in claim 1 wherein $R^7$ is alkyl containing 1-6 carbon atoms or alkoxy containing 1-6 carbon atoms.

**11.** A process according to claims 1 or 2 for the preparation of a compound as defined in claim 1 wherein $R^9$ is H.

**12.** A process according to claims 1 or 2 for the preparation of a compound as defined in claim 1 wherein D is

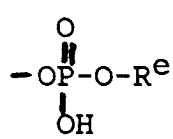

and alkali metal salts thereof, wherein $R^e$ is as defined in claim 1.

**13.** A process according to claims 1 or 2 for the preparation of a compound as defined in claim 1 wherein the substituent in position 1 of the benzimidazole nucleus is

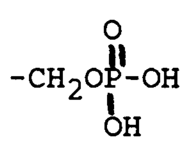

and alkali metal salts thereof.

**14.** A process according to claims 1 or 2 for the preparation of a compound as defined in claim 1 wherein the substituent in position 1 of the benzimidazole nucleus is

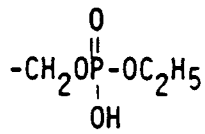

an alkali metal salts thereof.

**15.** A process according to claims 1 or 2 for the preparation of a compound as defined in claim 1 wherein $R^9$ is H and D is selected from

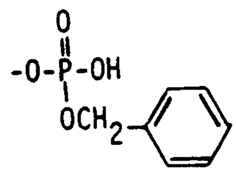

**16.** A process according to claims 1 or 2 for the preparation of a compound as defined in claim 1 where the pyridine fragment is 3,5-dimethyl-4-methoxy-, 3-methyl-4-methoxy-, 5-ethyl-4-methoxy-, 4-methoxy-, 4-ethoxy, 4-isopropoxy-, 3,5-dimethyl-, 3,4-dimethyl-, 4,5-dimethyl-, 3-methyl-4(2,2,2-trifluoro)ethoxy, 3,4-dimethoxy-, 4,5-dimethoxy-, 3-methyl-4-ethylthio, 3-methyl-4,5-dimethoxy, 3,4,5-trimethyl, 3-ethyl-4-methoxy-, 3-n-propyl-4-methoxy-, 3-isopropyl-4-methoxy-, or 3-t-butyl-4-methoxy - substituted.

**17.** A process according to claims 1 or 2 for the preparation of a compound as defined in claim 1 wherein the pyridine fragment is 3,5-dimethyl-4-methoxy-, 3-methyl-4-methoxy-, 3-ethyl-4-methoxy, 3-isopropyl-4-methoxy, 4-methoxy-, 4-ethoxy- or 4-isopropoxy-substituted.

**18.** A process according to claims 1 or 2 for the preparation of a compound as defined in claim 1 wherein
X is SO
$R^1$, $R^2$, $R^3$ and $R^4$ are as specified in claim 5
D is as specified in claim 12, and $R^6$ and $R^8$ are $CH_3$, and $R^7$ is $OCH_3$.

**19.** A process according to claims 1 or 2 for the preparation of a compound as defined in claim 1 wherein the pyridinylmethyl-sulfinyl benzimidazole moieties are

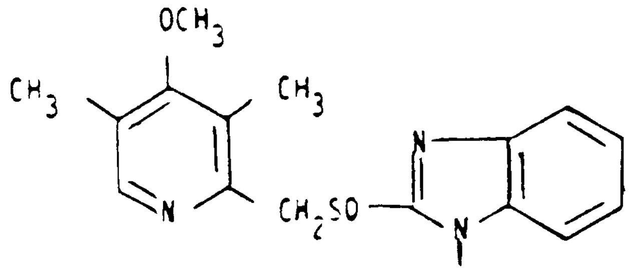

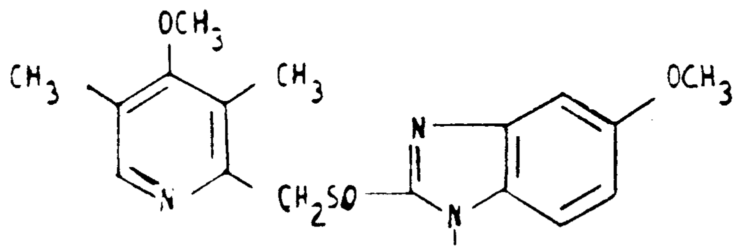

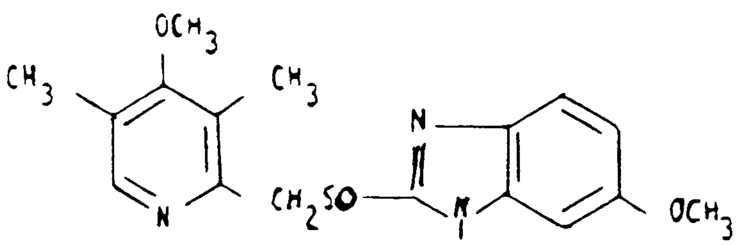

20. A process according to claims 1 or 2 for the preparation of a compound as defined in the formula

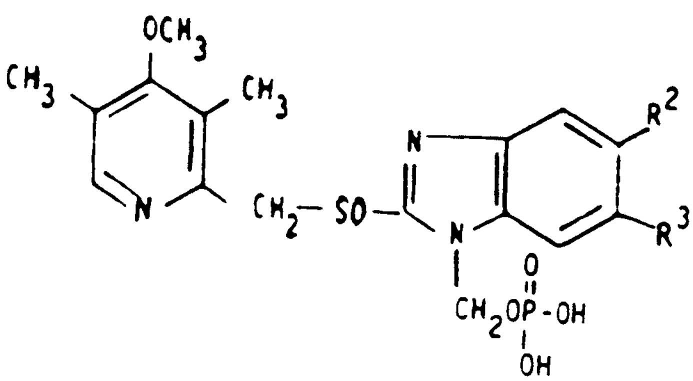

or a mono- or di-ionic salt thereof containing a physiologically acceptable counter cation, wherein $R^2$ and $R^3$ are combined as follows:

| $R^2$ | $R^3$ |
|---|---|
| H | $OCH_3$ |
| $OCH_3$ | H |

**21.** A compound of the formula

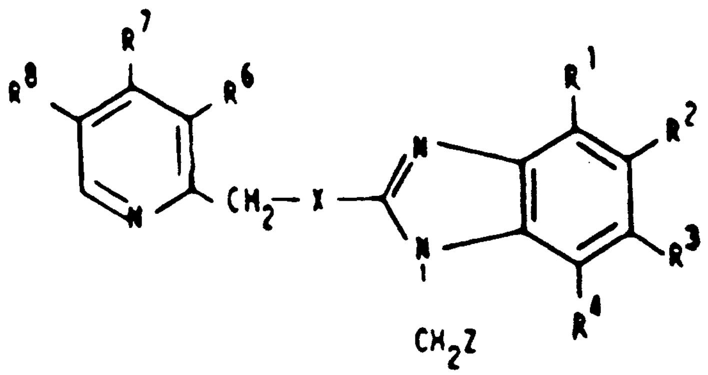

wherein $R^1$, $R^2$, $R^3$, $R^4$, $R^6$, $R^7$, $R^8$ and X are as defined in claim 1 and Z is halogen such as Cl, Br, or I.

**Patentansprüche**
**Patentansprüche für folgende Vertragsstaaten : AT, BE, CH, DE, FR, GB, IT, LI, LU, NL, SE**

**1.** Verbindung der Formel

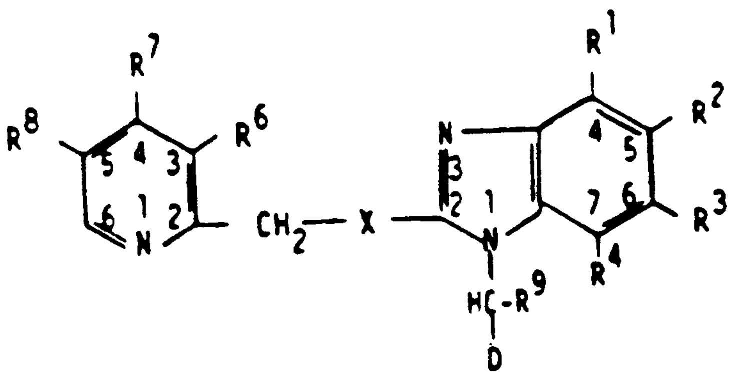

I

und physiologisch akzeptable Salze hievon,
worin

X für -S- oder -SO- steht;
$R^1$, $R^2$, $R^3$ und $R^4$, die gleich oder verschieden sind,
(a) H,
(b) Alkyl mit 1-8 Kohlenstoffatomen,
(c) Alkoxy mit 1-8 Kohlenstoffatomen,
(d) Alkoxyalkyl mit 1-3 Kohlenstoffatomen in jedem Alkylteil,
(e) Alkoxyalkoxy mit 1-3 Kohlenstoffatomen in jedem Alkylteil,
(f) Halogen,
(g) $-COR^{10}$,
(h) Alkylthio mit 1-6 Kohlenstoffatomen im Alkylteil,
(i) Alkylsulfinyl mit 1-7 Kohlenstoffatomen im Alkylteil,
(j) Phenylalkyl oder Phenylalkoxy mit 1-6 Kohlenstoffatomen im Alkyl- und

Alkoxyteil,
(k) Phenyl oder Phenoxy, wobei jede Phenylgruppe gegebenenfalls durch 1-3 Substituenten substituiert ist, die gleich oder verschieden sind und ausgewählt sind aus Halogen, $CF_3$, (1-5C)-Alkyl und (1-5C)-Alkoxy,
(l) Halogenalkoxy mit 1-6 Kohlenstoffatomen und 1-11 Halogenatomen bedeutet,

D für

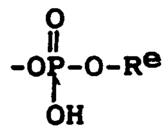

steht,

$R^6$ (a) H,
(b) Alkyl mit 1-8 Kohlenstoffatomen,
(c) Alkoxy mit 1-8 Kohlenstoffatomen,
(d) Halogen
bedeutet,

$R^8$ (a) H,
(b) Alkyl mit 1-8 Kohlenstoffatomen,
(c) Alkoxy mit 1-8 Kohlenstoffatomen,
(d) Halogen,
(e) Phenylalkyl mit 1-4 Kohlenstoffatomen im Alkylteil
bedeutet;

$R^7$ (a) H,
(b) Alkyl mit 1-7 Kohlenstoffatomen,
(c) Alkoxy mit 1-7 Kohlenstoffatomen,
(d) Alkoxyalkyl mit 1-3 Kohlenstoffatomen in jedem Alkylteil,
(e) Alkoxyalkoxy mit 1-3 Kohlenstoffatomen in jedem Alkylteil,
(f) Phenoxy, wobei die Phenylgruppe gegebenenfalls durch 1 oder 2 Substituenten substituiert ist, die gleich oder verschieden sind und ausgewählt sind aus Halogen, $CF_3$, (1-3C)-Alkyl oder (1-3C)-Alkoxy,
(g) Phenylalkyl oder Phenylalkoxy mit 1-7 Kohlenstoffatomen im Alkyl- bzw. Alkoxyteil, wobei der Phenylteil gegebenenfalls durch 1 oder 2 Substituenten substituiert ist, die gleich oder verschieden sind und ausgewählt sind aus Halogen, $CF_3$, (1-3C)-Alkyl und (1-3C)-Alkoxy,
(h) Alkenyloxy mit bis zu 7 Kohlenstoffatomen im Alkenylteil,
(i) Alkinyloxy mit bis zu 7 Kohlenstoffatomen im Alkinylteil,
(j) Alkylthio mit 1-7 Kohlenstoffatomen im Alkylteil,
(k) Phenylthio oder Phenylalkylthio mit 1-3 Kohlenstoffatomen im Alkylteil,
(l) Dialkylamino mit 1-7 Kohlenstoffatomen in jedem der Alkylteile,
(m) Morpholin,
(n) Piperidin,
(o) N-Methylpiperazin,
(p) Pyrrolidin,
(q) Fluoralkoxy mit 2-5 Kohlenstoffatomen und 1-9 Fluoratomen bedeuten,

oder $R^6$ und $R^7$, oder $R^7$ und $R^8$ zusammen mit den angrenzenden Kohlenstoffatomen im Pyridinring einen 5- oder 6-gliedrigen, gesättigten oder ungesättigten Ring bilden, der gegebenenfalls ein Sauerstoff-, Schwefel-, oder ein gegebenenfalls alkyliertes Stickstoffatom enthalten kann;

$R^9$ (a) H,
(b) Alkyl mit 1-4 Kohlenstoffatomen bedeutet;

$R^{10}$ (a) Alkyl mit 1-6 Kohlenstoffatomen,
(b) Alkoxy mit 1-6 Kohlenstoffatomen,
(c) Phenyl bedeutet,

$R^e$ (a) H
(b) (1-6C)-Alkyl,

(c) Phenyl-(0-3C)-alkyl bedeutet;

und wobei die Gruppe D vorzugsweise in Form eines mono- oder diionischen Salzes, das ein physiologisch akzeptables Gegen-Kation enthält, vorliegt, mit der Maßgabe, daß eines oder beides von $R^6$ und $R^8$ Halogen bedeutet, wenn $R^7$ Dialkylamino, Morpholin, Piperidin, N-Methyl-piperazin oder Pyrrolidin ist.

**2.** Verbindung nach Anspruch 1, worin X für SO steht.

**3.** Verbindung nach Anspruch 1, worin X für S steht.

**4.** Verbindung nach Anspruch 1, worin $R^1$, $R^2$, $R^3$ und $R^4$ gleich oder verschieden sind und ausgewählt sind aus Wasserstoff, (1-4C)-Alkyl, (5-6C)-Cycloalkyl, (1-4C)-Alkoxy, (1-2C)-Alkoxy-(1-2C)-alkyl, (1-2C)-Alkoxy-(1-2C)-alkoxy, Halogen, (2-4C)-Alkanoyl, Phenylcarbonyl, (1-2C)-Alkoxycarbonyl, Phenyl-(1-3C)-alkoxy, Phenyl oder Halogen-(1-4C)-alkoxy.

**5.** Verbindung nach Anspruch 1, worin

$R^1$, $R^2$, $R^3$ und $R^4$
(b) Alkyl mit 1-6 Kohlenstoffatomen,
(c) Alkoxy mit 1-6 Kohlenstoffatomen,
(l) Halogenalkoxy mit 1-6 Kohlenstoffatomen und 1-6 Halogenatomen bedeuten; und/oder

$R^6$
(b) Alkyl mit 1-6 Kohlenstoffatomen,
(c) Alkoxy mit 1-6 Kohlenstoffatomen bedeutet; und/oder

$R^8$
(b) Alkyl mit 1-6 Kohlenstoffatomen,
(c) Alkoxy mit 1-6 Kohlenstoffatomen bedeutet; und/oder

$R^7$
(j) Alkylthio mit 1-3 Kohlenstoffatomen im Alkylteil,
(k) Phenylalkylthio mit 1 Kohlenstoffatom im Alkylteil,
(l) Dialkylamino mit 1-3 Kohlenstoffatomen in jedem der Alkylteile bedeutet.

**6.** Verbindung nach Anspruch 1, worin $R^1$, $R^2$, $R^3$ und $R^4$ H, Alkyl mit 1-6 Kohlenstoffatomen oder Alkoxy mit 1-6 Kohlenstoffatomen bedeuten.

**7.** Verbindung nach Anspruch 1, worin $R^1$, $R^2$, $R^3$ und $R^4$ alle H bedeuten.

**8.** Verbindung nach Anspruch 1, worin $R^1$, $R^3$ und $R^4$ H bedeuten und $R^2$ für $OCH_3$ steht, oder $R^1$, $R^2$ und $R^4$ Wasserstoff bedeuten und $R^3$ für $OCH_3$ steht.

**9.** Verbindung nach Anspruch 1, worin $R^7$ Alkyl mit 1-6 Kohlenstoffatomen oder Alkoxy mit 1-6 Kohlenstoffatomen bedeutet.

**10.** Verbindung nach Anspruch 1, worin $R^9$ für H steht.

**11.** Verbindung nach Anspruch 1, worin D für

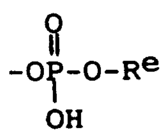

steht, und Alkalimetallsalze hievon, worin $R^e$ wie in Anspruch 1 definiert ist.

**12.** Verbindung nach Anspruch 1, worin der Substituent an der Position 1 des Benzimidazolkerns

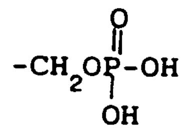

ist, und Alkalimetallsalze hievon.

**13.** Verbindung nach Anspruch 1, worin der Substituent an der Position 1 des Benzimidazolkerns

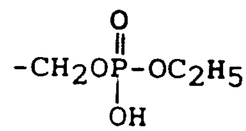

ist, und Alkalimetallsalze hievon.

**14.** Verbindung nach Anspruch 1, worin $R^9$ für H steht und D ausgewählt ist aus

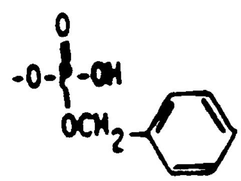

.

**15.** Verbindung nach Anspruch 1, worin das Pyridinfragment 3,5-Dimethyl-4-methoxy-, 3-Methyl-4-methoxy-, 5-Ethyl-4-methoxy-, 4-Methoxy-, 4-Ethoxy-, 4-Isopropoxy-, 3,5-Dimethyl-, 3,4-Dimethyl-, 4,5-Dimethyl-, 3-Methyl-4-(2,2,2-trifluor)-ethoxy-,3,4-Dimethoxy-, 4,5-Dimethoxy-, 3-Methyl-4-ethylthio-, 3-Methyl-4,5-dimethoxy-, 3,4,5-Trimethyl-, 3-Ethyl-4-methoxy-, 3-n-Propyl-4-methoxy-, 3-Isopropyl-4-methoxy- oder 3-t-Butyl-4-methoxy-substituiert ist.

**16.** Verbindung nach Anspruch 1, worin das Pyridinfragment 3,5-Dimethyl-4-methoxy-, 3-Methyl-4-methoxy-, 3-Ethyl-4-methoxy-, 3-Isopropyl-4-methoxy-, 4-Methoxy-, 4-Ethoxy- oder 4-Isopropoxy-substituiert ist.

**17.** Verbindung nach Anspruch 1, worin
X für SO steht,
$R^1$, $R^2$, $R^3$ und $R^4$ die in Anspruch 4 angegebene Bedeutung haben,
D die in Anspruch 11 angegebene Bedeutung hat, und $R^6$ und $R^8$ $CH_3$ sind und $R^7$ $OCH_3$ ist.

**18.** Verbindung nach Anspruch 1, worin die Pyridinylmethylsulfinyl-benzimidazolreste

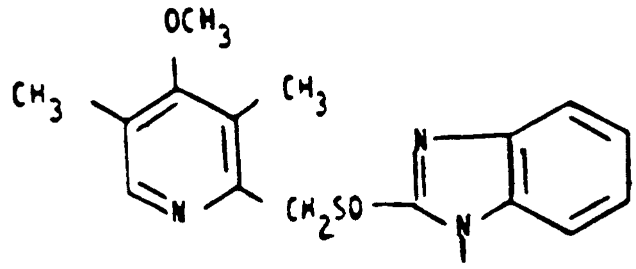

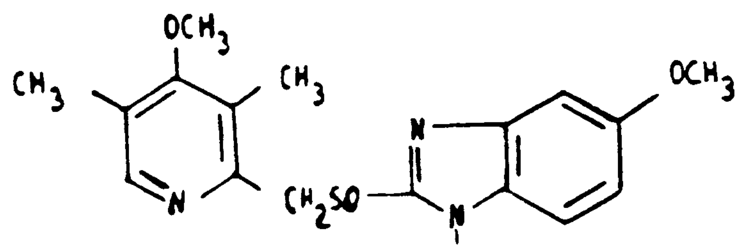

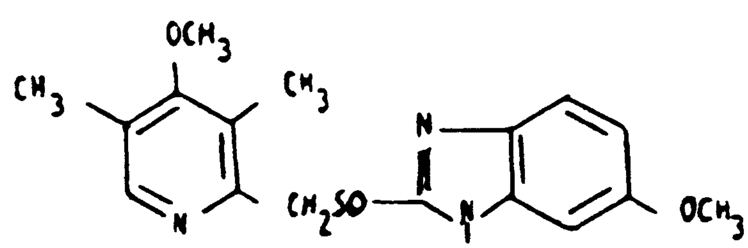

sind.

**19.** Verbindung der Formel

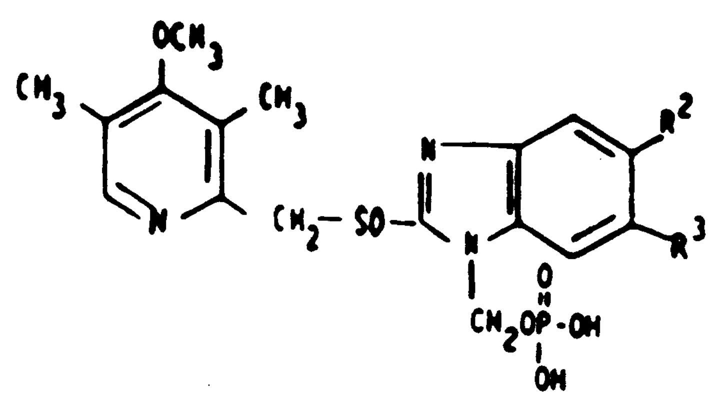

oder ein mono- oder di-ionisches Salz hievon, das ein physiologisch akzeptables Gegen-Kation enthält, worin $R^2$ und $R^3$ folgendermaßen kombiniert sind:

| $R^2$ | $R^3$ |
|---|---|
| H | $OCH_3$ |
| $OCH_3$ | H |

**20.** Pharmazeutische Zusammensetzung, die als aktives Ingrediens eine Verbindung nach einem der Ansprüche 1-19 enthält.

**21.** Verbindung nach einem der Ansprüche 1-19 zur Verwendung in der Therapie.

**22.** Verbindung nach einem der Ansprüche 1-19 zur Verwendung zur Hemmung der Magensäuresekretion bei Säugern und dem Menschen.

**23.** Verbindung nach einem der Ansprüche 1-19 zur Verwendung bei der Behandlung von entzündlichen Erkrankungen des Magendarmtraktes bei Säugern und dem Menschen.

**24.** Verbindung nach einem der Ansprüche 1-19 zur Verwendung bei der Herstellung eines Medikaments zur Hemmung der Magensäuresekretion bei Säugern und dem Menschen.

**25.** Verbindung nach einem der Ansprüche 1-19 zur Verwendung bei der Herstellung eines Medikaments zur Behandlung entzündlicher Erkrankungen des Magendarmtraktes bei Säugern und dem Menschen.

**26.** Verfahren zur Herstellung einer Verbindung der Formel I nach Anspruch 1 durch:
C. Oxidieren einer Verbindung der Formel I

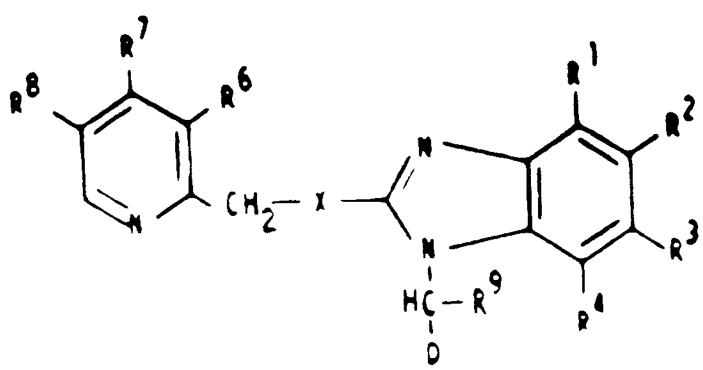

I ,

worin X für S steht und $R^1$, $R^2$, $R^3$, $R^4$, $R^6$, $R^7$, $R^8$, $R^9$ und D die in Anspruch 1 angegebene Bedeutung haben, zum Erhalt einer Verbindung derselben Formel I, worin X für SO steht;
D. Umsetzen einer Verbindung der Formel IIA

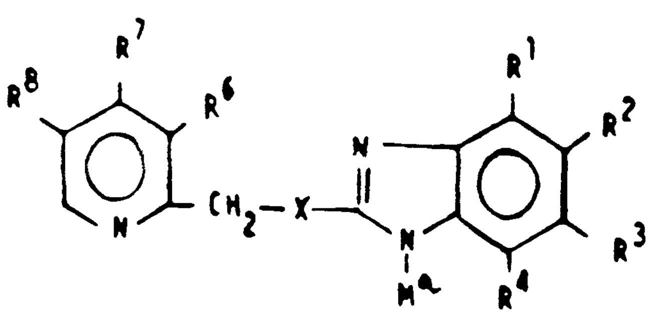

IIA,

worin $R^1$, $R^2$, $R^3$, $R^4$, $R^6$, $R^7$, $R^8$ und X wie in Formel I definiert sind und $M^a$ entweder ein

Metallkation, wie $Na^+$, $K^+$, $Li^+$ oder $Ag^+$, oder ein quaternäres Ammoniumion, wie Tetrabutylammonium, ist, mit einer Verbindung der Formel

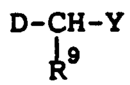

XII,

worin D und $R^9$ wie in Formel I definiert sind und Y Halogen, wie Cl, Br oder J, oder eine funktionell äquivalente Gruppe ist; oder

E. Entfernen einer Schutzgruppe im D-Substituenten;

wonach nötigenfalls die Schutzgruppen entfernt werden und die so erhaltene Verbindung der Formel I gewünschtenfalls in ein Salz oder in ein optisches Isomer übergeführt wird.

**27.** Verfahren zur Herstellung einer Verbindung der Formel I nach Anspruch 1, durch Umsetzung einer Verbindung der Formel IV

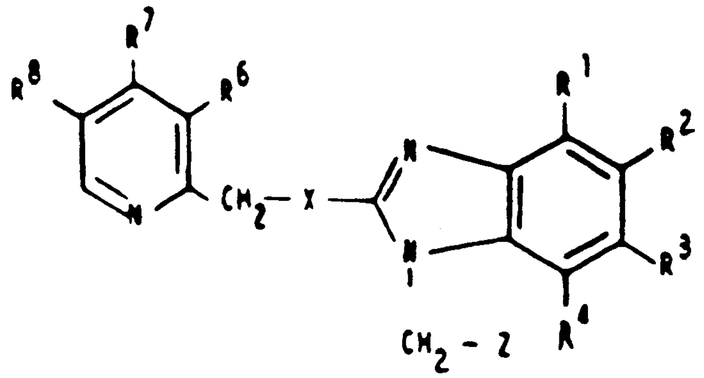

IV ,

worin $R^1$, $R^2$, $R^3$, $R^4$, $R^6$, $R^7$, $R^8$ und X wie in Formel I im Anspruch 1 definiert sind und Z Halogen, wie Cl, oder eine funktionell äquivalente Gruppe ist, mit einer Verbindung der Formel VI, VII, VIII oder IX

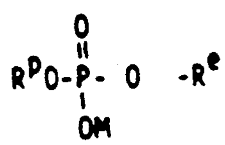

VI

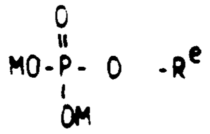 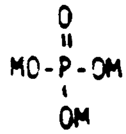

VII VIII

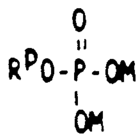

IX

,

worin $R^e$ wie oben in Formel I definiert ist, $R^p$ eine Schutzgruppe ist und M ein Gegen-Ion ist, wonach nötigenfalls Schutzgruppen entfernt werden und die so erhaltene Verbindung der Formel I gewünschtenfalls in ein Salz oder in ein optisches Isomer übergeführt wird.

**28.** Verfahren nach einem der Ansprüche 26 und 27 zur Herstellung einer Verbindung nach den Ansprüchen 2-19.

**29.** Verbindung der Formel

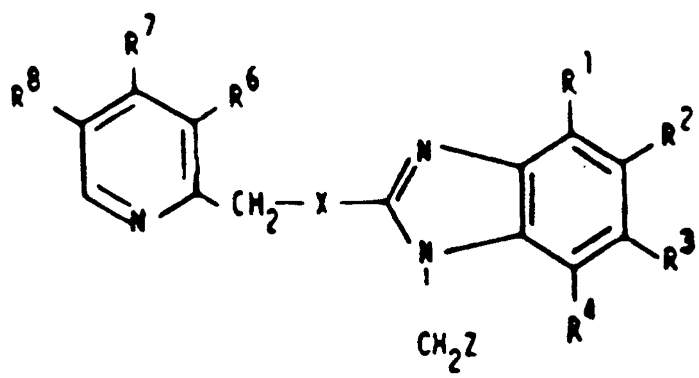

worin $R^1$, $R^2$, $R^3$, $R^4$, $R^6$, $R^7$, $R^8$ und X wie in Anspruch 1 definiert sind und Z Halogen, wie Cl, Br oder J, ist.

**Patentansprüche für folgenden Vertragsstaat : ES**

**1.** Verfahren zur Herstellung einer Verbindung der Formel

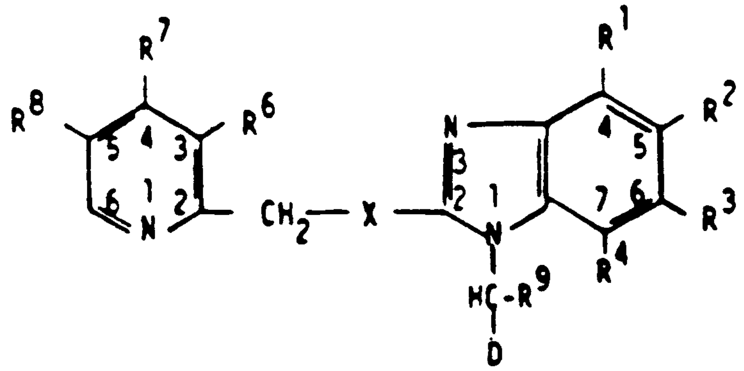

I

und physiologisch akzeptabler Salze hievon,
worin

X für -S- oder -SO- steht;

$R^1$, $R^2$, $R^3$ und $R^4$, die gleich oder verschieden sind,
(a) H,
(b) Alkyl mit 1-8 Kohlenstoffatomen,
(c) Alkoxy mit 1-8 Kohlenstoffatomen,
(d) Alkoxyalkyl mit 1-3 Kohlenstoffatomen in jedem Alkylteil,
(e) Alkoxyalkoxy mit 1-3 Kohlenstoffatomen in jedem Alkylteil,
(f) Halogen,
(g) $-COR^{10}$,
(h) Alkylthio mit 1-6 Kohlenstoffatomen im Alkylteil,
(i) Alkylsulfinyl mit 1-7 Kohlenstoffatomen im Alkylteil,
(j) Phenylalkyl oder Phenylalkoxy mit 1-6 Kohlenstoffatomen im Alkyl- und Alkoxyteil,
(k) Phenyl oder Phenoxy, wobei jede Phenylgruppe gegebenenfalls durch 1-3 Substituenten substituiert ist, die gleich oder verschieden sind und ausgewählt sind aus Halogen, $CF_3$, (1-5C)-Alkyl und (1-5C)-Alkoxy,
(l) Halogenalkoxy mit 1-6 Kohlenstoffatomen und 1-11 Halogenatomen
bedeutet,

D für

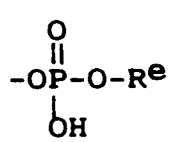

steht,

$R^6$
(a) H,
(b) Alkyl mit 1-8 Kohlenstoffatomen,
(c) Alkoxy mit 1-8 Kohlenstoffatomen,
(d) Halogen
bedeutet,

$R^8$
(a) H,
(b) Alkyl mit 1-8 Kohlenstoffatomen,
(c) Alkoxy mit 1-8 Kohlenstoffatomen,
(d) Halogen,
(e) Phenylalkyl mit 1-4 Kohlenstoffatomen im Alkylteil
bedeutet;

$R^7$
(a) H,
(b) Alkyl mit 1-7 Kohlenstoffatomen,
(c) Alkoxy mit 1-7 Kohlenstoffatomen,

(d) Alkoxyalkyl mit 1-3 Kohlenstoffatomen in jedem Alkylteil,
(e) Alkoxyalkoxy mit 1-3 Kohlenstoffatomen in jedem Alkylteil,
(f) Phenoxy, wobei die Phenylgruppe gegebenenfalls durch 1 oder 2 Substituenten substituiert ist, die gleich oder verschieden sind und ausgewählt sind aus Halogen, $CF_3$, (1-3C)-Alkyl oder (1-3C)-Alkoxy,
(g) Phenylalkyl oder Phenylalkoxy mit 1-7 Kohlenstoffatomen im Alkyl- bzw. Alkoxyteil, wobei der Phenylteil gegebenenfalls durch 1 oder 2 Substituenten substituiert ist, die gleich oder verschieden sind und ausgewählt sind aus Halogen, $CF_3$, (1-3C)-Alkyl und (1-3C)-Alkoxy,
(h) Alkenyloxy mit bis zu 7 Kohlenstoffatomen im Alkenylteil,
(i) Alkinyloxy mit bis zu 7 Kohlenstoffatomen im Alkinylteil,
(j) Alkylthio mit 1-7 Kohlenstoffatomen im Alkylteil,
(k) Phenylthio oder Phenylalkylthio mit 1-3 Kohlenstoffatomen im Alkylteil,
(l) Dialkylamino mit 1-7 Kohlenstoffatomen in jedem der Alkylteile,
(m) Morpholin,
(n) Piperidin,
(o) N-Methylpiperazin,
(p) Pyrrolidin,
(q) Fluoralkoxy mit 2-5 Kohlenstoffatomen und 1-9 Fluoratomen bedeuten,

oder $R^6$ und $R^7$, oder $R^7$ und $R^8$ zusammen mit den angrenzenden Kohlenstoffatomen im Pyridinring einen 5- oder 6-gliedrigen, gesättigten oder ungesättigten Ring bilden, der gegebenenfalls ein Sauerstoff-, Schwefel-, oder ein gegebenenfalls alkyliertes Stickstoffatom enthalten kann;

$R^9$ (a) H,
(b) Alkyl mit 1-4 Kohlenstoffatomen bedeutet;

$R^{10}$ (a) Alkyl mit 1-6 Kohlenstoffatomen,
(b) Alkoxy mit 1-6 Kohlenstoffatomen,
(c) Phenyl bedeutet,

$R^e$ (a) H
(b) (1-6C)-Alkyl,
(c) Phenyl-(0-3C)-alkyl bedeutet;

und wobei die Gruppe D vorzugsweise in Form eines mono- oder diionischen Salzes, das ein physiologisch akzeptables Gegen-Kation enthält, vorliegt, mit der Maßgabe, daß eines oder beides von $R^6$ und $R^8$ Halogen bedeutet, wenn $R^7$ Dialkylamino, Morpholin, Piperidin, N-Methyl-piperazin oder Pyrrolidin ist, durch

C. Oxidieren einer Verbindung der Formel I

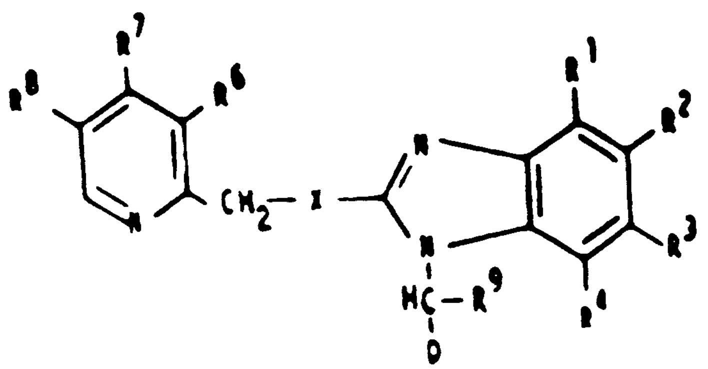

I,

worin X für S steht und $R^1$, $R^2$, $R^3$, $R^4$, $R^6$, $R^7$, $R^8$, $R^9$ und D die oben angegebene Bedeutung haben, zum Erhalt einer Verbindung derselben Formel I, worin X für SO steht;

D. Umsetzen einer Verbindung der Formel IIA

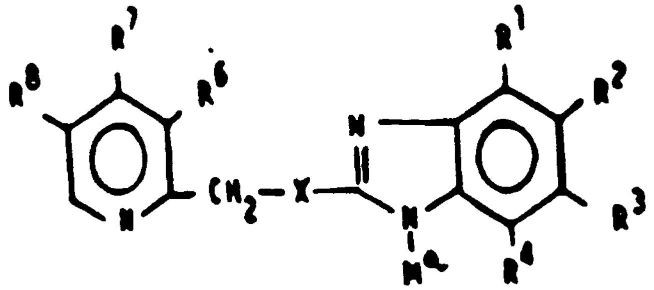

IIA,

worin $R^1$, $R^2$, $R^3$, $R^4$, $R^6$, $R^7$, $R^8$ und X wie oben definiert sind und $M^a$ entweder ein Metallkation, wie $Na^+$, $K^+$, $Li^+$ oder $Ag^+$, oder ein quaternäres Ammoniumion, wie Tetrabutylammonium, ist, mit einer Verbindung der Formel

$$\underset{\displaystyle R^9}{\text{D-CH-Y}} \qquad \text{XII,}$$

worin D und $R^9$ wie oben definiert sind und Y Halogen, wie Cl, Br oder J, oder eine funktionell äquivalente Gruppe ist; oder

E. Entfernen einer Schutzgruppe im D-Substituenten;

wonach nötigenfalls die Schutzgruppen entfernt werden und die so erhaltene Verbindung der Formel I gewünschtenfalls in ein Salz oder in ein optisches Isomer übergeführt wird.

**2.** Verfahren zur Herstellung einer Verbindung der Formel I, wie in Anspruch 1 definiert, durch Umsetzung einer Verbindung der Formel IV

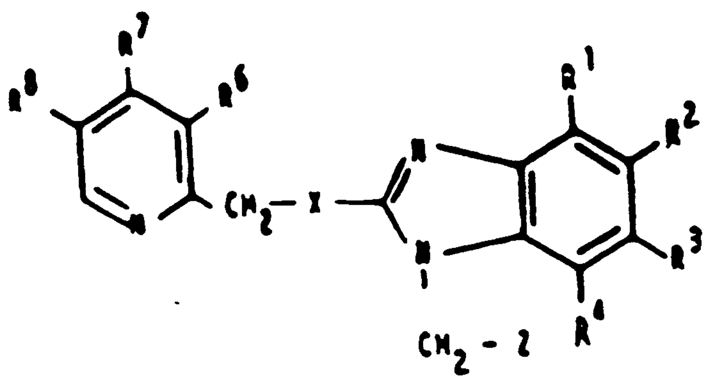

IV ,

worin $R^1$, $R^2$, $R^3$, $R^4$, $R^6$, $R^7$, $R^8$ und X wie in Formel I im Anspruch 1 definiert sind und Z Halogen, wie Cl, oder eine funktionell äquivalente Gruppe ist, mit einer Verbindung der Formel VI, VII, VIII oder IX

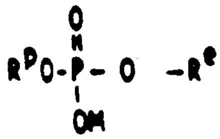

VI

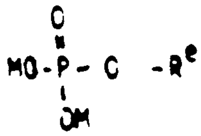

VII

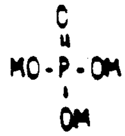

VIII

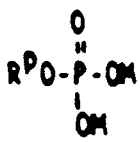

IX

worin $R^e$ wie oben in Formel I definiert ist, $R^p$ eine Schutzgruppe ist und M ein Gegen-Ion ist, wonach nötigenfalls Schutzgruppen entfernt werden und die so erhaltene Verbindung der Formel I gewünschtenfalls in ein Salz oder in ein optisches Isomer übergeführt wird.

**3.** Verfahren nach den Ansprüchen 1 oder 2 zur Herstellung einer Verbindung, wie in Anspruch 1 definiert, worin X für SO steht.

**4.** Verfahren nach den Ansprüchen 1 oder 2 zur Herstellung einer Verbindung, wie in Anspruch 1 definiert, worin X für S steht.

**5.** Verfahren nach den Ansprüchen 1 oder 2 zur Herstellung einer Verbindung, wie in Anspruch 1 definiert, worin $R^1$, $R^2$, $R^3$ und $R^4$ gleich oder verschieden sind und ausgewählt sind aus Wasserstoff, (1-4C)-Alkyl, (5-6C)-Cycloalkyl, (1-4C)-Alkoxy, (1-2C)-Alkoxy-(1-2C)-alkyl, (1-2C)-Alkoxy-(1-2C)-alkoxy, Halogen, (2-4C)-Alkanoyl, Phenylcarbonyl, (1-2C)-Alkoxycarbonyl, Phenyl-(1-3C)-alkoxy, Phenyl oder Halogen-(1-4C)-alkoxy.

**6.** Verfahren nach den Ansprüchen 1 oder 2 zur Herstellung einer Verbindung, wie in Anspruch 1 definiert, worin:

$R^1$, $R^2$, $R^3$ und $R^4$
(b) Alkyl mit 1-6 Kohlenstoffatomen,
(c) Alkoxy mit 1-6 Kohlenstoffatomen,
(l) Halogenalkoxy mit 1-6 Kohlenstoffatomen und 1-6 Halogenatomen bedeuten; und/oder

$R^6$
(b) Alkyl mit 1-6 Kohlenstoffatomen,
(c) Alkoxy mit 1-6 Kohlenstoffatomen bedeutet; und/oder

$R^8$
(b) Alkyl mit 1-6 Kohlenstoffatomen,
(c) Alkoxy mit 1-6 Kohlenstoffatomen bedeutet; und/oder

$R^7$
(j) Alkylthio mit 1-3 Kohlenstoffatomen im Alkylteil,
(k) Phenylalkylthio mit 1 Kohlenstoffatom im Alkylteil,
(l) Dialkylamino mit 1-3 Kohlenstoffatomen in jedem der Alkylteile bedeutet.

7. Verfahren nach den Ansprüchen 1 oder 2 zur Herstellung einer Verbindung, wie in Anspruch 1 definiert, worin $R^1$, $R^2$, $R^3$ und $R^4$ H, Alkyl mit 1-6 Kohlenstoffatomen oder Alkoxy mit 1-6 Kohlenstoffatomen bedeuten.

8. Verfahren nach den Ansprüchen 1 oder 2 zur Herstellung einer Verbindung, wie in Anspruch 1 definiert, worin $R^1$, $R^2$, $R^3$ und $R^4$ alle H bedeuten.

9. Verfahren nach den Ansprüchen 1 oder 2 zur Herstellung einer Verbindung, wie in Anspruch 1 definiert, worin $R^1$, $R^3$ und $R^4$ H bedeuten und $R^2$ für $OCH_3$ steht, oder $R^1$, $R^2$ und $R^4$ Wasserstoff bedeuten und $R^3$ für $OCH_3$ steht.

10. Verfahren nach den Ansprüchen 1 oder 2 zur Herstellung einer Verbindung, wie in Anspruch 1 definiert, worin $R^7$ Alkyl mit 1-6 Kohlenstoffatomen oder Alkoxy mit 1-6 Kohlenstoffatomen bedeutet.

11. Verfahren nach den Ansprüchen 1 oder 2 zur Herstellung einer Verbindung, wie in Anspruch 1 definiert, worin $R^9$ für H steht.

12. Verfahren nach den Ansprüchen 1 oder 2 zur Herstellung einer Verbindung, wie in Anspruch 1 definiert, worin D für

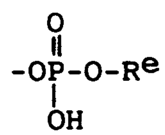

steht, und Alkalimetallsalze hievon, worin $R^e$ wie in Anspruch 1 definiert ist.

13. Verfahren nach den Ansprüchen 1 oder 2 zur Herstellung einer Verbindung, wie in Anspruch 1 definiert, worin der Substituent an der Position 1 des Benzimidazolkerns

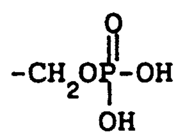

ist, und Alkalimetallsalze hievon.

14. Verfahren nach den Ansprüchen 1 oder 2 zur Herstellung einer Verbindung, wie in Anspruch 1 definiert, worin der Substituent an der Position 1 des Benzimidazolkerns

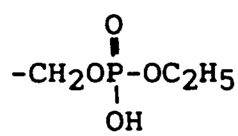

ist, und Alkalimetallsalze hievon.

15. Verfahren nach den Ansprüchen 1 oder 2 zur Herstellung einer Verbindung, wie in Anspruch 1 definiert, worin $R^9$ für H steht und D ausgewählt ist aus

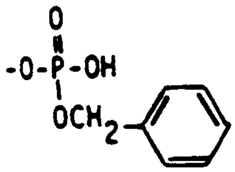

•

**16.** Verfahren nach den Ansprüchen 1 oder 2 zur Herstellung einer Verbindung, wie in Anspruch 1 definiert, worin das Pyridinfragment 3,5-Dimethyl-4-methoxy-, 3-Methyl-4-methoxy-, 5-Ethyl-4-methoxy-, 4-Methoxy-, 4-Ethoxy-, 4-Isopropoxy-, 3,5-Dimethyl-, 3,4-Dimethyl-, 4,5-Dimethyl-, 3-Methyl-4-(2,2,2-trifluor)-ethoxy-,3,4-Dimethoxy-, 4,5-Dimethoxy-, 3-Methyl-4-ethylthio-, 3-Methyl-4,5-dimethoxy-, 3,4,5-Trimethyl-, 3-Ethyl-4-methoxy-, 3-n-Propyl-4-methoxy-, 3-Isopropyl-4-methoxy- oder 3-t-Butyl-4-methoxy-substituiert ist.

**17.** Verfahren nach den Ansprüchen 1 oder 2 zur Herstellung einer Verbindung, wie in Anspruch 1 definiert, worin das Pyridinfragment 3,5-Dimethyl-4-methoxy-, 3-Methyl-4-methoxy-, 3-Ethyl-4-methoxy-, 3-Isopropyl-4-methoxy-, 4-Methoxy-, 4-Ethoxy- oder 4-Isopropoxy-substituiert ist.

**18.** Verfahren nach den Ansprüchen 1 oder 2 zur Herstellung einer Verbindung, wie in Anspruch 1 definiert, worin
X für SO steht,
$R^1$, $R^2$, $R^3$ und $R^4$ die in Anspruch 5 angegebene Bedeutung haben,
D die in Anspruch 12 angegebene Bedeutung hat, und $R^6$ und $R^8$ $CH_3$ sind und $R^7$ $OCH_3$ ist.

**19.** Verfahren nach den Ansprüchen 1 oder 2 zur Herstellung einer Verbindung, wie in Anspruch 1 definiert, worin die Pyridinylmethylsulfinyl-benzimidazolreste

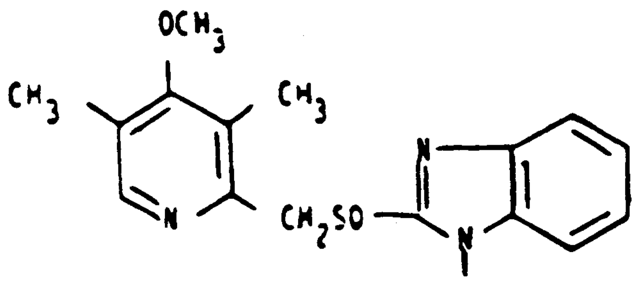

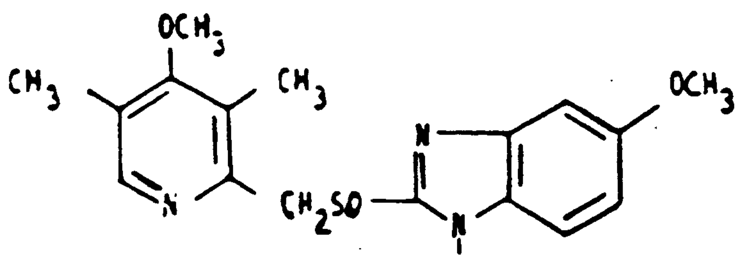

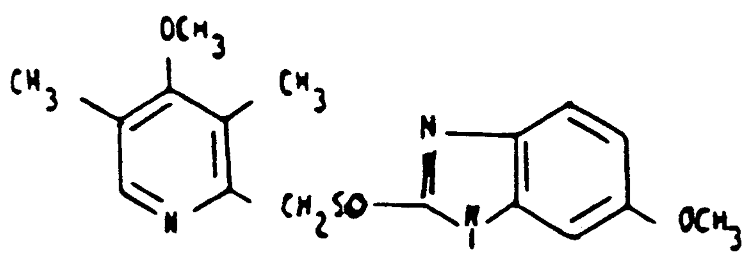

sind.

**20.** Verfahren nach den Ansprüchen 1 oder 2 zur Herstellung einer Verbindung, wie in der Formel

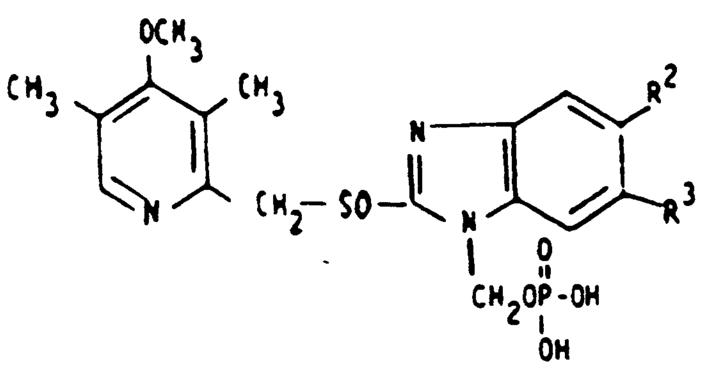

definiert, oder eines mono- oder di-ionischen Salzes hievon, das ein physiologisch akzeptables Gegen-Kation enthält, worin $R^2$ und $R^3$ folgendermaßen kombiniert sind:

| $R^2$ | $R^3$ |
|---|---|
| H | $OCH_3$ |
| $OCH_3$ | H |

**Patentansprüche für folgenden Vertragsstaat : GR**

**1.** Verfahren zur Herstellung einer Verbindung der Formel

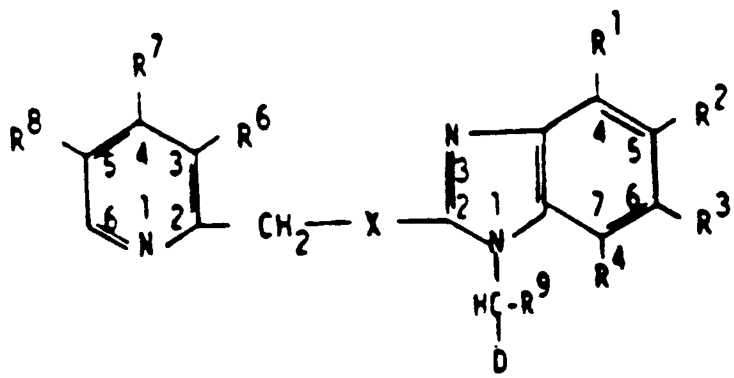

I

und physiologisch akzeptabler Salze hievon,
worin

X für -S- oder -SO- steht;
$R^1$, $R^2$, $R^3$ und $R^4$, die gleich oder verschieden sind,
(a) H,
(b) Alkyl mit 1-8 Kohlenstoffatomen,
(c) Alkoxy mit 1-8 Kohlenstoffatomen,
(d) Alkoxyalkyl mit 1-3 Kohlenstoffatomen in jedem Alkylteil,
(e) Alkoxyalkoxy mit 1-3 Kohlenstoffatomen in jedem Alkylteil,
(f) Halogen,
(g) $-COR^{10}$,
(h) Alkylthio mit 1-6 Kohlenstoffatomen im Alkylteil,
(i) Alkylsulfinyl mit 1-7 Kohlenstoffatomen im Alkylteil,
(j) Phenylalkyl oder Phenylalkoxy mit 1-6 Kohlenstoffatomen im Alkyl- und Alkoxyteil,
(k) Phenyl oder Phenoxy, wobei jede Phenylgruppe gegebenenfalls durch 1-3 Substituenten substituiert ist, die gleich oder verschieden sind und ausgewählt sind aus Halogen, $CF_3$, (1-5C)-Alkyl und (1-5C)-Alkoxy,
(l) Halogenalkoxy mit 1-6 Kohlenstoffatomen und 1-11 Halogenatomen
bedeutet,

D für

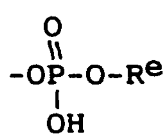

steht,

$R^6$
(a) H,
(b) Alkyl mit 1-8 Kohlenstoffatomen,
(c) Alkoxy mit 1-8 Kohlenstoffatomen,

(d) Halogen
bedeutet,

$R^8$ (a) H,
(b) Alkyl mit 1-8 Kohlenstoffatomen,
(c) Alkoxy mit 1-8 Kohlenstoffatomen,
(d) Halogen,
(e) Phenylalkyl mit 1-4 Kohlenstoffatomen im Alkylteil
bedeutet;

$R^7$ (a) H,
(b) Alkyl mit 1-7 Kohlenstoffatomen,
(c) Alkoxy mit 1-7 Kohlenstoffatomen,
(d) Alkoxyalkyl mit 1-3 Kohlenstoffatomen in jedem Alkylteil,
(e) Alkoxyalkoxy mit 1-3 Kohlenstoffatomen in jedem Alkylteil,
(f) Phenoxy, wobei die Phenylgruppe gegebenenfalls durch 1 oder 2 Substituenten substituiert ist, die gleich oder verschieden sind und ausgewählt sind aus Halogen, $CF_3$, (1-3C)-Alkyl oder (1-3C)-Alkoxy,
(g) Phenylalkyl oder Phenylalkoxy mit 1-7 Kohlenstoffatomen im Alkyl- bzw. Alkoxyteil, wobei der Phenylteil gegebenenfalls durch 1 oder 2 Substituenten substituiert ist, die gleich oder verschieden sind und ausgewählt sind aus Halogen, $CF_3$, (1-3C)-Alkyl und (1-3C)-Alkoxy,
(h) Alkenyloxy mit bis zu 7 Kohlenstoffatomen im Alkenylteil,
(i) Alkinyloxy mit bis zu 7 Kohlenstoffatomen im Alkinylteil,
(j) Alkylthio mit 1-7 Kohlenstoffatomen im Alkylteil,
(k) Phenylthio oder Phenylalkylthio mit 1-3 Kohlenstoffatomen im Alkylteil,
(l) Dialkylamino mit 1-7 Kohlenstoffatomen in jedem der Alkylteile,
(m) Morpholin,
(n) Piperidin,
(o) N-Methylpiperazin,
(p) Pyrrolidin,
(q) Fluoralkoxy mit 2-5 Kohlenstoffatomen und 1-9 Fluoratomen bedeuten,

oder $R^6$ und $R^7$, oder $R^7$ und $R^8$ zusammen mit den angrenzenden Kohlenstoffatomen im Pyridinring einen 5- oder 6-gliedrigen, gesättigten oder ungesättigten Ring bilden, der gegebenenfalls ein Sauerstoff-, Schwefel-, oder ein gegebenenfalls alkyliertes Stickstoffatom enthalten kann;

$R^9$ (a) H,
(b) Alkyl mit 1-4 Kohlenstoffatomen bedeutet;

$R^{10}$ (a) Alkyl mit 1-6 Kohlenstoffatomen,
(b) Alkoxy mit 1-6 Kohlenstoffatomen,
(c) Phenyl bedeutet,

$R^e$ (a) H
(b) (1-6C)-Alkyl,
(c) Phenyl-(0-3C)-alkyl bedeutet;

und wobei die Gruppe D vorzugsweise in Form eines mono- oder diionischen Salzes, das ein physiologisch akzeptables Gegen-Kation enthält, vorliegt, mit der Maßgabe, daß eines oder beides von $R^6$ und $R^8$ Halogen bedeutet, wenn $R^7$ Dialkylamino, Morpholin, Piperidin, N-Methyl-piperazin oder Pyrrolidin ist, durch

C. Oxidieren einer Verbindung der Formel I

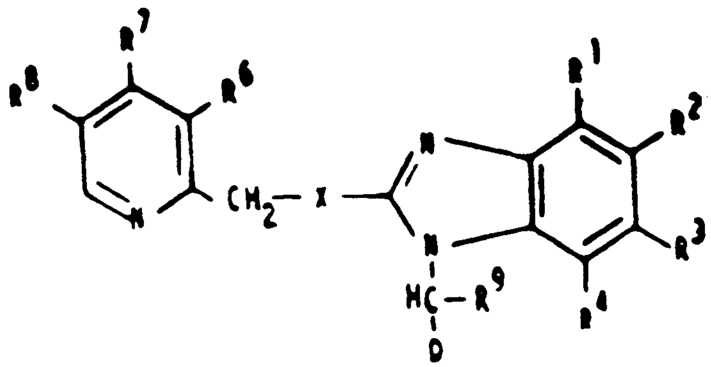

I ,

worin X für S steht und $R^1$, $R^2$, $R^3$, $R^4$, $R^6$, $R^7$, $R^8$, $R^9$ und D die oben angegebene Bedeutung haben, zum Erhalt einer Verbindung derselben Formel I, worin X für SO steht;
D. Umsetzen einer Verbindung der Formel IIA

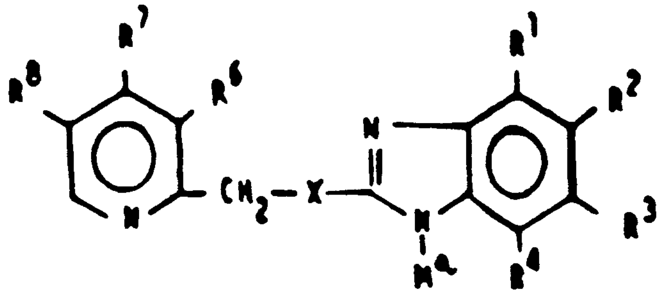

IIA,

worin $R^1$, $R^2$, $R^3$, $R^4$, $R^6$, $R^7$, $R^8$ und X wie oben definiert sind und $M^a$ entweder ein Metallkation, wie $Na^+$, $K^+$, $Li^+$ oder $Ag^+$, oder ein quaternäres Ammoniumion, wie Tetrabutylammonium, ist, mit einer Verbindung der Formel

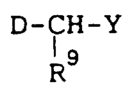

XII,

worin D und $R^9$ wie oben definiert sind und Y Halogen, wie Cl, Br oder J, oder eine funktionell äquivalente Gruppe ist; oder
E. Entfernen einer Schutzgruppe im D-Substituenten;
wonach nötigenfalls die Schutzgruppen entfernt werden und die so erhaltene Verbindung der Formel I gewünschtenfalls in ein Salz oder in ein optisches Isomer übergeführt wird.

**2.** Verfahren zur Herstellung einer Verbindung der Formel I, wie in Anspruch 1 definiert, durch Umsetzung einer Verbindung der Formel IV

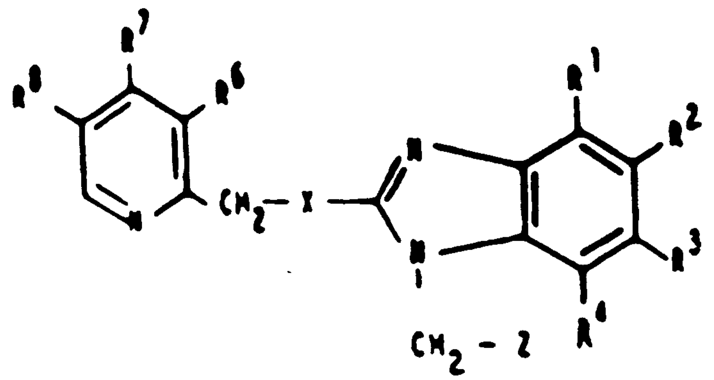

IV,

worin $R^1$, $R^2$, $R^3$, $R^4$, $R^6$, $R^7$, $R^8$ und X wie in Formel I im Anspruch 1 definiert sind und Z Halogen, wie Cl, oder eine funktionell äquivalente Gruppe ist, mit einer Verbindung der Formel VI, VII, VIII oder IX

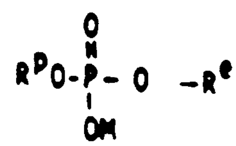

VI

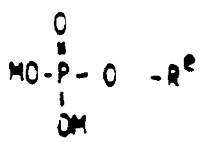

VII

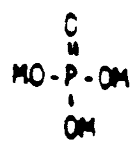

VIII

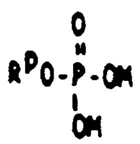

IX

worin $R^e$ wie oben in Formel I definiert ist, $R^p$ eine Schutzgruppe ist und M ein Gegen-Ion ist,
wonach nötigenfalls Schutzgruppen entfernt werden und die so erhaltene Verbindung der Formel I gewünschtenfalls in ein Satz oder in ein optisches Isomer übergeführt wird.

3. Verfahren nach den Ansprüchen 1 oder 2 zur Herstellung einer Verbindung, wie in Anspruch 1 definiert, worin X für SO steht.

4. Verfahren nach den Ansprüchen 1 oder 2 zur Herstellung einer Verbindung, wie in Anspruch 1 definiert, worin X für S steht.

5. Verfahren nach den Ansprüchen 1 oder 2 zur Herstellung einer Verbindung, wie in Anspruch 1 definiert, worin $R^1$, $R^2$, $R^3$ und $R^4$ gleich oder verschieden sind und ausgewählt sind aus Wasserstoff, (1-4C)-Alkyl, (5-6C)-Cycloalkyl, (1-4C)-Alkoxy, (1-2C)-Alkoxy-(1-2C)-alkyl, (1-2C)-Alkoxy-(1-2C)-alkoxy, Halogen, (2-4C)-Alkanoyl, Phenylcarbonyl, (1-2C)-Alkoxycarbonyl, Phenyl-(1-3C)-alkoxy, Phenyl oder

Halogen-(1-4C)-alkoxy.

**6.** Verfahren nach den Ansprüchen 1 oder 2 zur Herstellung einer Verbindung, wie in Anspruch 1 definiert, worin:

$R^1$, $R^2$, $R^3$ und $R^4$
(b) Alkyl mit 1-6 Kohlenstoffatomen,
(c) Alkoxy mit 1-6 Kohlenstoffatomen,
(l) Halogenalkoxy mit 1-6 Kohlenstoffatomen und 1-6 Halogenatomen bedeuten; und/oder

$R^6$ (b) Alkyl mit 1-6 Kohlenstoffatomen,
(c) Alkoxy mit 1-6 Kohlenstoffatomen bedeutet; und/oder

$R^8$ (b) Alkyl mit 1-6 Kohlenstoffatomen,
(c) Alkoxy mit 1-6 Kohlenstoffatomen bedeutet; und/oder

$R^7$ (j) Alkylthio mit 1-3 Kohlenstoffatomen im Alkylteil,
(k) Phenylalkylthio mit 1 Kohlenstoffatom im Alkylteil,
(l) Dialkylamino mit 1-3 Kohlenstoffatomen in jedem der Alkylteile bedeutet.

**7.** Verfahren nach den Ansprüchen 1 oder 2 zur Herstellung einer Verbindung, wie in Anspruch 1 definiert, worin $R^1$, $R^2$, $R^3$ und $R^4$ H, Alkyl mit 1-6 Kohlenstoffatomen oder Alkoxy mit 1-6 Kohlenstoffatomen bedeuten.

**8.** Verfahren nach den Ansprüchen 1 oder 2 zur Herstellung einer Verbindung, wie in Anspruch 1 definiert, worin $R^1$, $R^2$, $R^3$ und $R^4$ alle H bedeuten.

**9.** Verfahren nach den Ansprüchen 1 oder 2 zur Herstellung einer Verbindung, wie in Anspruch 1 definiert, worin $R^1$, $R^3$ und $R^4$ H bedeuten und $R^2$ für $OCH_3$ steht, oder $R^1$, $R^2$ und $R^4$ Wasserstoff bedeuten und $R^3$ für $OCH_3$ steht.

**10.** Verfahren nach den Ansprüchen 1 oder 2 zur Herstellung einer Verbindung, wie in Anspruch 1 definiert, worin $R^7$ Alkyl mit 1-6 Kohlenstoffatomen oder Alkoxy mit 1-6 Kohlenstoffatomen bedeutet.

**11.** Verfahren nach den Ansprüchen 1 oder 2 zur Herstellung einer Verbindung, wie in Anspruch 1 definiert, worin $R^9$ für H steht.

**12.** Verfahren nach den Ansprüchen 1 oder 2 zur Herstellung einer Verbindung, wie in Anspruch 1 definiert, worin D für

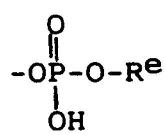

steht, und Alkalimetallsalze hievon, worin $R^e$ wie in Anspruch 1 definiert ist.

**13.** Verfahren nach den Ansprüchen 1 oder 2 zur Herstellung einer Verbindung, wie in Anspruch 1 definiert, worin der Substituent an der Position 1 des Benzimidazolkerns

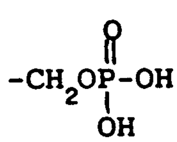

ist, und Alkalimetallsalze hievon.

**14.** Verfahren nach den Ansprüchen 1 oder 2 zur Herstellung einer Verbindung, wie in Anspruch 1 definiert, worin der Substituent an der Position 1 des Benzimidazolkerns

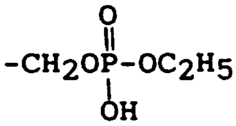

ist, und Alkalimetallsalze hievon.

**15.** Verfahren nach den Ansprüchen 1 oder 2 zur Herstellung einer Verbindung, wie in Anspruch 1 definiert, worin $R^9$ für H steht und D ausgewählt ist aus

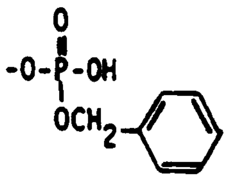

.

**16.** Verfahren nach den Ansprüchen 1 oder 2 zur Herstellung einer Verbindung, wie in Anspruch 1 definiert, worin das Pyridinfragment 3,5-Dimethyl-4-methoxy-, 3-Methyl-4-methoxy-, 5-Ethyl-4-methoxy-, 4-Methoxy-, 4-Ethoxy-, 4-Isopropoxy-, 3,5-Dimethyl-, 3,4-Dimethyl-, 4,5-Dimethyl-, 3-Methyl-4-(2,2,2-trifluor)-ethoxy-,3,4-Dimethoxy-, 4,5-Dimethoxy-, 3-Methyl-4-ethylthio-, 3-Methyl-4,5-dimethoxy-, 3,4,5-Trimethyl, 3-Ethyl-4-methoxy-, 3-n-Propyl-4-methoxy-, 3-Isopropyl-4-methoxy- oder 3-t-Butyl-4-methoxy-substituiert ist.

**17.** Verfahren nach den Ansprüchen 1 oder 2 zur Herstellung einer Verbindung, wie in Anspruch 1 definiert, worin das Pyridinfragment 3,5-Dimethyl-4-methoxy-, 3-Methyl-4-methoxy, 3-Ethyl-4-methoxy-, 3-Isopropyl-4-methoxy-, 4-Methoxy-, 4-Ethoxy- oder 4-Isopropoxy-substituiert ist.

**18.** Verfahren nach den Ansprüchen 1 oder 2 zur Herstellung einer Verbindung, wie in Anspruch 1 definiert, worin
X für SO steht,
$R^1$, $R^2$, $R^3$ und $R^4$ die in Anspruch 5 angegebene Bedeutung haben,
D die in Anspruch 12 angegebene Bedeutung hat, und $R^6$ und $R^8$ $CH_3$ sind und $R^7$ $OCH_3$ ist.

**19.** Verfahren nach den Ansprüchen 1 oder 2 zur Herstellung einer Verbindung, wie in Anspruch 1 definiert, worin die Pyridinylmethylsulfinyl-benzimidazolreste

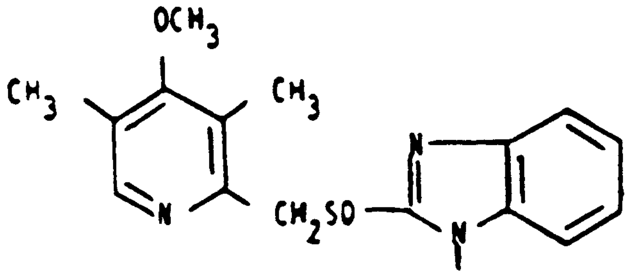

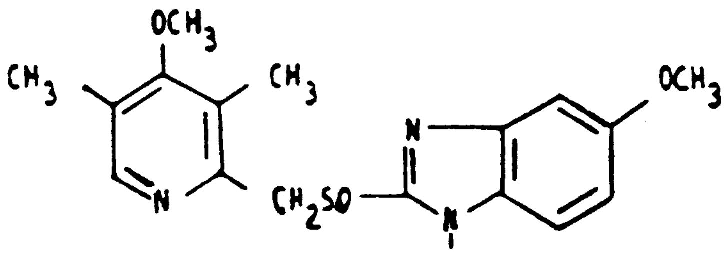

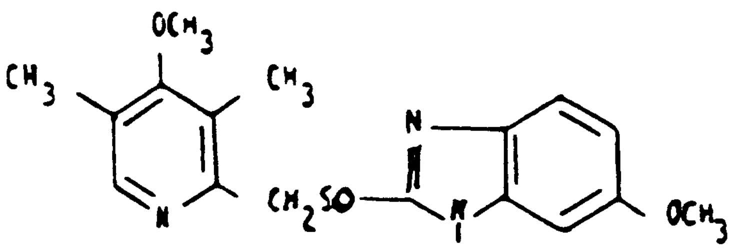

sind.

**20.** Verfahren nach den Ansprüchen 1 oder 2 zur Herstellung einer Verbindung, wie in der Formel

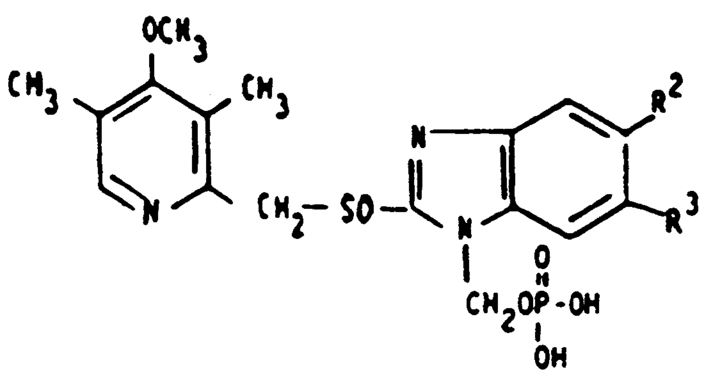

definiert, oder eines mono- oder di-ionischen Salzes hievon, das ein physiologisch akzeptables Gegen-Kation enthält, worin $R^2$ und $R^3$ folgendermaßen kombiniert sind:

| $R^2$ | $R^3$ |
|---|---|
| H | $OCH_3$ |
| $OCH_3$ | H |

**21.** Verbindung der Formel

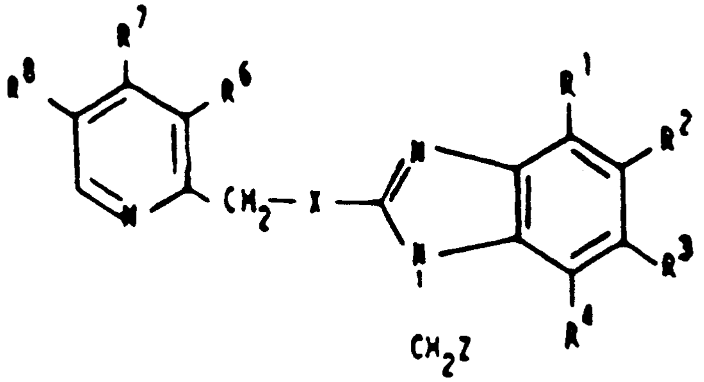

,

worin $R^1$, $R^2$, $R^3$, $R^4$, $R^6$, $R^7$, $R^8$ und X wie in Anspruch 1 definiert sind und Z Halogen, wie Cl, Br oder J, ist.

**Revendications**

**Revendications pour les Etats contractants suivants : AT, BE, CH, DE, FR, GB, IT, LI, LU, NL, SE**

**1.** Composé de formule

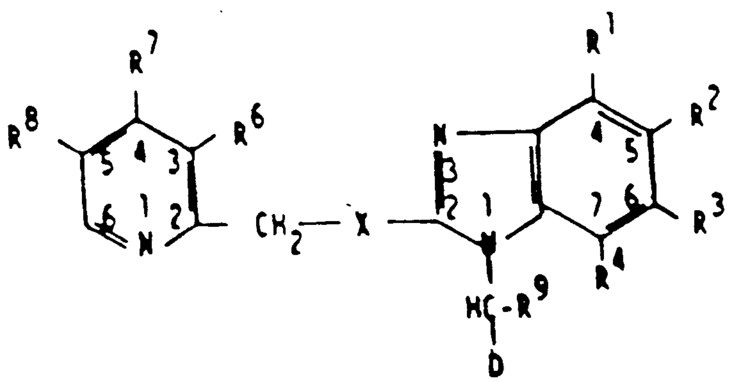

I

et sels physiologiquement acceptables de celui-ci,
formule dans laquelle

X est -S- ou -SO-;

$R^1$, $R^2$, $R^3$ et $R^4$, qui sont identiques ou différents, sont

(a) H

(b) un groupe alkyle contenant de 1 à 8 atomes de carbone,

(c) un groupe alcoxy contenant de 1 à 8 atomes de carbone,

(d) un groupe alcoxyalkyle contenant de 1 à 3 atomes de carbone dans chaque fragment alkyle,

(e) un groupe alcoxyalcoxy contenant de 1 à 3 atomes de carbone dans chaque fragment alkyle,

(f) un atome d'halogène,

(g) $-COR^{10}$,
(h) un groupe alkylthio contenant de 1 à 6 atomes de carbone dans le fragment alkyle,
(i) un groupe alxylsulfinyle contenant de 1 à 7 atomes de carbone dans le fragment alkyle,
(j) un groupe phénylalkyle ou phénylalcoxy contenant de 1 à 6 atomes de carbone dans les fragments alkyle et alcoxy,
(k) un groupe phényle ou phénoxy, chaque groupe phényle étant éventuellement substitué par 1-3 substituants identiques ou différents et choisis parmi des substituants halogène, $CF_3$, alkyle en $C_{1-5}$ et alcoxy en $C_{1-5}$,
(l) un groupe halogénoalcoxy contenant de 1 à 6 atomes de carbone et de 1 à 11 atomes d'halogène;

D est

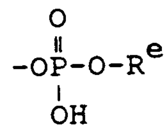

$R^6$ est

(a) H,
(b) un groupe alkyle contenant de 1 à 8 atomes de carbone,
(c) un groupe alcoxy contenant de 1 à 8 atomes de carbone,
(d) un atome d'halogène;

$R^8$ est

(a) H,
(b) un groupe alkyle contenant de 1 à 8 atomes de carbone,
(c) un groupe alcoxy contenant de 1 à 8 atomes de carbone,
(d) un atome d'halogène,
(e) un groupe phénylalkyle contenant de 1 à 4 atomes de carbone dans le fragment alkyle;

$R^7$ est

(a) H,
(b) un groupe alkyle contenant de 1 à 7 atomes de carbone,
(c) un groupe alcoxy contenant de 1 à 7 atomes de carbone,
(d) un groupe alcoxyalkyle contenant de 1 à 3 atomes de carbone dans chaque fragment alkyle,
(e) un groupe alcoxyalcoxy contenant de 1 à 3 atomes de carbone dans chaque fragment alkyle,
(f) un groupe phénoxy, le radical phényle étant éventuellement substitué par 1 ou 2 substituants, identiques ou différents et choisis parmi des substituants halogène, $CF_3$, alkyle en $C_{1-3}$ ou alcoxy en $C_{1-3}$,
(g) un groupe phénylalkyle ou phénylalcoxy contenant de 1 à 7 atomes de carbone dans le fragment alkyle ou alcoxy, le fragment phényle étant éventuellement substitué par 1 ou 2 substituants, identiques ou différents et choisis parmi des substituants halogène, $CF_3$, alkyle en $C_{1-3}$ et alcoxy en $C_{1-3}$,
(h) un groupe alcényloxy contenant jusqu'à 7 atomes de carbone dans le fragment alcényle,
(i) un groupe alcynyloxy contenant jusqu'à 7 atomes de carbone dans le fragment alcynyle,
(j) un groupe alkylthio contenant de 1 à 7 atomes de carbone dans le fragment alkyle,
(k) un groupe phénylthio ou phénylalkylthio contenant de 1 à 3 atomes de carbone dans le fragment alkyle,
(l) un groupe dialkylamino contenant de 1 à 7 atomes de carbone dans chacun des fragments alkyle,
(m) le groupe morpholino,

(n) le groupe pipéridino,
(o) le groupe N-méthylpipérazino,
(p) le groupe pyrrolidino,
(q) un groupe fluoroalcoxy contenant de 2 à 5 atomes de carbone et de 1 à 9 atomes de fluor;

ou $R^6$ et $R^7$, ou $R^7$ et $R^8$ forment, conjointement avec les atomes de carbone adjacents dans le cycle pyridine, un cycle à 5 ou 6 chaînons, saturé ou insaturé, qui peut éventuellement contenir un atome d'oxygène, de soufre ou un atome d'azote éventuellement alkylé;

$R^9$ est
(a) H,
(b) un groupe alkyle contenant de 1 à 4 atomes de carbone;

$R^{10}$ est
(a) un groupe alkyle contenant de 1 à 6 atomes de carbone,
(b) un groupe alcoxy contenant de 1 à 6 atomes de carbone,
(c) le groupe phényle;

$R^e$ est
(a) H,
(b) un groupe alkyle en $C_{1-6}$,
(c) un groupe phényl-alkyle($C_{0-3}$);

et le groupe D étant de préférence sous la forme d'un sel mono- ou di-ionique contenant un cation opposé physiologiquement acceptable, étant entendu que l'un ou l'autre de $R^6$ et $R^8$ ou les deux est-(sont) un(des) atome(s) d'halogène lorsque $R^7$ et un groupe dialkylamino, morpholino, pipéridino, N-méthylpipérazino ou pyrrolidino.

**2.** Composé selon la revendication 1, dans lequel X est SO.

**3.** Composé selon la revendication 1, dans lequel X est S.

**4.** Composé selon la revendication 1, dans lequel $R^1$, $R^2$, $R^3$ et $R^4$ sont identiques ou différents et choisis parmi un atome d'hydrogène ou d'halogène ou un groupe alkyle en $C_{1-4}$, cycloalkyle en $C_{5-6}$, alcoxy en $C_{1-4}$, alcoxy($C_{1-2}$)-alkyle($C_{1-2}$), alcoxy($C_{1-2}$)-alcoxy($C_{1-2}$), alcanoyle en $C_{2-4}$, phénylcarbonyle, alcoxy($C_{1-2}$)-carbonyle, phényl-alcoxy($C_{1-3}$), phényle ou halogénoalcoxy en $C_{1-4}$.

**5.** Composé selon la revendication 1, dans lequel

$R^1$, $R^2$, $R^3$ et $R^4$ sont
(b) un groupe alkyle contenant de 1 à 6 atomes de carbone,
(c) un groupe alcoxy contenant de 1 à 6 atomes de carbone,
(l) un groupe halogénoalcoxy contenant de 1 à 6 atomes de carbone et de 1 à 6 atomes d'halogène; et/ou

$R^6$ est
(b) un groupe alkyle contenant de 1 à 6 atomes de carbone,
(c) un groupe alcoxy contenant de 1 à 6 atomes de carbone; et/ou

$R^8$ est
(b) un groupe alkyle contenant de 1 à 6 atomes de carbone,
(c) un groupe alcoxy contenant de 1 à 6 atomes de carbone; et/ou

$R^7$ est
(j) un groupe alkylthio contenant de 1 à 3 atomes de carbone dans le fragment alkyle,
(k) un groupe phénylalkylthio contenant 1 atome de carbone dans le fragment alkyle,
(l) un groupe dialkylamino contenant de 1 à 3 atomes de carbone dans chacun des fragments alkyle.

**6.** Composé selon la revendication 1, dans lequel $R^1$, $R^2$, $R^3$ et $R^4$ sont H ou un groupe alkyle contenant de 1 à 6 atomes de carbone ou alcoxy contenant de 1 à 6 atomes de carbone.

**7.** Composé selon la revendication 1, dans lequel $R^1$, $R^2$, $R^3$ et $R^4$ sont tous H.

**8.** Composé selon la revendication 1, dans lequel $R^1$, $R^3$ et $R^4$ sont H et $R^2$ est $OCH_3$, ou $R^1$, $R^2$ et $R^4$ sont des atomes d'hydrogène et $R^3$ est $OCH_3$.

**9.** Composé selon la revendication 1, dans lequel $R^7$ est un groupe alkyle contenant de 1 à 6 atomes de carbone ou alcoxy contenant de 1 à 6 atomes de carbone.

**10.** Composé selon la revendication 1, dans lequel $R^9$ est H.

**11.** Composé selon la revendication 1, dans lequel D est

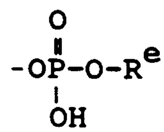

et sels alcalins de celui-ci, $R^e$ étant tel que défini dans la revendication 1.

**12.** Composé selon la revendication 1, dans lequel le substituant en position 1 du noyau benzimidazole est

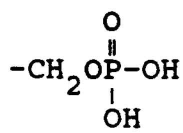

et sels alcalins de celui-ci.

**13.** Composé selon la revendication 1, dans lequel le substituant en position 1 du noyau benzimidazole est

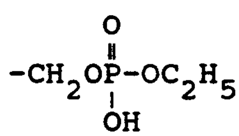

et sels alcalins de celui-ci.

**14.** Composé selon la revendication 1, dans lequel $R^9$ est H et D est

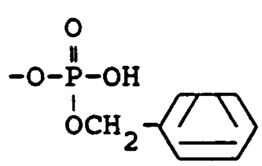

**15.** Composé selon la revendication 1, dans lequel le fragment pyridine est substitué par les groupes 3,5-diméthyl-4-méthoxy, 3-méthyl-4-méthoxy, 5-éthyl-4-méthoxy, 4-méthoxy, 4-éthoxy, 4-isopropoxy, 3,5-diméthyle, 3,4-diméthyle, 4,5-diméthyle, 3-méthyl-4-(2,2,2-trifluoro)éthoxy, 3,4-diméthoxy, 4,5-diméthoxy, 3-méthyl-4-éthylthio, 3-méthyl-4,5-diméthoxy, 3,4,5-triméthyle, 3-éthyl-4-méthoxy, 3-n-propyl-4-méthoxy, 3-isopropyl-4-méthoxy ou 3-tert-butyl-4-méthoxy.

**16.** Composé selon la revendication 1, dans lequel le fragment pyridine est substitué par les groupes 3,5-diméthyl-4-méthoxy, 3-méthyl-4-méthoxy, 3-éthyl-4-méthoxy, 3-isopropyl-4-méthoxy, 4-méthoxy, 4-éthoxy ou 4-isopropoxy.

17. Composé selon la revendication 1, dans lequel
X est SO,
$R^1$, $R^2$, $R^3$ et $R^4$ sont tels que définis dans la revendication 4,
D est tel que défini dans la revendication 11, et $R^6$ et $R^8$ sont $CH_3$, et $R^7$ est $OCH_3$.

18. Composé selon la revendication 1, dans lequel les fragments pyridinylméthylsulfinylbenzimidazole sont

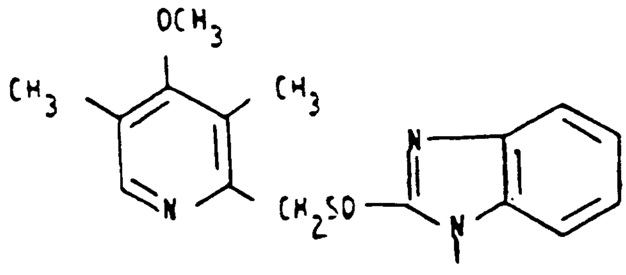

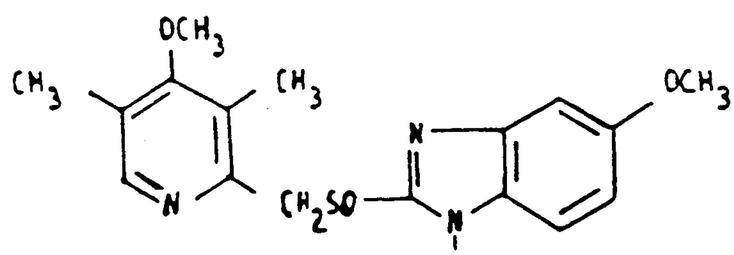

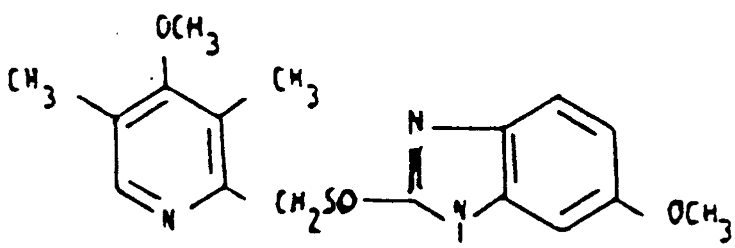

19. Composé de formule

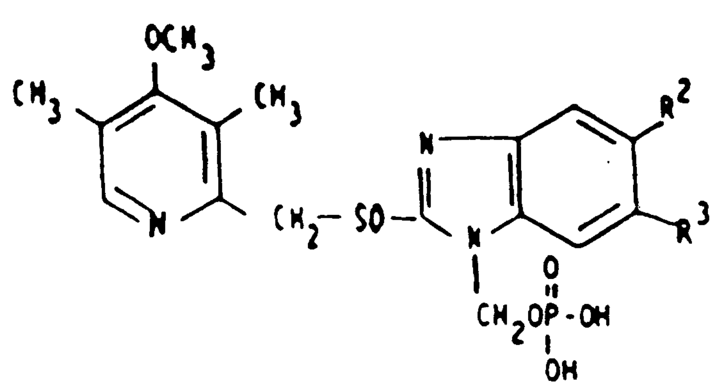

ou sel mono- ou di-ionique de celui-ci, contenant un cation opposé physiologiquement acceptable, dans lequel $R^2$ et $R^3$ sont combinés comme suit:

| $R^2$ | $R^3$ |
|---|---|
| H | $OCH_3$ |
| $OCH_3$ | H |

**20.** Composition pharmaceutique contenant, en tant que composant actif, un composé selon l'une quelconque des revendications 1 à 19.

**21.** Composé tel que défini dans l'une quelconque des revendications 1 à 19, pour utilisation en thérapeutique.

**22.** Composé tel que défini dans l'une quelconque des revendications 1 à 19, pour utilisation dans l'inhibition de la sécrétion d'acide gastrique chez des mammifères et l'homme.

**23.** Composé tel que défini dans l'une quelconque des revendications 1 à 19, pour utilisation dans le traitement de maladies gastro-intestinales inflammatoires chez des mammifères et l'homme.

**24.** Composé selon l'une quelconque des revendications 1 à 19, pour utilisation dans la fabrication d'un médicament pour l'inhibition de la sécrétion d'acide gastrique chez des mammifères et l'homme.

**25.** Composé selon l'une quelconque des revendications 1 à 19, pour utilisation dans la fabrication d'un médicament pour le traitement de maladies gastro-intestinales inflammatoires chez des mammifères et l'homme.

**26.** Procédé pour la préparation d'un composé de formule I selon la revendication 1, par

C. oxydation d'un composé de formule I

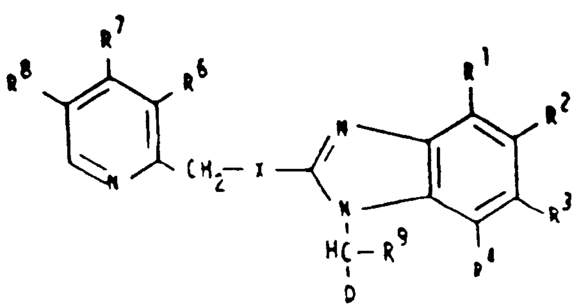

I

dans laquelle X est S, et $R^1$, $R^2$, $R^3$, $R^4$, $R^6$, $R^7$, $R^8$, $R^9$ et D ont les significations données dans la revendication 1, pour l'obtention d'un composé de même formule I dans laquelle X est SO;

D. mise en réaction d'un composé de formule IIA

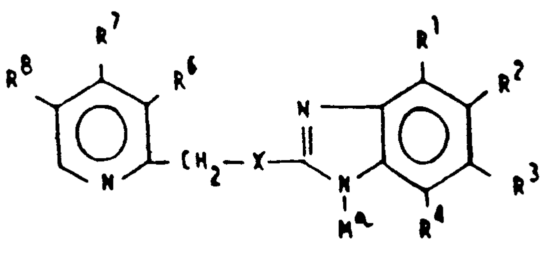

· IIA

dans laquelle $R^1$, $R^2$, $R^3$, $R^4$, $R^6$, $R^7$, $R^8$ et X sont tels que définis sous la formule I et $M^a$ est soit un cation métallique tel que $Na^+$, $K^+$, $Li^+$ ou $Ag^+$, soit un ion ammonium quaternaire, tel que

tétrabutylammonium, avec un composé de formule

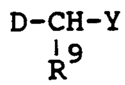

XII

dans laquelle D et $R^9$ sont tels que définis sous la formule I et Y est un atome d'halogène tel que Cl, Br ou I, ou un groupe fonctionnellement équivalent; ou
E. élimination d'un groupe protecteur dans le substituant D;
à la suite de quoi, si nécessaire, on élimine des groupes protecteurs et le composé de formule I ainsi obtenu, si on le désire, est converti en un sel ou en un isomère optique.

**27.** Procédé pour la préparation d'un composé de formule I selon la revendication 1, par mise en réaction d'un composé de formule IV

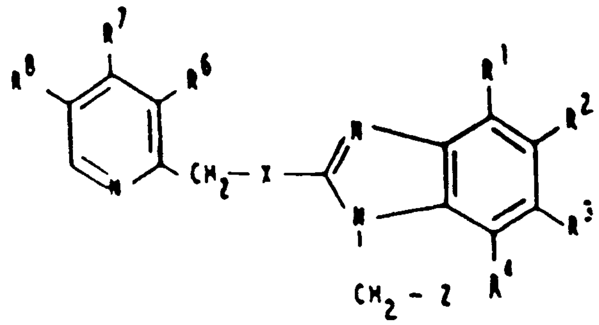

IV

dans laquelle $R^1$, $R^2$, $R^3$, $R^4$, $R^6$, $R^7$, $R^8$ et X sont tels que définis sous la formule I dans la revendication 1, et Z est un atome d'halogène tel que Cl ou un groupe fonctionnellement équivalent, avec un composé de formule VI, VII, VIII ou IX

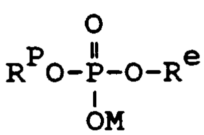

VI

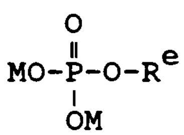

VII

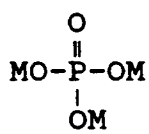

VIII

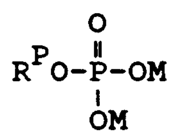

IX

$R^e$ étant tel que défini sous la formule I ci-dessus, $R^P$ étant un groupe protecteur et M étant un ion opposé,
à la suite de quoi, si nécessaire, on élimine des groupes protecteurs et le composé de formule I ainsi obtenu est, si on le désire, converti en un sel ou en un isomère optique.

**28.** Procédé selon la revendication 26 ou 27, pour la préparation d'un composé selon les revendications 2 à 19.

**29.** Composé de formule

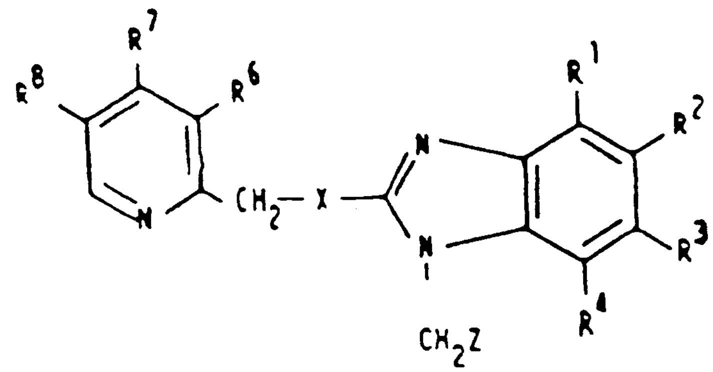

dans laquelle $R^1$, $R^2$, $R^3$, $R^4$, $R^6$, $R^7$, $R^8$ et X sont tels que définis dans la revendication 1, et Z est un atome d'halogène tel que Cl, Br ou I.

**Revendications pour l'Etat contractant suivant : ES**

**1.** Procédé pour la préparation d'un composé de formule

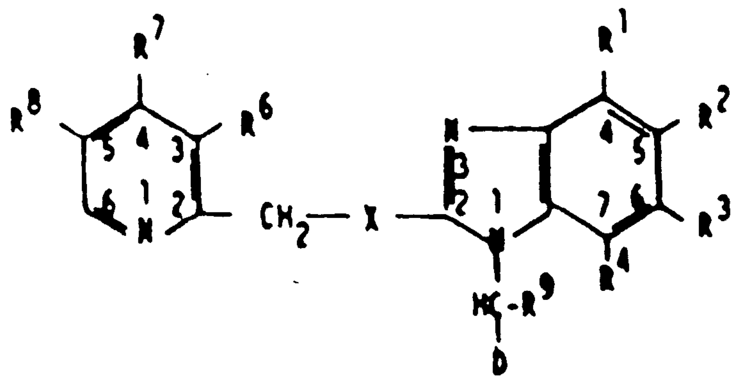

I

et de ses sels physiologiquement acceptables,
formule dans laquelle

X est -S- ou -SO-;

$R^1$, $R^2$, $R^3$ et $R^4$, qui sont identiques ou différents, sont
(a) H
(b) un groupe alkyle contenant de 1 à 8 atomes de carbone,
(c) un groupe alcoxy contenant de 1 à 8 atomes de carbone,
(d) un groupe alcoxyalkyle contenant de 1 à 3 atomes de carbone dans chaque fragment alkyle,
(e) un groupe alcoxyalcoxy contenant de 1 à 3 atomes de carbone dans chaque fragment alkyle,
(f) un atome d'halogène,
(g) $-COR^{10}$,
(h) un groupe alkylthio contenant de 1 à 6 atomes de carbone dans le fragment alkyle,
(i) un groupe alkylsulfinyle contenant de 1 à 7 atomes de carbone dans le fragment alkyle,
(j) un groupe phénylalkyle ou phénylalcoxy contenant de 1 à 6 atomes de carbone dans les fragments alkyle et alcoxy,
(k) un groupe phényle ou phénoxy, chaque groupe phényle étant éventuellement substitué par 1-3 substituants identiques ou différents et choisis parmi des substituants halogène, $CF_3$, alkyle en $C_{1-5}$ et alcoxy en $C_{1-5}$,
(l) un groupe halogénoalcoxy contenant de 1 à 6 atomes de carbone et de 1 à 11 atomes d'halogène;

D est

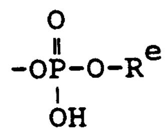

$R^6$ est

(a) H,
(b) un groupe alkyle contenant de 1 à 8 atomes de carbone,
(c) un groupe alcoxy contenant de 1 à 8 atomes de carbone,
(d) un atome d'halogène;

$R^8$ est

(a) H,
(b) un groupe alkyle contenant de 1 à 8 atomes de carbone,
(c) un groupe alcoxy contenant de 1 à 8 atomes de carbone,
(d) un atome d'halogène,
(e) un groupe phénylalkyle contenant de 1 à 4 atomes de carbone dans le fragment alkyle;

$R^7$ est

(a) H,
(b) un groupe alkyle contenant de 1 à 7 atomes de carbone,
(c) un groupe alcoxy contenant de 1 à 7 atomes de carbone,
(d) un groupe alcoxyalkyle contenant de 1 à 3 atomes de carbone dans chaque fragment alkyle,
(e) un groupe alcoxyalcoxy contenant de 1 à 3 atomes de carbone dans chaque fragment alkyle,
(f) un groupe phénoxy, le radical phényle étant éventuellement substitué par 1 ou 2 substituants, identiques ou différents et choisis parmi des substituants halogène, $CF_3$, alkyle en $C_{1-3}$ ou alcoxy en $C_{1-3}$,
(g) un groupe phénylalkyle ou phénylalcoxy contenant de 1 à 7 atomes de carbone dans le fragment alkyle ou alcoxy, le fragment phényle étant éventuellement substitué par 1 ou 2 substituants, identiques ou différents et choisis parmi des substituants halogène, $CF_3$, alkyle en $C_{1-3}$ et alcoxy en $C_{1-3}$,
(h) un groupe alcényloxy contenant jusqu'à 7 atomes de carbone dans le fragment alcényle,
(i) un groupe alcynyloxy contenant jusqu'à 7 atomes de carbone dans le fragment alcynyle,
(j) un groupe alkylthio contenant de 1 à 7 atomes de carbone dans le fragment alkyle,
(k) un groupe phénylthio ou phénylalkylthio contenant de 1 à 3 atomes de carbone dans le fragment alkyle,
(l) un groupe dialkylamino contenant de 1 à 7 atomes de carbone dans chacun des fragments alkyle,
(m) le groupe morpholino,
(n) le groupe pipéridino,
(o) le groupe N-méthylpipérazino,
(p) le groupe pyrrolidino,
(q) un groupe fluoroalcoxy contenant de 2 à 5 atomes de carbone et de 1 à 9 atomes de fluor;

ou $R^6$ et $R^7$ ou $R^7$ et $R^8$ forment, conjointement avec les atomes de carbone adjacents dans le cycle pyridine, un cycle à 5 ou 6 chaînons, saturé ou insaturé, qui peut éventuellement contenir un atome d'oxygène, de soufre ou un atome d'azote éventuellement alkylé;

$R^9$ est

(a) H,
(b) un groupe alkyle contenant de 1 à 4 atomes de carbone;

$R^{10}$ est

(a) un groupe alkyle contenant de 1 à 6 atomes de carbone,
(b) un groupe alcoxy contenant de 1 à 6 atomes de carbone,
(c) le groupe phényle;

$R^e$ est

(a) H,
(b) un groupe alkyle en $C_{1-6}$,
(c) un groupe phényl-alkyle($C_{0-3}$);

et le groupe D étant de préférence sous la forme d'un sel mono- ou di-ionique contenant un cation opposé physiologiquement acceptable, étant entendu que l'un ou l'autre de $R^6$ et $R^8$ ou les deux est-(sont) un(des) atome(s) d'halogène lorsque $R^7$ et un groupe dialkylamino, morpholino, pipéridino, N-méthylpipérazino ou pyrrolidino, par

C. oxydation d'un composé de formule I

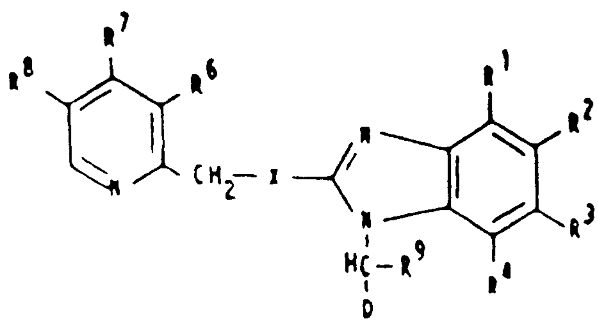

I

dans laquelle X est S et $R^1$, $R^2$, $R^3$, $R^4$, $R^6$, $R^7$, $R^8$, $R^9$ et D ont les significations données plus haut, pour l'obtention d'un composé de même formule I dans laquelle X est SO;

D. mise en réaction d'un composé de formule IIA

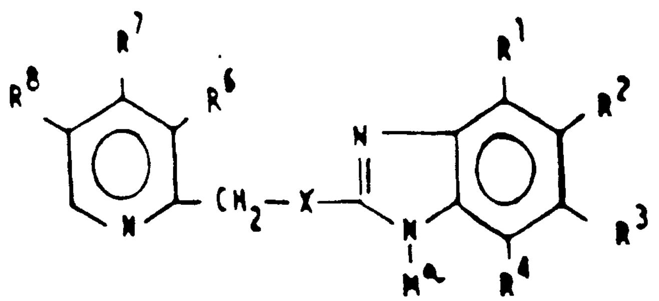

IIA

dans laquelle $R^1$, $R^2$, $R^3$, $R^4$, $R^6$, $R^7$, $R^8$ et X sont tels que définis plus haut et $M^a$ est soit un cation métallique tel que $Na^+$, $K^+$, $Li^+$ ou $Ag^+$, soit un ion ammonium quaternaire, tel que tétrabutylammonium, avec un composé de formule

$$\underset{\substack{| \\ R^9}}{D-CH-Y} \qquad XII$$

dans laquelle D et $R^9$ sont tels que définis plus haut et Y est un atome d'halogène tel que Cl, Br ou I, ou un groupe fonctionnellement équivalent; ou

E. élimination d'un groupe protecteur dans le substituant D;

à la suite de quoi, si nécessaire, on élimine des groupes protecteurs et le composé de formule I ainsi obtenu, si on le désire, est converti en un sel ou en un isomère optique.

**2.** Procédé pour la préparation d'un composé de formule I tel que défini dans la revendication 1, par mise en réaction d'un composé de formule IV

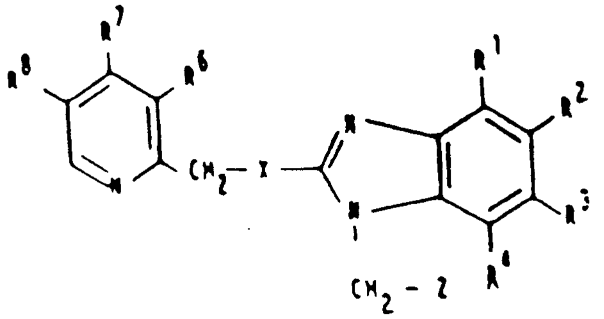

IV

dans laquelle $R^1$, $R^2$ $R^3$, $R^4$, $R^6$, $R^7$, $R^8$ et X sont tels que définis sous la formule I dans la revendication 1, et Z est un atome d'halogène tel que Cl ou un groupe fonctionnellement équivalent, avec un composé de formule VI, VII, VIII ou IX

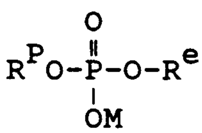

VI

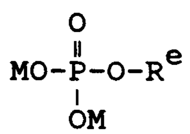

VII

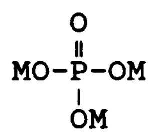

VIII

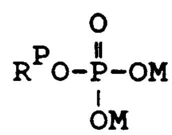

IX

$R^e$ étant tel que défini sous la formule I ci-dessus, $R^P$ étant un groupe protecteur et M étant un ion opposé,
à la suite de quoi, si nécessaire, on élimine des groupes protecteurs et le composé de formule I ainsi obtenu est, si on le désire, converti en un sel ou en un isomère optique.

3. Procédé selon la revendication 1 ou 2, pour la préparation d'un composé tel que défini dans la revendication 1, dans lequel X est SO.

4. Procédé selon la revendication 1 ou 2, pour la préparation d'un composé tel que défini dans la revendication 1, dans lequel X est S.

5. Procédé selon la revendication 1 ou 2 pour la préparation d'un composé tel que défini dans la revendication 1, dans lequel $R^1$, $R^2$, $R^3$ et $R^4$ sont identiques ou différents et choisis parmi un atome d'hydrogène ou d'halogène ou un groupe alkyle en $C_{1-4}$, cycloalkyle en $C_{5-6}$, alcoxy en $C_{1-4}$, alcoxy-$(C_{1-2})$- alkyle$(C_{1-2})$, alcoxy$(C_{1-2})$-alcoxy$(C_{1-2})$, alcanoyle en $C_{2-4}$, phénylcarbonyle, alcoxy$(C_{1-2})$-carbonyle, phényl-alcoxy$(C_{1-3})$, phényle ou halogénoalcoxy en $C_{1-4}$.

6. Procédé selon la revendication 1 ou 2 pour la préparation d'un composé tel que défini dans la revendication 1, dans lequel

$R^1$, $R^2$, $R^3$ et $R^4$ sont
(b) un groupe alkyle contenant de 1 à 6 atomes de carbone,
(c) un groupe alcoxy contenant de 1 à 6 atomes de carbone;
(l) un groupe halogénoalcoxy contenant de 1 à 6 atomes de carbone et de 1 à 6 atomes d'halogène; et/ou

$R^6$ est
(b) un groupe alkyle contenant de 1 à 6 atomes de carbone,
(c) un groupe alcoxy contenant de 1 à 6 atomes de carbone; et/ou

$R^8$ est
(b) un groupe alkyle contenant de 1 à 6 atomes de carbone,
(c) un groupe alcoxy contenant de 1 à 6 atomes de carbone; et/ou

$R^7$ est
(j) un groupe alkylthio contenant de 1 à 3 atomes de carbone dans le fragment alkyle,
(k) un groupe phénylalkylthio contenant 1 atome de carbone dans le

fragment alkyle,
(l) un groupe dialkylamino contenant de 1 à 3 atomes de carbone dans chacun des fragments alkyle.

**7.** Procédé selon la revendication 1 ou 2 pour la préparation d'un composé tel que défini dans la revendication 1, dans lequel $R^1$, $R^2$, $R^3$ et $R^4$ sont H ou un groupe alkyle contenant de 1 à 6 atomes de carbone ou alcoxy contenant de 1 à 6 atomes de carbone.

**8.** Procédé selon la revendication 1 ou 2 pour la préparation d'un composé tel que défini dans la revendication 1, dans lequel $R^1$, $R^2$, $R^3$ et $R^4$ sont tous H.

**9.** Procédé selon la revendication 1 ou 2 pour la préparation d'un composé tel que défini dans la revendication 1, dans lequel $R^1$, $R^3$ et $R^4$ sont H et $R^2$ est $OCH_3$, ou $R^1$, $R^2$ et $R^4$ sont des atomes d'hydrogène et $R^3$ est $OCH_3$.

**10.** Procédé selon la revendication 1 ou 2 pour la préparation d'un composé tel que défini dans la revendication 1, dans lequel $R^7$ est un groupe alkyle contenant de 1 à 6 atomes de carbone ou alcoxy contenant de 1 à 6 atomes de carbone.

**11.** Procédé selon la revendication 1 ou 2 pour la préparation d'un composé tel que défini dans la revendication 1, dans lequel $R^9$ est H.

**12.** Procédé selon la revendication 1 ou 2 pour la préparation d'un composé tel que défini dans la revendication 1, dans lequel D est

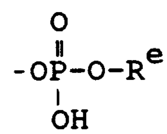

et de ses sels alcalins, $R^e$ étant tel que défini dans la revendication 1.

**13.** Procédé selon la revendication 1 ou 2 pour la préparation d'un composé tel que défini dans la revendication 1, dans lequel le substituant en position 1 du noyau benzimidazole est

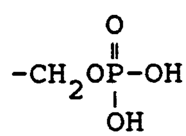

et de ses sels alcalins.

**14.** Procédé selon la revendication 1 ou 2 pour la préparation d'un composé tel que défini dans la revendication 1, dans lequel le substituant en position 1 du noyau benzimidazole est

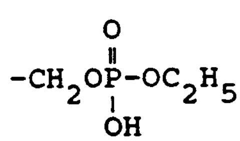

et de ses sels alcalins.

**15.** Procédé selon la revendication 1 ou 2 pour la préparation d'un composé tel que défini dans la revendication 1, dans lequel $R^9$ est H et D est

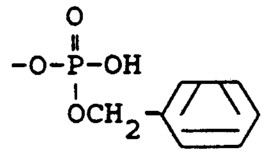

**16.** Procédé selon la revendication 1 ou 2 pour la préparation d'un composé tel que défini dans la revendication 1, dans lequel le fragment pyridine est substitué par les groupes 3,5-diméthyl-4-méthoxy, 3-méthyl-4-méthoxy, 5-éthyl-4-méthoxy, 4-méthoxy, 4-éthoxy, 4-isopropoxy, 3,5-diméthyle, 3,4-diméthyle, 4,5-diméthyle, 3-méthyl-4-(2,2,2-trifluoro)éthoxy, 3,4-diméthoxy, 4,5-diméthoxy, 3-méthyl-4-éthylthio, 3-méthyl-4,5-diméthoxy, 3,4,5-triméthyle, 3-éthyl-4-méthoxy, 3-n-propyl-4-méthoxy, 3-isopropyl-4-méthoxy ou 3-tert-butyl-4-méthoxy.

**17.** Procédé selon la revendication 1 ou 2 pour la préparation d'un composé tel que défini dans la revendication 1, dans lequel le fragment pyridine est substitué par les groupes 3,5-diméthyl-4-méthoxy, 3-méthyl-4-méthoxy, 3-éthyl-4-méthoxy, 3-isopropyl-4-méthoxy, 4-méthoxy, 4-éthoxy ou 4-isopropoxy.

**18.** Procédé selon la revendication 1 ou 2 pour la préparation d'un composé tel que défini dans la revendication 1, dans lequel
X est SO,
$R^1$, $R^2$, $R^3$ et $R^4$ sont tels que définis dans la revendication 5,
D est tel que défini dans la revendication 12, et $R^6$ et $R^8$ sont $CH_3$, et $R^7$ est $OCH_3$.

**19.** Procédé selon la revendication 1 ou 2 pour la préparation d'un composé tel que défini dans la revendication 1, dans lequel les fragments pyridinylméthylsulfinylbenzimidazole sont

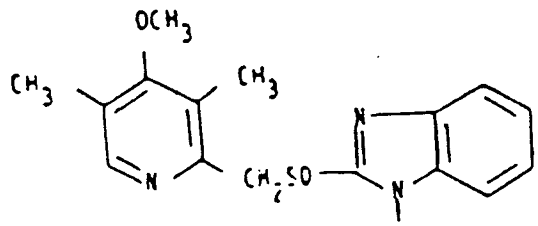

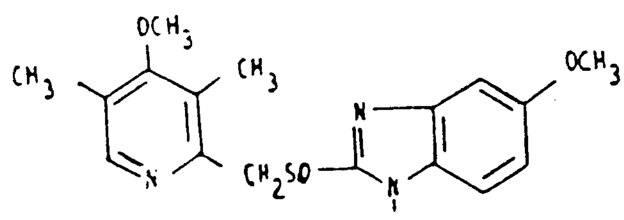

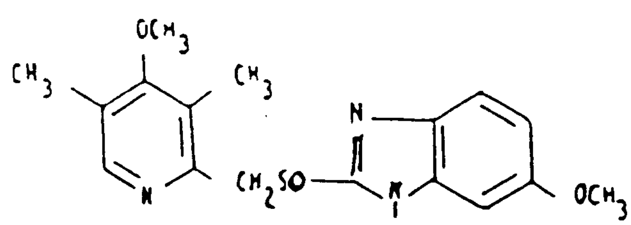

**20.** Procédé selon la revendication 1 ou 2 pour la préparation d'un composé tel que défini dans la formule

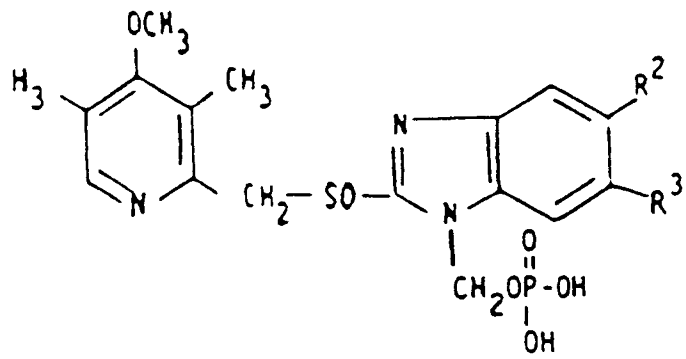

ou d'un sel mono- ou di-ionique de celui-ci, contenant un cation opposé physiologiquement acceptable, dans lequel $R^2$ et $R^3$ sont combinés comme suit:

| $R^2$ | $R^3$ |
|---|---|
| H<br>$OCH_3$ | $OCH_3$<br>H |

## Revendications pour l'Etat contractant suivant : GR

**1.** Procédé pour la préparation d'un composé de formule

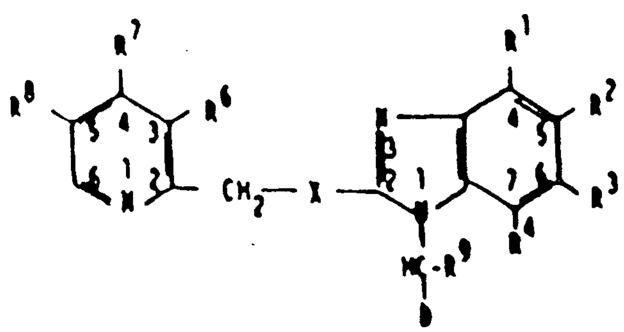

I

et de ses sels physiologiquement acceptables,
formule dans laquelle

X est -S- ou -SO-;

$R^1$, $R^2$, $R^3$ et $R^4$, qui sont identiques ou différents, sont

(a) H

(b) un groupe alkyle contenant de 1 à 8 atomes de carbone,

(c) un groupe alcoxy contenant de 1 à 8 atomes de carbone,

(d) un groupe alcoxyalkyle contenant de 1 à 3 atomes de carbone dans chaque fragment alkyle,

(e) un groupe alcoxyalcoxy contenant de 1 à 3 atomes de carbone dans chaque fragment alkyle,

(f) un atome d'halogène,

(g) $-COR^{10}$,

(h) un groupe alkylthio contenant de 1 à 6 atomes de carbone dans le fragment alkyle,

(i) un groupe alkylsulfinyle contenant de 1 à 7 atomes de carbone dans le fragment alkyle,

(j) un groupe phénylalkyle ou phénylalcoxy contenant de 1 à 6 atomes de carbone dans les fragments alkyle et alcoxy,
(k) un groupe phényle ou phénoxy, chaque groupe phényle étant éventuellement substitué par 1-3 substituants identiques ou différents et choisis parmi des substituants halogène, $CF_3$, alkyle en $C_{1-5}$ et alcoxy en $C_{1-5}$,
(l) un groupe halogénoalcoxy contenant de 1 à 6 atomes de carbone et de 1 à 11 atomes d'halogène;

D est

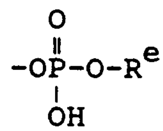

$R^6$ est

(a) H,
(b) un groupe alkyle contenant de 1 à 8 atomes de carbone,
(c) un groupe alcoxy contenant de 1 à 8 atomes de carbone,
(d) un atome d'halogène;

$R^8$ est

(a) H,
(b) un groupe alkyle contenant de 1 à 8 atomes de carbone,
(c) un groupe alcoxy contenant de 1 à 8 atomes de carbone,
(d) un atome d'halogène,
(e) un groupe phénylalkyle contenant de 1 à 4 atomes de carbone dans le fragment alkyle;

$R^7$ est

(a) H,
(b) un groupe alkyle contenant de 1 à 7 atomes de carbone,
(c) un groupe alcoxy contenant de 1 à 7 atomes de carbone,
(d) un groupe alcoxyalkyle contenant de 1 à 3 atomes de carbone dans chaque fragment alkyle,
(e) un groupe alcoxyalcoxy contenant de 1 à 3 atomes de carbone dans chaque fragment alkyle,
(f) un groupe phénoxy, le radical phényle étant éventuellement substitué par 1 ou 2 substituants, identiques ou différents et choisis parmi des substituants halogène, $CF_3$, alkyle en $C_{1-3}$ ou alcoxy en $C_{1-3}$,
(g) un groupe phénylalkyle ou phénylalcoxy contenant de 1 à 7 atomes de carbone dans le fragment alkyle ou alcoxy, le fragment phényle étant éventuellement substitué par 1 ou 2 substituants, identiques ou différents et choisis parmi des substituants halogène, $CF_3$, alkyle en $C_{1-3}$ et alcoxy en $C_{1-3}$,
(h) un groupe alcényloxy contenant jusqu'à 7 atomes de carbone dans le fragment alcényle,
(i) un groupe alcynyloxy contenant jusqu'à 7-atomes de carbone dans le fragment alcynyle,
(j) un groupe alkylthio contenant de 1 à 7 atomes de carbone dans le fragment alkyle,
(k) un groupe phénylthio ou phénylalkylthio contenant de 1 à 3 atomes de carbone dans le fragment alkyle,
(l) un groupe dialkylamino contenant de 1 à 7 atomes de carbone dans chacun des fragments alkyle,
(m) le groupe morpholino,
(n) le groupe pipéridino,
(o) le groupe N-méthylpipérazino,
(p) le groupe pyrrolidino,
(q) un groupe fluoroalcoxy contenant de 2 à 5 atomes de carbone et de 1 à 9

atomes de fluor;

ou $R^6$ et $R^7$ ou $R^7$ et $R^8$ forment, conjointement avec les atomes de carbone adjacents dans le cycle pyridine, un cycle à 5 ou 6 chaînons, saturé ou insaturé, qui peut éventuellement contenir un atome d'oxygène, de soufre ou un atome d'azote éventuellement alkylé;

$R^9$ est

(a) H,
(b) un groupe alkyle contenant de 1 à 4 atomes de carbone;

$R^{10}$ est

(a) un groupe alkyle contenant de 1 à 6 atomes de carbone,
(b) un groupe alcoxy contenant de 1 à 6 atomes de carbone,
(c) le groupe phényle;

$R^e$ est

(a) H,
(b) un groupe alkyle en $C_{1-6}$,
(c) un groupe phényl-alkyle($C_{0-3}$);

et le groupe D étant de préférence sous la forme d'un sel mono- ou di-ionique contenant un cation opposé physiologiquement acceptable, étant entendu que l'un ou l'autre de $R^6$ et $R^8$ ou les deux est-(sont) un(des) atome(s) d'halogène lorsque $R^7$ et un groupe dialkylamino, morpholino, pipéridino, N-méthylpipérazino ou pyrrolidino, par

C. oxydation d'un composé de formule I

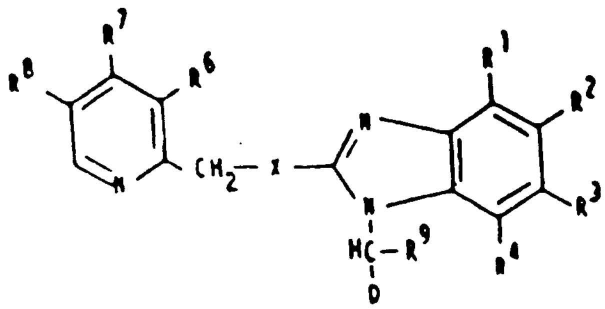

I

dans laquelle X est S et $R^1$, $R^2$, $R^3$, $R^4$, $R^6$, $R^7$, $R^8$, $R^9$ et D ont les significations données plus haut, pour l'obtention d'un composé de même formule I dans laquelle X est SO;

D. mise en réaction d'un composé de formule IIA

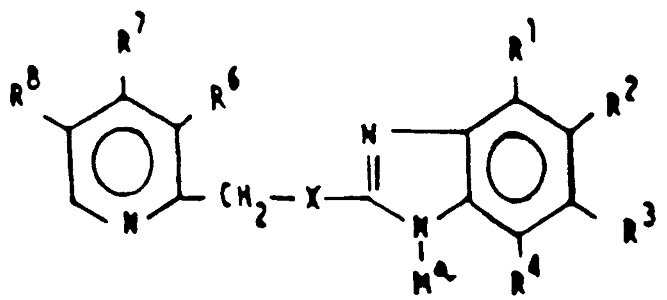

IIA

dans laquelle $R^1$, $R^2$, $R^3$, $R^4$, $R^6$, $R^7$, $R^8$ et X sont tels que définis plus haut et $M^a$ est soit un cation métallique tel que $Na^+$, $K^+$, $Li^+$ ou $Ag^+$, soit un ion ammonium quaternaire, tel que tétrabutylammonium, avec un composé de formule

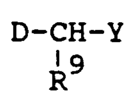

XII

dans laquelle D et $R^9$ sont tels que définis plus haut et Y est un atome d'halogène tel que Cl, Br ou

I, ou un groupe fonctionnellement équivalent; ou

E. élimination d'un groupe protecteur dans le substituant D;

à la suite de quoi, si nécessaire, on élimine des groupes protecteurs et le composé de formule I ainsi obtenu, si on le désire, est converti en un sel ou en un isomère optique.

**2.** Procédé pour la préparation d'un composé de formule I tel que défini dans la revendication 1, par mise en réaction d'un composé de formule IV

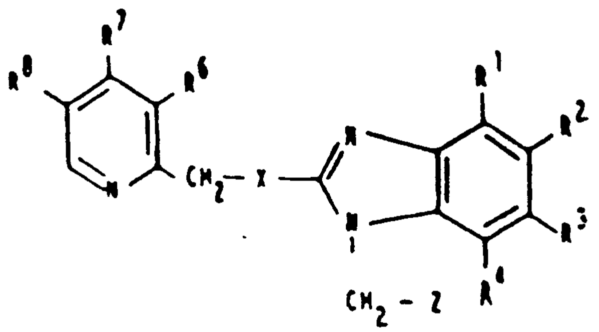

IV

dans laquelle $R^1$, $R^2$, $R^3$, $R^4$, $R^6$, $R^7$, $R^8$ et X sont tels que définis sous la formule I dans la revendication 1, et Z est un atome d'halogène tel que Cl ou un groupe fonctionnellement équivalent, avec un composé de formule VI, VII, VIII ou IX

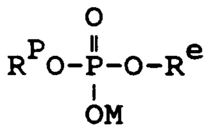

VI

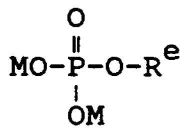

VII

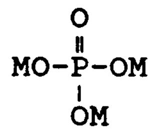

VIII

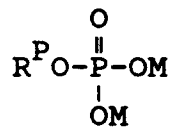

IX

$R^e$ étant tel que défini sous la formule I ci-dessus, $R^P$ étant un groupe protecteur et M étant un ion opposé,

à la suite de quoi, si nécessaire, on élimine des groupes protecteurs et le composé de formule I ainsi obtenu est, si on le désire, converti en un sel ou en un isomère optique.

**3.** Procédé selon la revendication 1 ou 2, pour la préparation d'un composé tel que défini dans la revendication 1, dans lequel X est SO.

**4.** Procédé selon la revendication 1 ou 2, pour la préparation d'un composé tel que défini dans la revendication 1, dans lequel X est S.

**5.** Procédé selon la revendication 1 ou 2 pour la préparation d'un composé tel que défini dans la revendication 1, dans lequel $R^1$, $R^2$, $R^3$ et $R^4$ sont identiques ou différents et choisis parmi un atome d'hydrogène ou d'halogène ou un groupe alkyle en $C_{1-4}$, cycloalkyle en $C_{5-6}$, alcoxy en $C_{1-4}$, alcoxy-($C_{1-2}$)- alkyle($C_{1-2}$), alcoxy($C_{1-2}$)-alcoxy($C_{1-2}$), alcanoyle en $C_{2-4}$, phénylcarbonyle, alcoxy($C_{1-2}$)-carbonyle, phényl-alcoxy($C_{1-3}$), phényle ou halogénoalcoxy en $C_{1-4}$.

**6.** Procédé selon la revendication 1 ou 2 pour la préparation d'un composé tel que défini dans la revendication 1, dans lequel

$R^1$, $R^2$, $R^3$ et $R^4$ sont

(b) un groupe alkyle contenant de 1 à 6 atomes de carbone,

(c) un groupe alcoxy contenant de 1 à 6 atomes de carbone;

(l) un groupe halogénoalcoxy contenant de 1 à 6 atomes de carbone et de 1 à 6 atomes d'halogène; et/ou

$R^6$ est

(b) un groupe alkyle contenant de 1 à 6 atomes de carbone,

(c) un groupe alcoxy contenant de 1 à 6 atomes de carbone; et/ou

$R^8$ est

(b) un groupe alkyle contenant de 1 à 6 atomes de carbone,
(c) un groupe alcoxy contenant de 1 à 6 atomes de carbone; et/ou

$R^7$ est

(j) un groupe alkylthio contenant de 1 à 3 atomes de carbone dans le fragment alkyle,
(k) un groupe phénylalkylthio contenant 1 atome de carbone dans le fragment alkyle,
(l) un groupe dialkylamino contenant de 1 à 3 atomes de carbone dans chacun des fragments alkyle.

**7.** Procédé selon la revendication 1 ou 2 pour la préparation d'un composé tel que défini dans la revendication 1, dans lequel $R^1$, $R^2$, $R^3$ et $R^4$ sont H ou un groupe alkyle contenant de 1 à 6 atomes de carbone ou alcoxy contenant de 1 à 6 atomes de carbone.

**8.** Procédé selon la revendication 1 ou 2 pour la préparation d'un composé tel que défini dans la revendication 1, dans lequel $R^1$, $R^2$, $R^3$ et $R^4$ sont tous H.

**9.** Procédé selon la revendication 1 ou 2 pour la préparation d'un composé tel que défini dans la revendication 1, dans lequel $R^1$, $R^3$ et $R^4$ sont H et $R^2$ est $OCH_3$, ou $R^1$, $R^2$ et $R^4$ sont des atomes d'hydrogène et $R^3$ est $OCH_3$.

**10.** Procédé selon la revendication 1 ou 2 pour la préparation d'un composé tel que défini dans la revendication 1, dans lequel $R^7$ est un groupe alkyle contenant de 1 à 6 atomes de carbone ou alcoxy contenant de 1 à 6 atomes de carbone.

**11.** Procédé selon la revendication 1 ou 2 pour la préparation d'un composé tel que défini dans la revendication 1, dans lequel $R^9$ est H.

**12.** Procédé selon la revendication 1 ou 2 pour la préparation d'un composé tel que défini dans la revendication 1, dans lequel D est

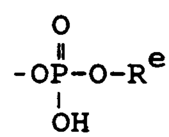

et de ses sels alcalins, $R^e$ étant tel que défini dans la revendication 1.

**13.** Procédé selon la revendication 1 ou 2 pour la préparation d'un composé tel que défini dans la revendication 1, dans lequel le substituant en position 1 du noyau benzimidazole est

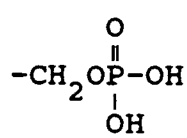

et de ses sels alcalins.

**14.** Procédé selon la revendication 1 ou 2 pour la préparation d'un composé tel que défini dans la revendication 1, dans lequel le substituant en position 1 du noyau benzimidazole est

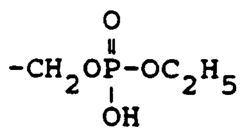

et de ses sels alcalins.

**15.** Procédé selon la revendication 1 ou 2 pour la préparation d'un composé tel que défini dans la revendication 1, dans lequel $R^9$ est H et D est

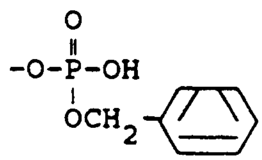

**16.** Procédé selon la revendication 1 ou 2 pour la préparation d'un composé tel que défini dans la revendication 1, dans lequel le fragment pyridine est substitué par les groupes 3,5-diméthyl-4-méthoxy, 3-méthyl-4-méthoxy, 5-éthyl-4-méthoxy, 4-méthoxy, 4-éthoxy, 4-isopropoxy, 3,5-diméthyle, 3,4-diméthyle, 4,5-diméthyle, 3-méthyl-4-(2,2,2-trifluoro)éthoxy, 3,4-diméthoxy, 4,5-diméthoxy, 3-méthyl-4-éthylthio, 3-méthyl-4,5-diméthoxy, 3,4,5-triméthyle, 3-éthyl-4-méthoxy, 3-n-propyl-4-méthoxy, 3-isopropyl-4-méthoxy ou 3-tert-butyl-4-méthoxy.

**17.** Procédé selon la revendication 1 ou 2 pour la préparation d'un composé tel que défini dans la revendication 1, dans lequel le fragment pyridine est substitué par les groupes 3,5-diméthyl-4-méthoxy, 3-méthyl-4-méthoxy, 3-éthyl-4-méthoxy, 3-isopropyl-4-méthoxy, 4-méthoxy, 4-éthoxy ou 4-isopropoxy.

**18.** Procédé selon la revendication 1 ou 2 pour la préparation d'un composé tel que défini dans la revendication 1, dans lequel
X est SO,
$R^1$, $R^2$, $R^3$ et $R^4$ sont tels que définis dans la revendication 5,
D est tel que défini dans la revendication 12, et $R^6$ et $R^8$ sont $CH_3$, et $R^7$ est $OCH_3$.

**19.** Procédé selon la revendication 1 ou 2 pour la préparation d'un composé tel que défini dans la revendication 1, dans lequel les fragments pyridinylméthylsulfinylbenzimidazole sont

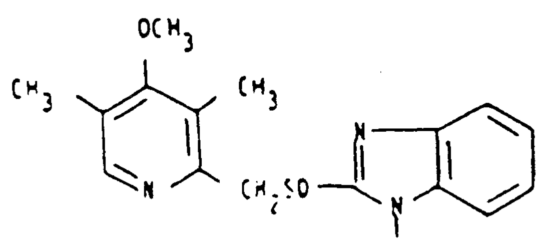

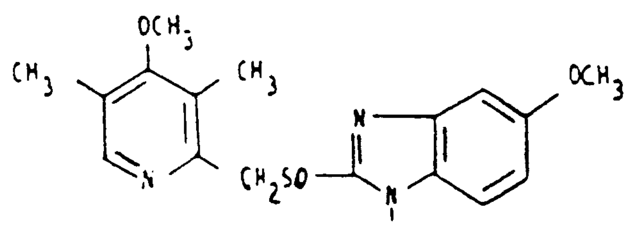

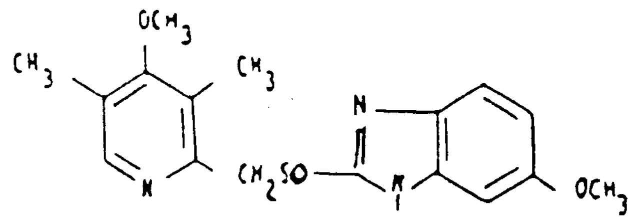

**20.** Procédé selon la revendication 1 ou 2 pour la préparation d'un composé tel que défini dans la formule

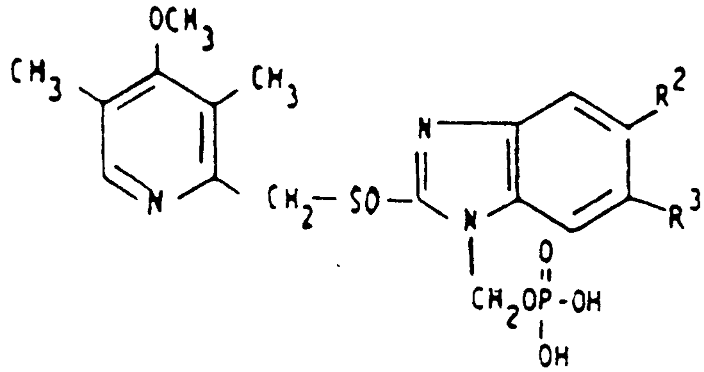

ou d'un sel mono- ou di-ionique de celui-ci, contenant un cation opposé physiologiquement acceptable, dans lequel $R^2$ et $R^3$ sont combinés comme suit:

| $R^2$ | $R^3$ |
|---|---|
| H | $OCH_3$ |
| $OCH_3$ | H |

**21.** Composé de formule

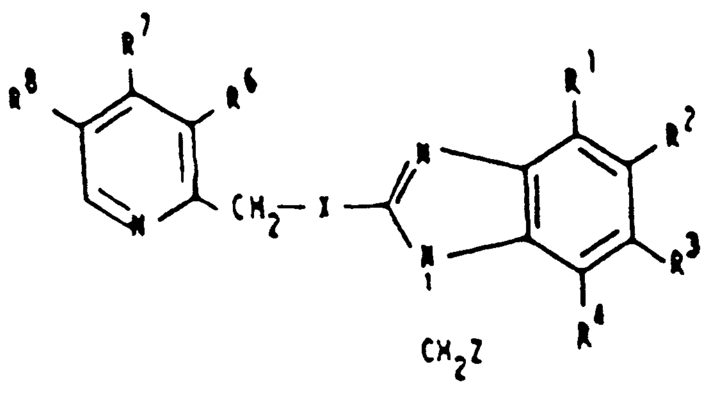

dans laquelle $R^1$, $R^2$, $R^3$, $R^4$, $R^6$, $R^7$, $R^8$ et X sont tels que définis dans la revendication 1, et Z est un atome d'halogène tel que Cl, Br ou I.